# EUROPEAN PATENT APPLICATION

(11) **EP 4 361 138 A1**
(43) Date of publication of application: **01.05.2024**
(21) Application number: 22827611.9
(22) Date of filing: 22.06.2022
(51) Int. Cl.: C07D 401/12, C07D 401/14, C07D 409/14, C07D 411/14, C07D 417/14, A61K 31/444, A61K 31/497, A61P 29/00, A61P 37/00

(54) **SULFOXIMINE COMPOUND AND USE THEREOF**

(30) Priority: 22.06.2021 CN 202110694497; 04.01.2022 CN 202210002094; 13.06.2022 CN 202210665753
(71) Applicant: Medshine Discovery Inc., Nanjing, Jiangsu 210032 (CN)
(72) Inventor: QIAN, Wenyuan, Shanghai 200131 (CN); YANG, Chundao, Shanghai 200131 (CN)
(74) Representative: Cabinet Becker et Associés
(86) International application number: PCT/CN2022/100435
(87) International publication number: WO 2022/268119

(57) **Abstract**

A sulfoximine compound and a use thereof. Specifically disclosed is a compound represented by the following formula or a pharmaceutically acceptable salt thereof.

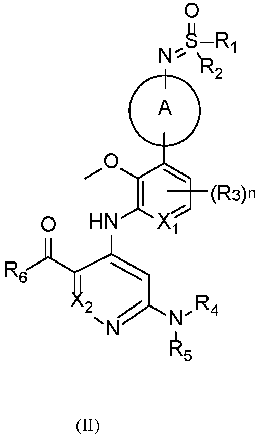

## Description

**This application claims the priority of:**
CN202110694497.1, filed on June 22, 2021;
CN202210002094.0, filed on January 14, 2022;
CN202210665753.9, filed on June 13, 2022.

### FIELD OF THE INVENTION

The present disclosure relates to a class of sulfoximine compounds and use thereof, specifically to a compound represented by formula (II) or a pharmaceutically acceptable salt thereof.

### BACKGROUND OF THE INVENTION

The family of Janus kinases (JAKs) is a class of intracellular non-receptor tyrosine kinases that are mainly responsible for regulating signaling pathways mediated by cytokine receptors. These signaling pathways can be activated by a variety of cytokines, growth factors, and receptors, and are involved in important physiological processes such as proliferation, differentiation, apoptosis, angiogenesis, and immune regulation of various types of cells. The family of Janus kinases includes four different isoforms in mammals: JAK1, JAK2, JAK3 and TYK2 (tyrosine kinase 2).

Like other kinases in the same family, TYK2 also has structurally 4 conserved domains consisting of 7 homologous domains (JAK homology domain, JH), including the C-terminal pseudokinase region (JH2) and kinase region (JH1), as well as the N-terminal FERM region (Four. 1 protein, Ezrin, Radixin, Moesin) and SH2 domain (srchomology 2 domain).

In cells, TYK2 forms a dimer with JAK2 to mediate the signaling of IL-23 and IL-12, and can also forms a dimer with JAK1 to mediate the response to type I interferon. These cytokines are involved in the pathogenesis of various inflammatory and autoimmune diseases such as psoriasis, inflammatory bowel disease (IBD) and systemic lupus erythematosus (SLE). By inhibiting TYK2, the signaling pathways of some inflammatory cytokines can be blocked to achieve the purpose of treating related diseases.

Current TYK2 inhibitors mainly include orthosteric inhibitors that inhibit the kinase domain (JH1) and allosteric inhibitors that inhibit the pseudokinase domain (JH2). Orthosteric inhibitors are represented by Pfizer's PF-06826647, which is used to treat diseases such as plaque and ulcerative colitis, and is currently in phase II clinical trials. Allosteric inhibitors are represented by BMS-986165. The clinical trial of BMS-986165 for the treatment of massive psoriasis has been advanced to Phase III, with outstanding clinical effects and good safety. At the same time, BMS-986165 is also in clinical research for a variety of autoimmune diseases including Crohn's disease, psoriatic arthritis, and systemic lupus erythematosus. In addition to BMS-986165, Nimbus also has several TYK2 allosteric inhibitors in preclinical screening, and Fronthera's TYK2 allosteric inhibitor FTP-637 that has been acquired by Hisco (reported recently) is preparing to enter phase I clinical trials.

### SUMMARY OF THE INVENTION

The present disclosure provides a compound of formula (II) or a pharmaceutically acceptable salt thereof, 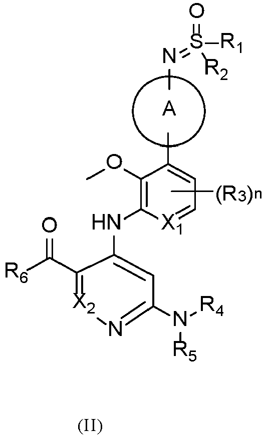 wherein
ring A is 6-membered heteroaryl;
X₁ and X₂ are each independently selected from N and CH;
R₁ and R₂ are independently selected from C₁₋₃ alkyl, wherein the C₁₋₃ alkyl is optionally substituted with 1, 2, 3 or 4 Rₐ;
alternatively, R₁ and R₂ are taken together with the S atom to which they are attached to form 4- to 6-membered heterocycloalkyl, wherein the 4- to 6-membered heterocycloalkyl is optionally substituted with 1, 2, 3 or 4 Rₐ;
each R₃ is independently selected from hydrogen, deuterium, fluorine, chlorine, bromine, C₁₋₃ alkyl and C₁₋₃ alkoxy;
R₄ is selected from hydrogen, -C(=O)R₄₁, -C(=O)NR₄₂R₄₃, 5- to 10-membered heteroaryl and phenyl, wherein the 5- to 10-membered heteroaryl and phenyl are optionally substituted with 1, 2 or 3 R_{b};
R₄₁ is selected from C₁₋₃ alkyl, C₃₋₈ cycloalkyl, 5- to 6-membered heteroaryl, phenyl and 4- to 6-membered heterocycloalkyl, wherein the C₁₋₃ alkyl, C₃₋₈ cycloalkyl, 5- to 6-membered heteroaryl, phenyl and 4- to 6-membered heterocycloalkyl are optionally substituted with 1, 2, 3 or 4 R_{c};
R₄₂ is selected from hydrogen and C₁₋₃ alkyl;
R₄₃ is selected from C₁₋₃ alkyl, C₃₋₈ cycloalkyl, 5- to 6-membered heteroaryl and 4- to 6-membered heterocycloalkyl, wherein the C₁₋₃ alkyl, C₃₋₈ cycloalkyl, 5- to 6-membered heteroaryl and 4- to 6-membered heterocycloalkyl are optionally substituted with 1, 2, 3 or 4 R_{c};
R₅ is selected from hydrogen and C₁₋₃ alkyl;
R₆ is selected from C₁₋₃ alkyl, C₁₋₃ alkoxy, C₃₋₆ cycloalkyl, -NH-C₁₋₃ alkyl and -NH-C₃₋₆ cycloalkyl, wherein the C₁₋₃ alkyl, C₁₋₃ alkoxy, C₃₋₆ cycloalkyl, -NH-C₁₋₃ alkyl and -NH-C₃₋₆ cycloalkyl are optionally substituted with 1, 2, 3 or 4 R_{d};
Rₐ, R_{b}, R_{c} and R_{d} are independently selected from H, deuterium, fluorine, chlorine, bromine, iodine, CN, NH₂, C₁₋₃ alkyl and C₁₋₃ alkoxy;
n is selected from 0, 1, 2 and 3.

The present disclosure provides a compound of formula (II) or a pharmaceutically acceptable salt thereof, 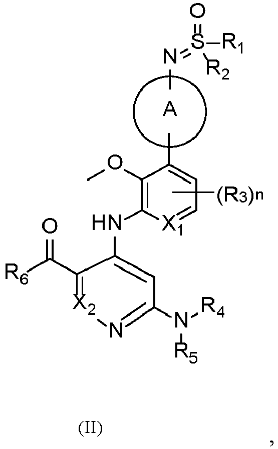 wherein
ring A is 6-membered heteroaryl;
X₁ and X₂ are independently selected from N and CH;
R₁ and R₂ are independently selected from C₁₋₃ alkyl, wherein the C₁₋₃ alkyl is optionally substituted with 1, 2, 3 or 4 Rₐ;
alternatively, R₁ and R₂ are taken together with the S atom to which they are attached to form 4- to 6-membered heterocycloalkyl, wherein the 4- to 6-membered heterocycloalkyl is optionally substituted with 1, 2, 3 or 4 Rₐ;
each R₃ is independently selected from hydrogen, deuterium, fluorine, chlorine, bromine, C₁₋₃ alkyl and C₁₋₃ alkoxy;
R₄ is selected from hydrogen, -C(=O)R₄₁ and 5- to 6-membered heteroaryl, wherein the 5- to 6-membered heteroaryl is optionally substituted with 1, 2 or 3 R_{b};
R₄₁ is selected from C₁₋₃ alkyl and C₃₋₆ cycloalkyl, wherein the C₁₋₃ alkyl and C₃₋₆ cycloalkyl are optionally substituted with 1, 2, 3 or 4 R_{c};
R₅ is selected from hydrogen and C₁₋₃ alkyl;
R₆ is selected from C₁₋₃ alkyl, C₁₋₃ alkoxy, C₃₋₆ cycloalkyl, -NH-C₁₋₃ alkyl and -NH-C₃₋₆ cycloalkyl, wherein the C₁₋₃ alkyl, C₁₋₃ alkoxy, C₃₋₆ cycloalkyl, -NH-C₁₋₃ alkyl and -NH-C₃₋₆ cycloalkyl are optionally substituted with 1, 2, 3 or 4 R_{d};
Rₐ, R_{b}, R_{c} and R_{d} are independently selected from H, deuterium, fluorine, chlorine, bromine, iodine, CN, NH₂, C₁₋₃ alkyl and C₁₋₃ alkoxy;
n is 0, 1, 2 or 3.

In some embodiments of the present disclosure, the above-mentioned ring A is selected from 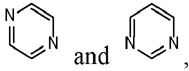 and other variables are as defined herein.

In some embodiments of the present disclosure, the above-mentioned ring A is selected from 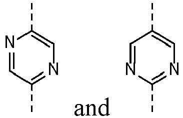 , and other variables are as defined herein.

In some embodiments of the present disclosure, the above-mentioned R₁ and R₂ are independently selected from methyl, ethyl and propyl, wherein the methyl, ethyl and propyl are optionally substituted with 1, 2, 3 or 4 Rₐ, and other variables are as defined herein.

In some embodiments of the present disclosure, the above-mentioned R₁ and R₂ are independently selected from methyl and ethyl, and other variables are as defined herein.

In some embodiments of the present disclosure, the above-mentioned R₁ and R₂ are independently selected from methyl, and other variables are as defined herein.

In some embodiments of the present disclosure, the above-mentioned R₁ and R₂ are taken together with the S atom to which they are attached to form 4- to 6-membered heterocycloalkyl, wherein the 4- to 6-membered heterocycloalkyl is optionally substituted with 1, 2, 3 or 4 Rₐ, and other variables are as defined herein.

In some embodiments of the present disclosure, the above-mentioned R₁ and R₂ are taken together with the S atom to which they are attached to form 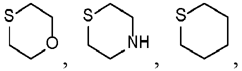 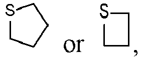 wherein the 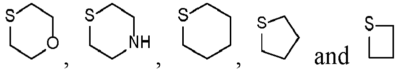 are optionally substituted with 1, 2, 3 or 4 Rₐ, and other variables are as defined herein.

In some embodiments of the present disclosure, the above-mentioned R₁ and R₂ are taken together with the S atom to which they are attached to form 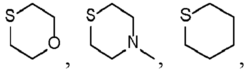 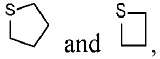 and other variables are as defined herein.

In some embodiments of the present disclosure, the above-mentioned Rₐ is selected from hydrogen, and other variables are as defined herein.

In some embodiments of the present disclosure, the above-mentioned structural moiety 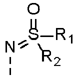 is selected from 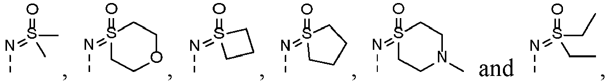 and other variables are as defined herein.

In some embodiments of the present disclosure, the above-mentioned structural moiety 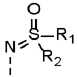 is selected from 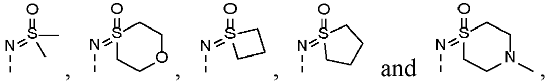 and other variables are as defined herein.

In some embodiments of the present disclosure, the above-mentioned each X₁ is selected from N, and other variables are as defined herein.

In some embodiments of the present disclosure, the above-mentioned each X₁ is selected from CH, and other variables are as defined herein.

In some embodiments of the present disclosure, the above-mentioned each R₃ is independently selected from hydrogen and fluorine, and other variables are as defined herein.

In some embodiments of the present disclosure, the above-mentioned structural moiety 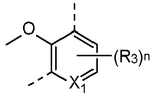 is selected from 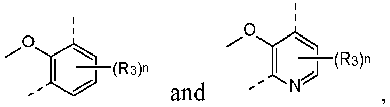 and other variables are as defined herein.

In some embodiments of the present disclosure, the above-mentioned 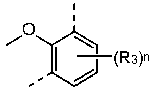 is selected from 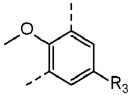 , and other variables are as defined herein.

In some embodiments of the present disclosure, the above-mentioned 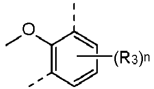 is selected from 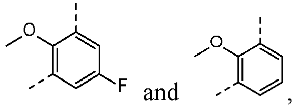 and other variables are as defined herein.

In some embodiments of the present disclosure, the above-mentioned 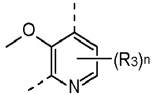 is selected from 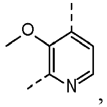 and other variables are as defined herein.

In some embodiments of the present disclosure, the above-mentioned R₄ is selected from hydrogen, -C(O)R₄₁, -C(O)NR₄₂R₄₃, 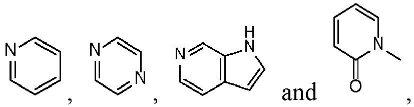 wherein the 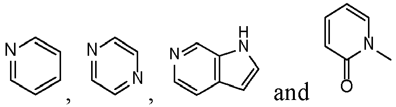 are optionally substituted with 1, 2 or 3 R_{b}, and other variables are as defined herein.

In some embodiments of the present disclosure, the above-mentioned R₄ is selected from hydrogen, -C(O)R₄₁, 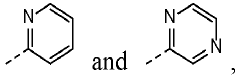 wherein the 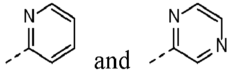 are optionally substituted with 1, 2 or 3 R_{b}, and other variables are as defined herein.

In some embodiments of the present disclosure, the above-mentioned R_{b} is selected from hydrogen, deuterium, fluorine, CN, NH₂, methyl, ethyl, methoxy and ethoxy, and other variables are as defined herein.

In some embodiments of the present disclosure, the above-mentioned R_{b} is selected from hydrogen, fluorine, CN and methyl, and other variables are as defined herein.

In some embodiments of the present disclosure, the above-mentioned R_{b} is selected from hydrogen, and other variables are as defined herein.

In some embodiments of the present disclosure, the above-mentioned R_{b} is selected from fluorine, and other variables are as defined herein.

In some embodiments of the present disclosure, the above-mentioned R_{b} is selected from CN, and other variables are as defined herein.

In some embodiments of the present disclosure, the above-mentioned R_{b} is selected from methyl, and other variables are as defined herein.

In some embodiments of the present disclosure, the above-mentioned R_{b} is selected from methoxy, and other variables are as defined herein.

In some embodiments of the present disclosure, the above-mentioned R₄₁ is selected from methyl, ethyl, propyl, C₃₋₈ cycloalkyl, 5- to 6-membered heteroaryl and 4- to 6-membered heterocycloalkyl, wherein the methyl, ethyl, propyl, C₃₋₈ cycloalkyl, 5- to 6-membered heteroaryl and 4- to 6-membered heterocycloalkyl are optionally substituted with 1, 2, 3 or 4 R_{c}, and other variables are as defined herein.

In some embodiments of the present disclosure, the above-mentioned R₄₁ is selected from methyl, ethyl, propyl and C₃₋₄ cycloalkyl, wherein the methyl, ethyl, propyl and C₃₋₄ cycloalkyl are optionally substituted with 1, 2, 3 or 4 R_{c}, and other variables are as defined herein.

In some embodiments of the present disclosure, the above-mentioned C₃₋₄ cycloalkyl is selected from cyclopropyl and cyclobutyl, wherein the cyclopropyl and cyclobutyl are optionally substituted with 1, 2, 3 or 4 R_{c}, and other variables are as defined herein.

In some embodiments of the present disclosure, the above-mentioned R₄₁ is selected from methyl, ethyl, propyl, cyclopropyl, cyclobutyl, 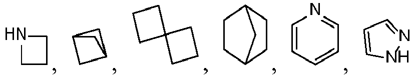 and 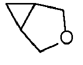 wherein the methyl, ethyl, propyl, cyclopropyl, cyclobutyl, 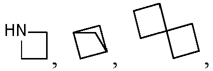 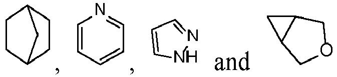 are optionally substituted with 1, 2, 3 or 4 R_{c}, and other variables are as defined herein.

In some embodiments of the present disclosure, the above-mentioned R₄₁ is selected from cyclopropyl and cyclobutyl, and other variables are as defined herein.

In some embodiments of the present disclosure, the above-mentioned R_{c} is hydrogen, and other variables are as defined herein.

In some embodiments of the present disclosure, the above-mentioned R₄₁ is selected from methyl, ethyl, propyl, cyclopropyl, cyclobutyl, 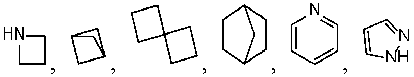 and 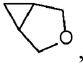 and other variables are as defined herein.

In some embodiments of the present disclosure, the above-mentioned R₄₂ is selected from hydrogen, and other variables are as defined herein.

In some embodiments of the present disclosure, the above-mentioned R₄₃ is selected from methyl, ethyl, propyl, cyclopropyl, and cyclobutyl, wherein the methyl, ethyl, propyl, cyclopropyl and cyclobutyl are optionally substituted with 1, 2, 3 or 4 R_{c}, and other variables are as defined herein.

In some embodiments of the present disclosure, the above-mentioned R₄₃ is selected from cyclopropyl, and other variables are as defined herein.

In some embodiments of the present disclosure, the above-mentioned R₄ is selected from 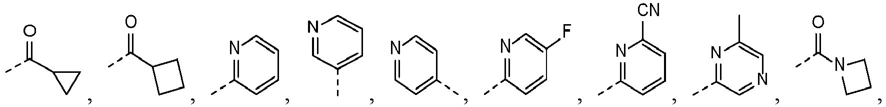 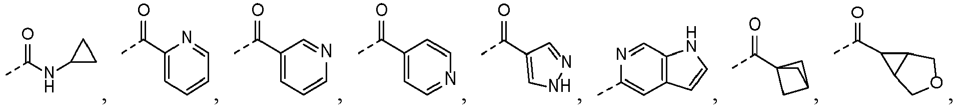 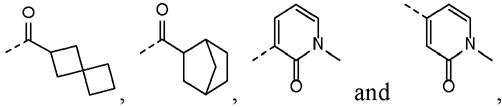 and other variables are as defined herein.

In some embodiments of the present disclosure, the above-mentioned R₄ is selected from 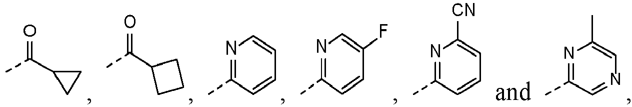 and other variables are as defined herein.

In some embodiments of the present disclosure, the above-mentioned R₅ is selected from hydrogen, methyl and ethyl, and other variables are as defined herein.

In some embodiments of the present disclosure, the above-mentioned R₅ is selected from hydrogen, and other variables are as defined herein.

In some embodiments of the present disclosure, the above-mentioned R₅ is selected from methyl, and other variables are as defined herein.

In some embodiments of the present disclosure, the above-mentioned R₆ is selected from methyl, ethyl, propyl, methoxy, ethoxy, cyclopropyl, cyclobutyl, -NHCH₃, -NHCH₂CH₃, -NH-cyclopropyl and -NH-cyclobutyl, wherein the methyl, ethyl, propyl, methoxy, ethoxy, cyclopropyl, cyclobutyl, -NHCH₃, -NHCH₂CH₃, -NH-cyclopropyl and -NH-cyclobutyl are optionally substituted with 1, 2, 3 or 4 R_{d}, and other variables are as defined herein.

In some embodiments of the present disclosure, the above-mentioned R₆ is selected from methyl, ethyl, -NHCH₃ and cyclopropyl, wherein the methyl, ethyl, -NHCH₃ and cyclopropyl are optionally substituted with 1, 2, 3 or 4 R_{d}, and other variables are as defined herein.

In some embodiments of the present disclosure, the above-mentioned R₆ is selected from methyl, ethyl and -NHCH₃, wherein the methyl, ethyl and -NHCH₃ are optionally substituted with 1, 2, 3 or 4 Rₐ, and other variables are as defined herein.

In some embodiments of the present disclosure, the above-mentioned R_{d} is selected from hydrogen, and other variables are as defined herein.

In some embodiments of the present disclosure, the above-mentioned R_{d} is selected from deuterium, and other variables are as defined herein.

In some embodiments of the present disclosure, the above-mentioned R_{d} is selected from methoxy, and other variables are as defined herein.

In some embodiments of the present disclosure, the above-mentioned R₆ is selected from -CH₂CD₃, -CH₂CH₃, -NHCD₃, -CH₂OCH₃, -CH₃ and cyclopropyl, and other variables are as defined herein.

In some embodiments of the present disclosure, the above-mentioned R₆ is selected from -CH₂CD₃, -CH₂CH₃, -NHCD₃ and -CH₂OCH₃, and other variables are as defined herein.

In some embodiments of the present disclosure, the compound is represented by formula (II-1) or (II-2): 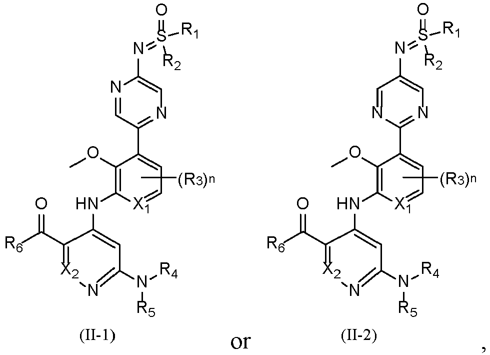 wherein R₁, R₂, R₃, R₄, R₅, R₆, X₁, X₂ and n are as defined herein.

In some embodiments of the present disclosure, the compound is represented by formula (11-1-1) or (II-1-2): 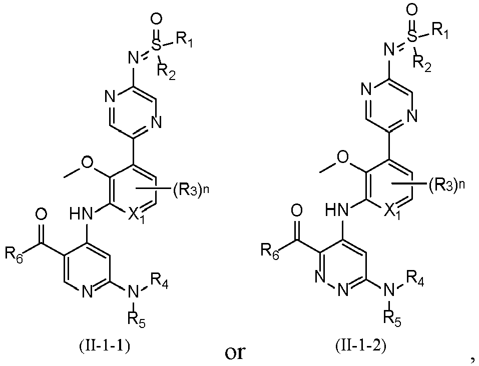 wherein R₁, R₂, R₃, R₄, R₅, R₆, X₁ and n are as defined herein.

In some embodiments of the present disclosure, the compound is represented by formula (II-1-1-1) or (II-1-1-2): 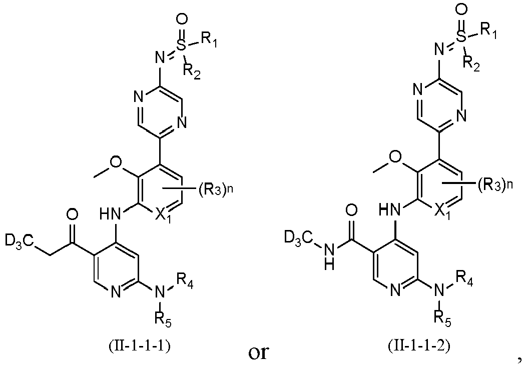 wherein R₁, R₂, R₃, R₄, R₅, X₁ and n are as defined herein.

The present disclosure also includes some embodiments obtained by any combination of the above variables.

In some embodiments of the present disclosure, the above-mentioned compound is selected from

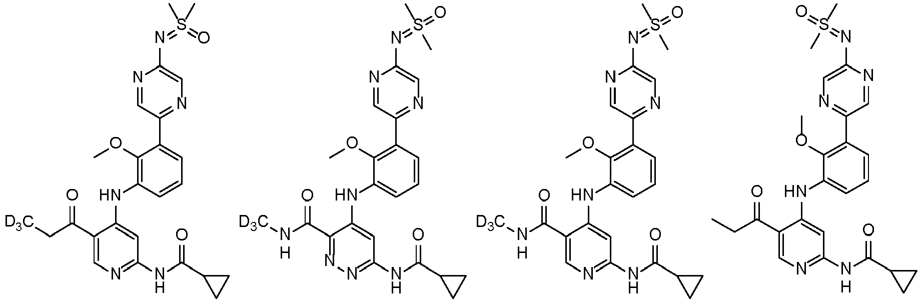

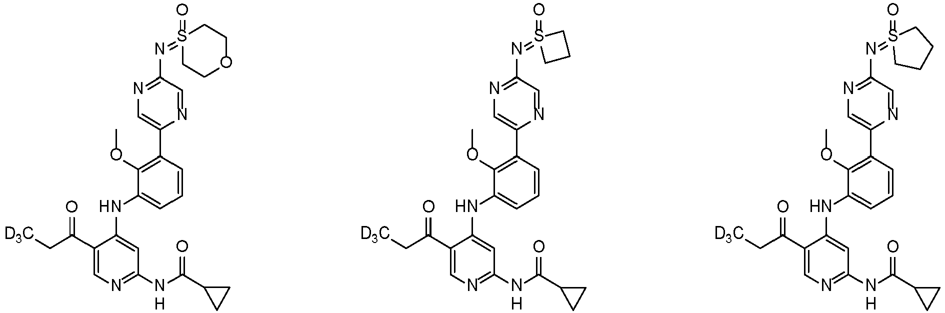

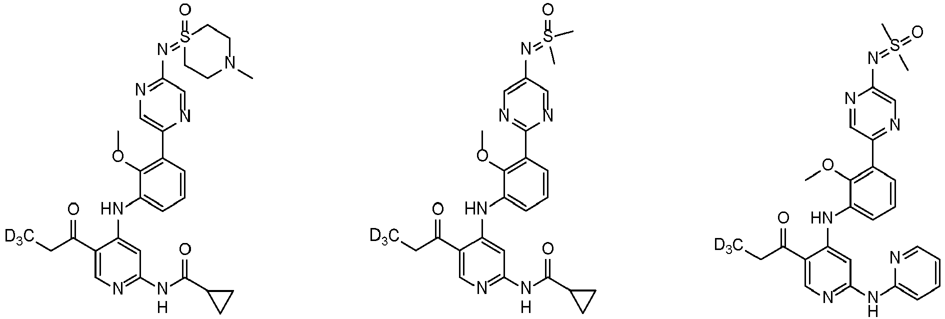

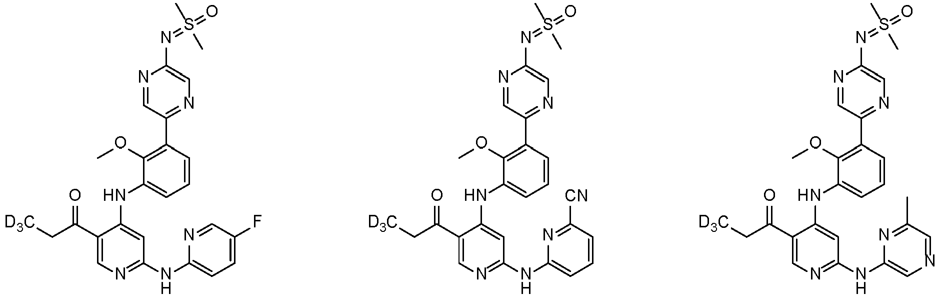

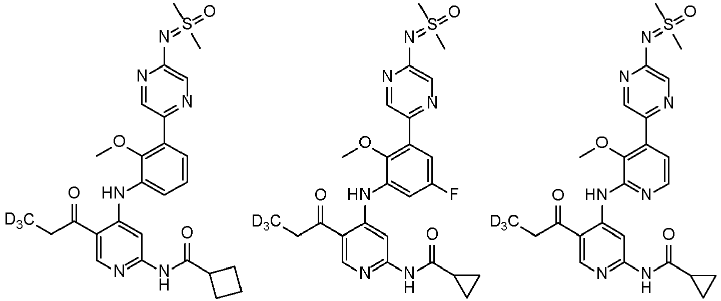

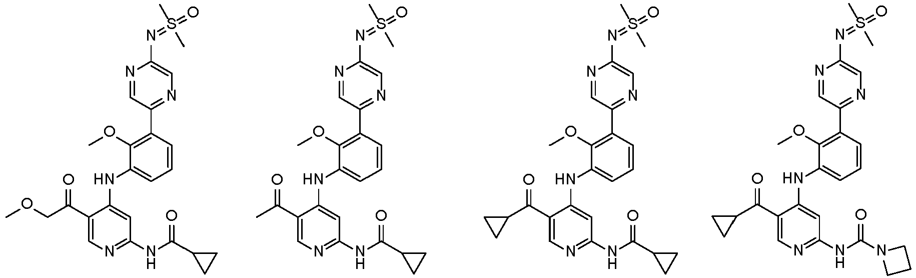

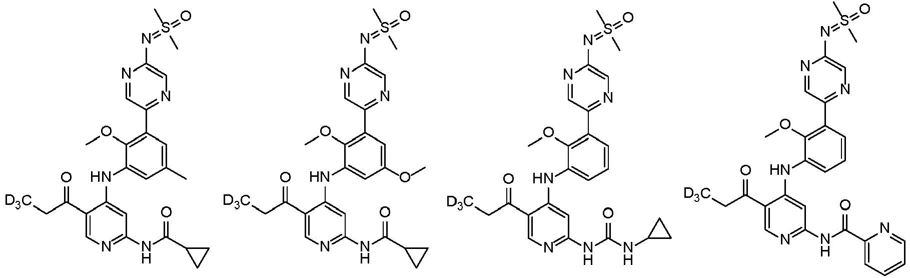

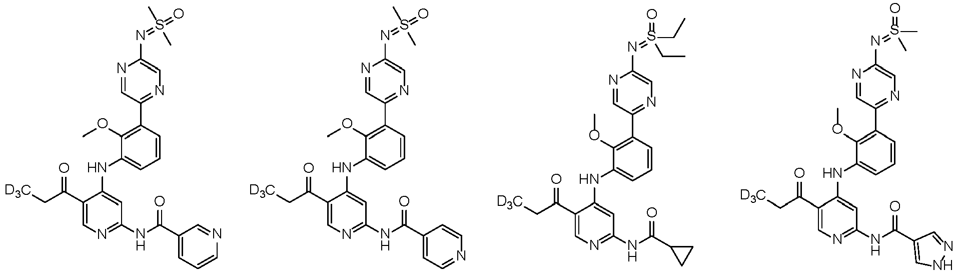

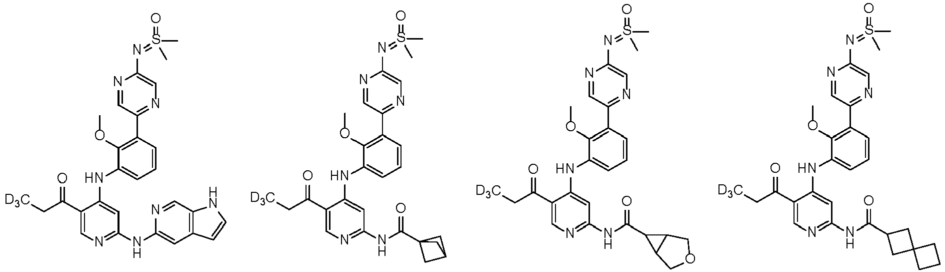

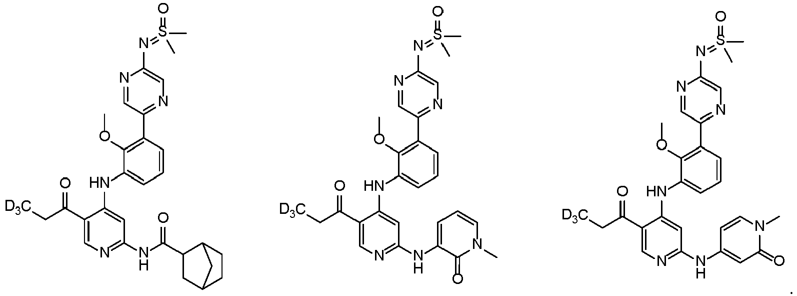

The present disclosure also provides a use of the above-mentioned compounds or pharmaceutically acceptable salts thereof in the manufacture of a medicament for the treatment of diseases related to Tyk2 JH2.

The present disclosure also provides a method for treating diseases related to Tyk2 JH2 in a subject in need thereof, comprising providing to the subject an effective dose of the compound defined in any of the above-mentioned technical solutions or a pharmaceutically acceptable salt thereof.

### Technical effect

The compounds of the present disclosure have strong inhibitory activity against the Tyk2 pseudokinase region (Tyk2 JH2).

### Definition and term

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" means a salt of compounds disclosed herein that is prepared by reacting the compound having a specific substituent disclosed herein with a relatively non-toxic acid or base. When compounds disclosed herein contain a relatively acidic functional group, a base addition salt can be obtained by bringing the compound into contact with a sufficient amount of base in a pure solution or a suitable inert solvent. When compounds disclosed herein contain a relatively basic functional group, an acid addition salt can be obtained by bringing the compound into contact with a sufficient amount of acid in a pure solution or a suitable inert solvent. Certain specific compounds disclosed herein contain both basic and acidic functional groups and can be converted to any base or acid addition salt.

The pharmaceutically acceptable salt disclosed herein can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

Compounds disclosed herein may be present in a specific geometric or stereoisomeric form. The present disclosure contemplates all such compounds, including cis and trans isomers, (-)- and (+)-enantiomers, (R)- and (S)-enantiomers, diastereoisomer, (D)-isomer, (L)-isomer, and a racemic mixture and other mixtures, for example, a mixture enriched in enantiomer or diastereoisomer, all of which are encompassed within the scope disclosed herein. The substituent such as alkyl may have an additional asymmetric carbon atom. All these isomers and mixtures thereof are encompassed within the scope disclosed herein.

Unless otherwise specified, the term "enantiomer" or "optical isomer" means stereoisomers that are in a mirrored relationship with each other.

Unless otherwise specified, the term "cis-trans isomer" or "geometric isomer" is generated by the inability of a double bond or a single bond between ring-forming carbon atoms to rotate freely.

Unless otherwise specified, the term "diastereomer" means a stereoisomer in which two or more chiral centers of are contained in a molecule and is in a non-mirrored relationship between molecules.

Unless otherwise specified, "(+)" means dextroisomer, "(-)" means levoisomer, and "(±)" means racemate.

Unless otherwise specified, a wedged solid bond ( 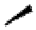 ) and a wedged dashed bond ( 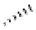 ) indicate the absolute configuration of a stereocenter; a straight solid bond ( 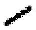 ) and a straight dashed bond ( 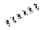 ) indicate the relative configuration of a stereocenter; a wavy line ( 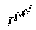 ) indicates a wedged solid bond ( 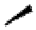 ) or a wedged dashed bond ( 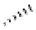 ); or a wavy line ( 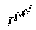 ) indicates a straight solid bond ( 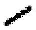 ) and a straight dashed bond ( 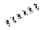 ).

Unless otherwise specified, the terms "tautomer" or "tautomeric form" means that different functional groups in an isomer are in dynamic equilibrium and can be rapidly converted into each other at room temperature. If tautomers are possible (as in solution), a chemical equilibrium of tautomers can be achieved. For example, proton tautomers (also known as prototropic tautomers) include interconversions by proton transfer, such as keto-enol isomerization and imine-enamine isomerization. Valence tautomers include interconversions by recombination of some bonding electrons. A specific example of keto-enol tautomerization is interconversion between two tautomers pentane-2,4-dione and 4-hydroxypent-3-en-2-one.

Unless otherwise specified, the term "enriched in one isomer", "isomer enriched", "enriched in one enantiomer" or "enantiomeric enriched" means that the content of one isomer or enantiomer is less than 100%, and the content of the isomer or enantiomer is 60% or more, or 70% or more, or 80% or more, or 90% or more, or 95% or more, or 96% or more, or 97% or more, or 98% or more, or 99% or more, or 99.5% or more, or 99.6% or more, or 99.7% or more, or 99.8% or more, or 99.9% or more.

Unless otherwise specified, the term "isomer excess" or "enantiomeric excess" means the difference between the relative percentages of two isomers or two enantiomers. For example, if one isomer or enantiomer is present in an amount of 90% and the other isomer or enantiomer is present in an amount of 10%, the isomer or enantiomeric excess (ee value) is 80%.

Optically active (*R*)- and (*S*)-isomer, or *D* and *L* isomer can be prepared using chiral synthesis or chiral reagents or other conventional techniques. If one kind of enantiomer of certain compound disclosed herein is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to afford the pure enantiomer. In addition, the enantiomer and the diastereoisomer are generally isolated through chromatography which uses a chiral stationary phase and optionally combines with a chemical derivative method (for example, carbamate generated from amine).

Compounds disclosed herein may contain an unnatural proportion of atomic isotopes at one or more of atoms that make up the compounds. For example, a compound may be labeled with a radioisotope such as tritium (³H), iodine-125 (¹²⁵I) or C-14(¹⁴C). For another example, hydrogen can be replaced by heavy hydrogen to form a deuterated drug. The bond between deuterium and carbon is stronger than that between ordinary hydrogen and carbon. Compared with undeuterated drugs, deuterated drugs have advantages of reduced toxic side effects, increased drug stability, enhanced efficacy, and prolonged biological half-life of drugs. All changes in the isotopic composition of compounds disclosed herein, regardless of radioactivity, are included within the scope of the present disclosure.

Unless otherwise specified, the term "D" or "²H" refers to deuterium, another stable form of hydrogen isotope, also known as heavy hydrogen.

The term "optional" or "optionally" means that the subsequently described event or condition may but not necessarily occur, and that the description includes instances in which the event or condition occurs and instances in which the event or condition does not occur.

The term "substituted" means that one or more hydrogen atoms on a specific atom are substituted by a substituent, including deuterium and hydrogen variants, as long as the valence of the specific atom is normal and the substituted compound is stable. When the substituent is oxo (i.e., =O), it means that two hydrogen atoms are substituted. Positions on an aromatic ring cannot be substituted by oxo.

The term "optionally substituted" means that an atom can be substituted or not. Unless otherwise specified, the species and number of a substituent may be arbitrary so long as being chemically achievable.

When any variable (such as R) occurs in the constitution or structure of the compound more than once, the definition of the variable at each occurrence is independent. Thus, for example, if a group is substituted by 0-2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of variables is a single bond, it means that the two groups linked by the single bond are connected directly. For example, when L in A-L-Z represents a single bond, it means that the structure of A-L-Z is actually A-Z.

When a substituent is vacant, it means that the substituent does not exist. For example, when X is vacant in A-X, the structure of A-X is actually A. When an enumerative substituent does not indicate through which atom it is linked to the substituted group, such substituent can be bonded through any of its atoms. For example, a pyridyl group as a substituent may be linked to the substituted group through any one of carbon atoms on the pyridine ring.

Unless otherwise specified, the terms "6-membered heteroaromatic ring" and "6-membered heteroaryl" may be used interchangeably. The term "6-membered heteroaryl" means a monocyclic group having a conjugated pi electron system and composed of 6 ring atoms, in which 1, 2, 3 or 4 ring atoms are heteroatoms independently selected from O, S and N, and the remainder ring atoms are carbon atoms, wherein the nitrogen atom is optionally quaternized and the nitrogen and sulfur heteroatoms may be optionally oxidized (i.e., NO and S(O)ₚ, wherein p is 1 or 2). A 6-membered heteroaryl can be attached to the remainder of a molecule through a heteroatom or a carbon atom. Examples of 6-membered heteroaryl include, but are not limited to, pyridyl (including 2-pyridyl, 3-pyridyl, 4-pyridyl, etc.), pyrazinyl or pyrimidinyl (including 2-pyrimidinyl and 4-pyrimidinyl, etc.).

Unless otherwise specified, the term "C₁₋₃ alkyl" is used to indicate a linear or branched saturated hydrocarbon group composed of 1 to 3 carbon atoms. C₁₋₃ alkyl group includes C₁₋₂ and C₂₋₃ alkyl groups and the like. It may be monovalent (e.g., methyl), divalent (e.g., methylene) or multivalent (e.g., methenyl). Examples of C₁₋₃ alkyl groups include, but are not limited to, methyl (Me), ethyl (Et), propyl (including n-propyl and isopropyl), and the like.

Unless otherwise specified, the term "C₁₋₃ alkoxy" means alkyl groups containing 1 to 3 carbon atoms and attached to the remainder of a molecule by an oxygen atom. C₁₋₃ alkoxy group includes C₁₋₂, C₂₋₃, C₃, and C₂ alkoxy groups, and the like. Examples of C₁₋₃ alkoxy groups include, but are not limited to, methoxy, ethoxy, propoxy (including n-propoxy and isopropoxy), and the like.

Unless otherwise specified, the term "4- to 6-membered heterocycloalkyl" alone or in combination with other terms respectively means a saturated cyclic group composed of 4 to 6 ring atoms, in which 1, 2, 3 or 4 ring atoms are heteroatoms independently selected from O, S and N, and the remainder ring atoms are carbon atoms, wherein the nitrogen atom is optionally quaternized and the nitrogen and sulfur heteroatoms may be optionally oxidized (i.e., NO and S(O)ₚ, wherein p is 1 or 2). The 4- to 6-membered heterocycloalkyl comprises a single ring system and a double ring system, wherein the double ring system comprises a sprio-ring, a fused-ring, and a bridge-ring. In addition, with respect to the "4- to 6-membered heterocycloalkyl", the heteroatom may be present on the position of attachment of the heterocycloalkyl group to the remainder of a molecule. The 4- to 6-membered heterocycloalkyl group includes 5- to 6-membered, 4-membered, 5-membered, and 6-membered heterocycloalkyl groups, and the like. Examples of 4- to 6-membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl and tetrahydrothien-3-yl and the like), tetrahydrofuranyl (including tetrahydrofuran-2-yl and the like), tetrahydropyranyl, piperidinyl (including 1-piperidinyl, 2-piperidinyl and 3-piperidinyl and the like), piperazinyl (including 1-piperazinyl and 2-piperazinyl and the like), morpholinyl (including 3-morpholinyl and 4-morpholinyl and the like), dioxanyl, dithianyl, isoxazolidinyl, isothiazolidinyl, 1,2-oxazinyl, 1,2-thiazinyl, hexahydropyridazinyl, homopiperazinyl, or homopiperidinyl and the like.

Unless otherwise specified, the terms "5- to 10-membered heteroaromatic ring" and "5- to 10-membered heteroaryl" may be used interchangeably. The term "5- to 10-membered heteroaryl" means a cyclic group having a conjugated pi electron system and composed of 5 to 10 ring atoms, in which 1, 2, 3 or 4 ring atoms are heteroatoms independently selected from O, S and N, and the remainder ring atoms are carbon atoms. It may be a monocyclic, fused bicyclic or fused tricyclic ring system, wherein each ring is aromatic, and wherein the nitrogen atom is optionally quaternized and the nitrogen and sulfur heteroatoms may be optionally oxidized (i.e., NO and S(O)ₚ, wherein p is 1 or 2). A 5- to 10-membered heteroaryl can be attached to the remainder of a molecule through a heteroatom or a carbon atom. The 5- to 10-membered heteroaryl group includes 5- to 8-membered, 5- to 7-membered, 5- to 6-membered, 5-membered and 6-membered heteroaryl groups, and the like. Examples of the 5-10 membered heteroaryl include, but are not limited to, pyrrolyl (including N-pyrrolyl, 2-pyrrolyl, 3-pyrrolyl, and the like), pyrazolyl (including 2-pyrazolyl and 3-pyrazolyl, and the like), imidazolyl (including N-imidazolyl, 2-imidazolyl, 4-imidazolyl, and 5-imidazolyl, and the like), oxazolyl (including 2-oxazolyl, 4-oxazolyl, and 5-oxazolyl, and the like), triazolyl (1H-1,2,3-triazolyl, 2H-1,2,3-triazolyl, 1H-1,2,4-triazolyl and 4H-1,2,4-triazolyl, and the like), tetrazolyl, isoxazolyl (3-isoxazolyl, 4-isoxazolyl and 5-isoxazolyl, and the like), thiazolyl (including 2-thiazolyl, 4-thiazolyl and 5-thiazolyl, and the like), furyl (including 2-furyl and 3-furyl, and the like), thienyl (including 2-thienyl and 3-thienyl, and the like), pyridyl (including 2-pyridyl, 3-pyridyl and 4-pyridyl, and the like), pyrazinyl or pyrimidinyl (including 2-pyrimidinyl and 4-pyrimidinyl, and the like), benzothiazolyl (including 5-benzothiazolyl, and the like), purinyl, benzimidazolyl (including 2-benzimidazolyl, and the like), benzoxazolyl, indolyl (including 5-indolyl, and the like), isoquinolyl (including 1-isoquinolyl, 5-isoquinolyl, and the like), quinoxalinyl (including 2-quinoxalinyl, 5-quinoxalinyl, and the like) or quinolyl (including 3-quinolyl, 6-quinolyl, and the like), substituted or unsubstituted pyridonyl (such as 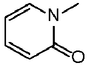 or

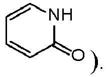

Unless otherwise specified, the terms "5- to 6-membered heteroaromatic ring" and "5-to 6-membered heteroaryl" may be used interchangeably. The term "5- to 6-membered heteroaryl" means a monocyclic group having a conjugated pi electron system and composed of 5 to 6 ring atoms, in which 1, 2, 3 or 4 ring atoms are heteroatoms independently selected from O, S and N, and the remainder ring atoms are carbon atoms, wherein the nitrogen atom is optionally quaternized and the nitrogen and sulfur heteroatoms may be optionally oxidized (i.e., NO and S(O)ₚ, wherein p is 1 or 2). The 5- to 6-membered heteroaryl group may be attached to the remainder of a molecule by a heteroatom or a carbon atom. The 5- to 6-membered heteroaryl group includes 5-membered and 6-membered heteroaryl groups. Examples of the 5-to 6-membered heteroaryl group include, but are not limited to, pyrrolyl (including N-pyrrolyl, 2-pyrrolyl, 3-pyrrolyl, and the like), pyrazolyl (including 2-pyrazolyl and 3-pyrazolyl, and the like), imidazolyl (including N-imidazolyl, 2-imidazolyl, 4-imidazolyl, and 5-imidazolyl, and the like), oxazolyl (including 2-oxazolyl, 4-oxazolyl, and 5-oxazolyl, and the like), triazolyl (1H-1,2,3-triazolyl, 2H-1,2,3-triazolyl, 1H-1,2,4-triazolyl and 4H-1,2,4-triazolyl, and the like), tetrazolyl, isoxazolyl (3-isoxazolyl, 4-isoxazolyl and 5-isoxazolyl, and the like), thiazolyl (including 2-thiazolyl, 4-thiazolyl and 5-thiazolyl, and the like), furyl (including 2-furyl and 3-furyl, and the like), thienyl (including 2-thienyl and 3-thienyl, and the like), pyridyl (including 2-pyridyl, 3-pyridyl and 4-pyridyl, and the like), pyrazinyl or pyrimidinyl (including 2-pyrimidinyl and 4-pyrimidinyl, and the like).

Unless otherwise specified, "C₃₋₈ cycloalkyl" means a saturated cyclic hydrocarbon group composed of 3 to 8 carbon atoms, which includes monocyclic and bicyclic systems, wherein the bicyclic system includes spiro rings, fused rings and bridged rings. The C₃₋₈ cycloalkyl group includes C₃₋₆, C₃₋₅, C₄₋₈, C₄₋₆, C₄₋₅, C₅₋₈ or C₅₋₆ cycloalkyl group, etc.; it can be monovalent, divalent or multivalent. Examples of C₃₋₈ cycloalkyl include, but are not limited to, cyclopropyl, cyclobutyl, cyclopentyl, cyclohexyl, cycloheptyl, and the like.

Unless otherwise specified, "C₃₋₆ cycloalkyl" means a saturated cyclic hydrocarbon group composed of 3 to 6 carbon atoms, which is a monocyclic or bicyclic system. C₃₋₆ cycloalkyl includes C₃₋₅, C₄₋₅ and C₅₋₆ cycloalkyl, etc.; it can be monovalent, divalent or multivalent. Examples of C₃₋₆ cycloalkyl include, but are not limited to, cyclopropyl, cyclobutyl, cyclopentyl, cyclohexyl, and the like.

Unless otherwise specified, "C₃₋₄ cycloalkyl" means a saturated cyclic hydrocarbon group composed of 3 to 4 carbon atoms, which is a monocyclic system. C₃₋₅ cycloalkyl includes C₃ and C₄ cycloalkyl, etc.; it may be monovalent, divalent or multivalent. Examples of C₃₋₄ cycloalkyl include, but are not limited to, cyclopropyl and cyclobutyl. The term "leaving group" refers to a functional group or atom that can be replaced by another functional group or atom through a substitution reaction (such as a nucleophilic substitution reaction). For example, representative leaving groups include triflate; chlorine, bromine and iodine; sulfonate group, such as mesylate, tosylate, p-bromobenzenesulfonate, p-toluenesulfonate and the like; acyloxy, such as acetoxy, trifluoroacetoxy and the like.

The term "protecting group" includes, but is not limited to "amino protecting group", "hydroxy protecting group" or "thio protecting group". The term "amino protecting group" refers to a protecting group suitable for blocking a side reaction on the nitrogen of an amino. Representative amino protecting groups include, but are not limited to: formyl; acyl, such as alkanoyl (e.g. acetyl, trichloroacetyl or trifluoroacetyl); alkoxycarbonyl, such as tert-butoxycarbonyl (Boc); arylmethoxycarbonyl such as benzyloxycarbonyl (Cbz) and 9-fluorenylmethoxycarbonyl (Fmoc); arylmethyl such as benzyl (Bn), trityl (Tr), 1,1-bis-(4'-methoxyphenyl)methyl; silyl such as trimethylsilyl (TMS) and tert-butyldimethylsilyl (TBS) and the like. The term "hydroxy protecting group" refers to a protecting group suitable for blocking a side reaction on hydroxy. Representative hydroxy protecting groups include, but are not limited to: alkyl such as methyl, ethyl and tert-butyl; acyl such as alkanoyl (e.g. acetyl); arylmethyl such as benzyl (Bn), p-methoxybenzyl (PMB), 9-fluorenylmethyl (Fm), and diphenylmethyl (benzhydryl, DPM); silyl such as trimethylsilyl (TMS) and tert-butyl dimethyl silyl (TBS) and the like.

Compounds disclosed herein can be prepared by a variety of synthetic methods well known to those skilled in the art, including the following enumerated embodiment, the embodiment formed by the following enumerated embodiment in combination with other chemical synthesis methods, and equivalent replacement well known to those skilled in the art. Alternative embodiments include, but are not limited to examples disclosed herein.

The structures of compounds disclosed herein can be confirmed by conventional methods well known to those skilled in the art. If the present disclosure relates to an absolute configuration of a compound, the absolute configuration can be confirmed by conventional techniques in the art, such as single crystal X-ray diffraction (SXRD). In the single crystal X-ray diffraction (SXRD), the diffraction intensity data of the cultivated single crystal is collected using a Bruker D8 venture diffractometer with a light source of CuKα radiation in a scanning mode of ϕ/ω scan; after collecting the relevant data, the crystal structure is further analyzed by the direct method (Shelxs97) to confirm the absolute configuration.

Compounds are named according to general naming principles in the art or by ChemDraw^{®} software, and commercially available compounds are named with their vendor directory names.

### DETAILED DESCRIPTION OF THE INVENTION

The present disclosure is described in detail below by means of examples. However, it is not intended that these examples have any disadvantageous limitations to the present disclosure. The present disclosure has been described in detail herein, and embodiments are also disclosed herein. It will be apparent to those skilled in the art that various changes and modifications may be made to the embodiments disclosed herein without departing from the spirit and scope disclosed herein.

### Example 1

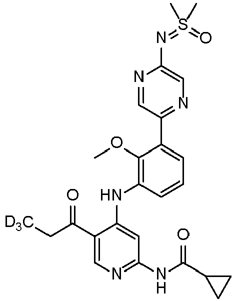

### Synthetic route:

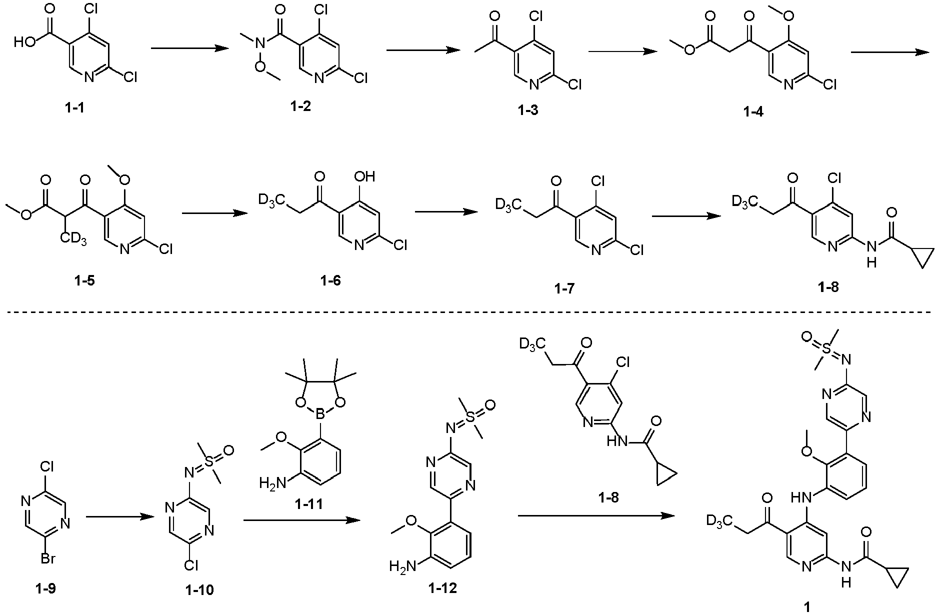

### Step 1: Synthesis of compound 1-2

To a solution of compound **1-1** (13 g, 67.71 mmol) in dichloromethane (200 mL) were added N,N-diisopropylethylamine (43.75 g, 338.54 mmol) and O-(7-azabenzotriazole-1-yl)-N,N,N,N-tetramethyluronium hexafluorophosphate (30.89 g, 81.25 mmol), and the mixture was stirred at 20 °C for 0.5 hours. N,O-dimethylhydroxylamine hydrochloride (7.93 g, 81.25 mmol) was added. After stirring at 20 °C for 15.5 hours, the reaction solution was concentrated, and the resulting crude product was purified by column chromatography (silica gel, petroleum ether/ethyl acetate = 10/1 - 3/1) to give compound **1-2.**

MS *m*/*z*: 235[M+H]⁺.

### Step 2: Synthesis of compound 1-3

To a solution of compound **1-2** (13 g, 55.30 mmol) in tetrahydrofuran (130 mL) was added methylmagnesium bromide (3M, a solution in diethyl ether, 36.87 mL) under nitrogen at 0 °C, and the mixture was stirred at 0 °C for 2 hours. The reaction solution was quenched with saturated aqueous ammonium chloride solution (60 mL), diluted with water (100 mL), and then extracted with ethyl acetate (100 mL×2). The organic phases were combined, washed with brine (100 mL×2), dried over anhydrous sodium sulfate, and filtered. The filtrate was concentrated under reduced pressure to give a crude product, which was purified by column chromatography (silica gel, petroleum ether/ethyl acetate = 20/1 - 15/1) to give compound 1-**3.**

¹H NMR (400 MHz, CDCl₃) δ 8.59 (s, 1H), 7.44 (s, 1H), 2.66 (s, 3H).

### Step 3: Synthesis of compound 1-4

To a solution of **1-3** (8 g, 42.10 mmol) and dimethyl carbonate (42.80 g, 475.15 mmol) in tetrahydrofuran (40 mL) was slowly added 60% sodium hydride (5.05 g, 126.30 mmol) in batches at 0 °C, and the mixture was stirred at 20 °C for 16 hours. The reaction solution was diluted with ethyl acetate (100 mL), quenched with aqueous hydrochloric acid (50 mL 2M), and washed with saturated brine (50 mL × 2). The organic phase was dried over anhydrous sodium sulfate, and filtered. The filtrate was concentrated under reduced pressure to give a crude product. The crude product was purified by column chromatography (silica gel, petroleum ether/ethyl acetate = 10/1 - 3/1) to give compound **1-4.**

MS *m*/*z*: 244[M+H]⁺.

### Step 4: Synthesis of compound 1-5

To a solution of **1-4** (8.2 g, 32.65 mmol) and potassium carbonate (4.96 g, 35.91 mmol) in N,N-dimethylformamide (80 mL) was added deuterated iodomethane (4.97g, 34.28 mmol) at 0 °C, and the mixture was stirred at 20 °C for 6 hours. The reaction solution was diluted with water (100 mL), and extracted with ethyl acetate (100 mL×2). The organic phases were combined, washed with saturated brine (100 mL×2), dried over anhydrous sodium sulfate, and filtered. The filtrate was concentrated under reduced pressure to give a crude product. The product was purified by column chromatography (silica gel, petroleum ether/ethyl acetate = 20/1 - 4/1) to give compound **1-5.**

MS *m*/*z*: 261[M+H]⁺.

### Step 5: Synthesis of compound 1-6

To a solution of **1-5** (5.8 g, 18.02 mmol, 81%) in acetic acid (30 mL) was added 35% concentrated hydrochloric acid (61.20 g, 587.48 mmol), and the mixture was stirred at 130 °C for 16 hours. The reaction solution was concentrated under reduced pressure to give a crude product, which was purified by column chromatography (silica gel, petroleum ether/ethyl acetate = 30/1 - 1/1) to give compound **1-6.**

MS *m*/*z*: 189[M+H]⁺.

### Step 6: Synthesis of compound 1-7

To a solution of **1-6** (2.5 g, 12.33 mmol) in acetonitrile (80 mL) was added phosphorus oxychloride (7.56 g, 49.30 mmol), and the mixture was stirred at 85 °C for 1 hour. The reaction solution was concentrated and diluted with ethyl acetate (100 mL). The organic phase was washed with saturated aqueous sodium bicarbonate solution (50 mL × 3), dried over anhydrous sodium sulfate, and filtered. The filtrate was concentrated under reduced pressure to give a crude product, which was purified by column chromatography (silica gel, petroleum ether/ethyl acetate = 1/0 - 50/1) to give compound **1-7.**

MS *m*/*z*: 207[M+H]⁺.

### Step 7: Synthesis of compound 1-8

A solution of compound **1-7** (50 mg, 241.46 µmol), cyclopropylcarboxamide (20.55 mg, 241.46 µmol), potassium carbonate (66.75 mg, 482.92 µmol), 4,5-bis(diphenylphosphino)-9,9-dimethylxanthene (27.94 mg, 48.29 µmol) and tris(dibenzylideneacetone)dipalladium chloroform complex (22.11 mg, 24.15 µmol) in dioxane (2 mL) was purged three times with nitrogen. The mixture was stirred at 80 °C for 2 hours. The reaction solution was concentrated to give a crude product, which was purified by column chromatography (silica gel, petroleum ether/ethyl acetate = 20/1 - 10/1) to give compound **1-8.**

MS *m*/*z*: 256[M+H]⁺.

### Step 8: Synthesis of compound 1-10

Compound **1-9** (2 g, 10.34 mmol) was dissolved in dioxane (40 mL). Dimethylsulfoximine (1.01 g, 10.86 mmol), cesium carbonate (6.74 g, 20.68 mmol), tris(dibenzylideneacetone)dipalladium (946.85 mg, 1.03 mmol) and 4,5-bis(diphenylphosphino)-9,9-dimethylxanthene (1.20 g, 2.07 mmol) were added. The atmosphere was replaced with nitrogen three times, and the mixture was heated to 110°C and stirred under nitrogen for 4 hours. The reaction solution was directly concentrated under reduced pressure to give a crude product, which was purified by column chromatography (silica gel, petroleum ether/ethyl acetate = 10/1 ~ 1/4). The fraction was concentrated under reduced pressure, and stirred with petroleum ether/ethyl acetate = 5/1 (12 mL) at 20 °C for 1 hour. The mixture was filtered, and the filter cake was collected and dried to give compound **1-10.**

MS *m*/*z*: 206[M+H]⁺.

### Step 9: Synthesis of compound 1-12

Compound **1-10** (400 mg, 1.94 mmol) was dissolved in dioxane (8 mL) and water (2 mL). Compound **1-11** (532.95 mg, 2.14 mmol), potassium phosphate (825.68 mg, 3.89 mmol), and 1,1-bis(diphenylphosphino)ferrocene palladium chloride (142.31 mg, 194.49 µmol) were added. The atmosphere was replaced with nitrogen three times, and the mixture was heated to 100 °C, and stirred under nitrogen for 2 hours. The reaction solution was directly concentrated under reduced pressure to give a crude product, which was purified by column chromatography (silica gel, petroleum ether/ethyl acetate = 5/1 - 0/1). The fraction was concentrated under reduced pressure, and stirred with petroleum ether/ethyl acetate = 1/1 (4 mL) at 20 °C for 1 hour. The mixture was filtered, and the filter cake was collected and dried to give compound **1-12.**

MS *m*/*z*: 293[M+H]⁺

### Step 10: Synthesis of compound 1;

A solution of compound **1-8** (70 mg, 273.74 µmol), compound **1-12** (88.03 mg, 301.12 µmol), cesium carbonate (178.38 mg, 547.48 µmol), 2,2-bis(diphenylphosphino)-1,1-binaphthyl (34.09 mg, 54.75 µmol) and tris(dibenzylideneacetone)dipalladium chloroform complex (25.07 mg, 27.37 µmol) in dioxane (4 mL) was purged with nitrogen three times, and the mixture was stirred at 110°C for 4 hours. The reaction solution was concentrated to give a crude product, which was purified by column chromatography (silica gel, petroleum ether/ethyl acetate = 3/1 ~ 1/3), and then separated by preparative high-performance liquid chromatography (column: Waters Xbridge 150* 25mm*5µm; mobile phase: [water (10mM ammonium bicarbonate)-acetonitrile]; acetonitrile %: 34%-64%, 8min) to give compound 1.

MS *m*/*z*: 512[M+H]⁺;

¹H NMR (400 MHz, DMSO-*d₆*) δ 11.03 (s, 1H), 10.92 (s, 1H), 8.88 (s, 1H), 8.59 (d, *J* = 1.3 Hz, 1H), 8.14 (d, *J* = 1.5 Hz, 1H), 8.03 (s, 1H), 7.53 (dd, *J* = 1.4, 7.8 Hz, 1H), 7.48 (dd, *J* = 1.3, 7.8 Hz, 1H), 7.32 - 7.25 (m, 1H), 3.52 (s, 3H), 3.47 (s, 6H), 3.11 (s, 2H), 2.06 - 1.96 (m, 1H), 0.79 (d, *J =* 6.1 Hz, 4H).

### Example 2

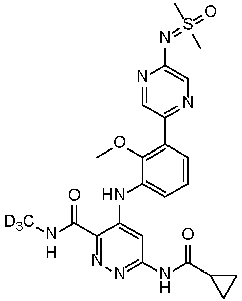

### Synthetic route:

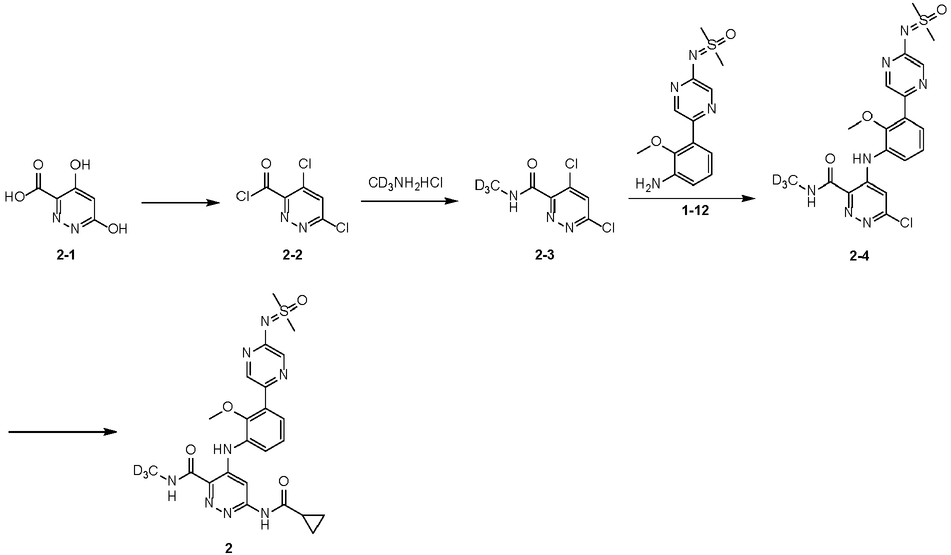

### Step 1: Synthesis of compound 2-2

Compound **2-1** (1 g, 6.41 mmol) was dissolved in phosphorus oxychloride (5 mL) at 20°C, and triethylamine (648.25 mg, 6.41 mmol, 891.68 µL) was added. The mixture was stirred at 110 °C for 120 minutes. The reaction solution was directly concentrated under reduced pressure, then diluted by adding 1,2-dichloroethane (40 mL) and concentrated under reduced pressure again to give a crude product **2-2,** which was directly used in the next reaction.

### Step 2: Synthesis of compound 2-3

Compound **2-2** (1.35 g, 6.38 mmol) was dissolved in tetrahydrofuran (15 mL) at 20°C, and deuterated methylamine hydrochloride (225.19 mg, 3.19 mmol) and N,N-diisopropylethylamine (2.48 g, 19.15 mmol, 3.34 mL) were added. The mixture was stirred at 20 °C for 16 hours. The reaction solution was diluted with water (20 mL), and extracted with ethyl acetate (40 mL×3). The combined organic phase was washed with saturated brine (5 mL×2), dried over anhydrous sodium sulfate, and filtered. The filtrate was concentrated under reduced pressure, and then purified by column chromatography (silica gel, petroleum ether/ethyl acetate = 10/1 - 5/1) to give a crude product. The crude product was separated by preparative HPLC (Waters Xbridge C18 150*50mm*10µm; mobile phase: [water (10mM ammonium bicarbonate)-acetonitrile]; acetonitrile%: 3%-33%, 11min) to give compound **2-3.**

MS *m*/*z*: 209[M+H]⁺; ¹H NMR (400 MHz, DMSO-d₆) δ 8.90 (br s, 1H), 8.48 (s, 1H).

### Step 3: Synthesis of compound 2-4

Compound **2-3** (55.06 mg, 263.38 µmol) and compound **1-12** (70 mg, 239.43 µmol) were dissolved in tetrahydrofuran (3 mL) at 0°C under nitrogen, and lithium methyldisilazide (1 M, 718.30 µL) was added dropwise. The mixture was stirred at 20 °C for 1 h. The reaction solution was quenched by adding aqueous ammonium chloride solution (10 mL) at 0 °C, diluted with water (10 mL), and extracted with ethyl acetate (20 mL×3). The combined organic phase was washed with saturated brine (5 mL×2), dried over anhydrous sodium sulfate, and filtered. The filtrate was concentrated under reduced pressure to give a crude product. The crude product was purified by thin layer chromatography (silica gel, petroleum ether/ethyl acetate = 1/5) to give compound **2-4.**

MS *m*/*z*: 465[M+H]⁺

### Step 4: Synthesis of compound 2

Compound **2-4** (75 mg, 161.31 µmol) was dissolved in dioxane (2 mL). Cyclopropylformamide (41.18 mg, 483.93 µmol), cesium carbonate (157.67 mg, 483.93 µmol) and (2-dicyclohexylphosphino)-3,6-dimethoxy-2',4',6'-triisopropyl-1,1'-biphenyl)(2'-amino-1,1'-biphenyl-2-yl)palladium (II) mesylate (14.62 mg, 16.13 µmol) were added. The atmosphere was replaced with nitrogen three times, then the mixture was heated to 110°C and stirred under nitrogen for 3 hours. The reaction solution was directly concentrated under reduced pressure to give a crude product, which was purified by column chromatography (silica gel, petroleum ether/ethyl acetate = 1/1 - 1/3.5) to give compound **2.**

MS *m*/*z*: 514[M+H]⁺;

¹H NMR (400 MHz, CDCl₃) δ 11.06 (s, 1H), 9.02 (br s, 1H), 8.74 (d, *J* = 1.2 Hz, 1H), 8.27 (d, *J* = 1.3 Hz, 1H), 8.24 (s, 1H), 8.12 (s, 1H), 7.59 (dd, *J* = 1.5, 7.8 Hz, 1H), 7.47 (dd, *J* = 1.2, 7.8 Hz, 1H), 7.29 (s, 1H), 3.60 (s, 3H), 3.44 (s, 6H), 1.75 - 1.70 (m, 1H), 1.15 - 1.10 (m, 2H), 0.96 - 0.90 (m, 2H).

### Example 3

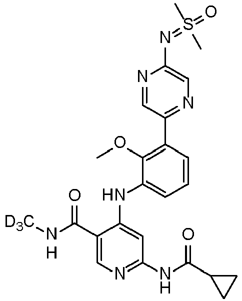

### Synthetic route:

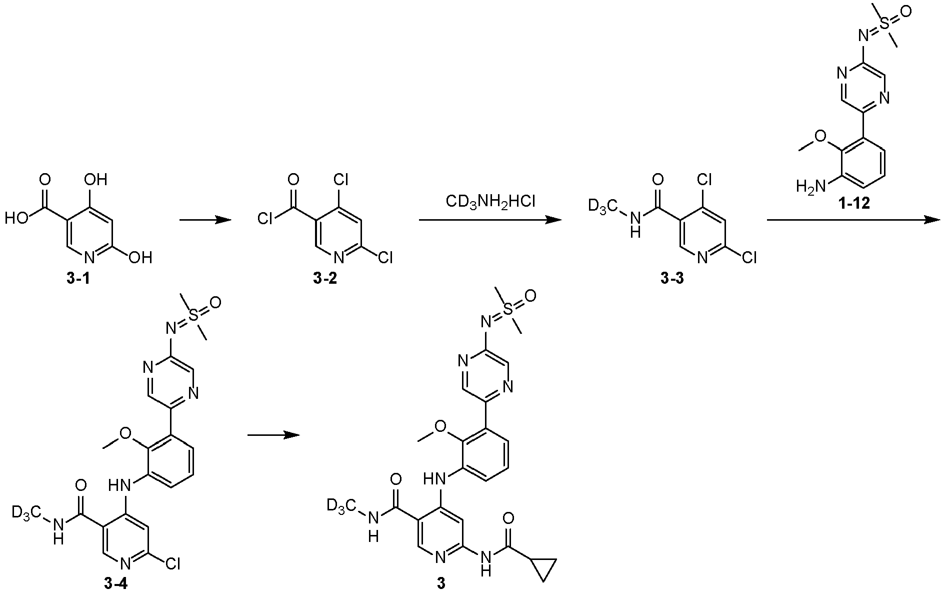

### Step 1: Synthesis of compound 3-2

Compound **3-1** (2 g, 10.42 mmol) was dissolved in dichloromethane (10 mL) at 0° C, and oxalyl chloride (1.98 g, 15.63 mmol, 1.37 mL) and N,N -dimethylformamide (19.00 mg, 259.94 µmol, 20 µL) were added. The mixture was stirred at 20 °C for 1 hour. TLC showed that the starting material was consumed completely. The reaction solution was directly concentrated under reduced pressure to give a crude product **3-2,** which was directly used in the next reaction.

### Step 2: Synthesis of compound 3-3

Compound **3-2** (804.43 mg, 11.40 mmol) was dissolved in dichloromethane (20 mL) at 0°C, and N,N-diisopropylethylamine (27.53 g, 213.04 mmol, 37.11 mL) and deuterated methylamine hydrochloride (2 g, 9.50 mmol) were added. The mixture was stirred at 15 °C for 2 hours. The reaction solution was diluted with water (20 mL), and extracted with ethyl acetate (20 mL×3). The combined organic phase was washed with saturated brine (5 mL×2), dried over anhydrous sodium sulfate, and filtered. The filtrate was concentrated under reduced pressure to give a crude product. The crude product was slurried with petroleum ether/ethyl acetate = 3/1 (10 mL) at 15°C for 1 hour, filtered and dried to give compound **3-3.**

MS *m*/*z*: 208[M+H]⁺; ¹H NMR (400 MHz, DMSO-d₆) δ 8.59 (br s, 1H), 8.48 (s, 1H), 7.90 (s, 1H).

### Step 3: Synthesis of compound 3-4

Compound **3-3** (55.00 mg, 264.35 µmol) and compound **1-12** (70.26 mg, 240.32 µmol) were dissolved in tetrahydrofuran (3 mL). Lithium hexamethyldisilazide (1 M, 720.95 µL) was added dropwise to the mixture under nitrogen at 0°C. The mixture was stirred at 20 °C for 1 hour. The reaction solution was quenched with saturated aqueous ammonium chloride solution (10 mL) in an ice-water bath, diluted with water (10 mL), and extracted with ethyl acetate (20 mL×3). The combined organic phase was washed with saturated brine (5 mL×2), dried over anhydrous sodium sulfate, and filtered. The filtrate was concentrated under reduced pressure to give a crude product. The crude product was stirred with ethyl acetate (2 mL) at 20°C for 0.5 hours, filtered and dried to give compound **3-4.**

MS *m*/*z*: 464[M+H]⁺.

### Step 4: Synthesis of compound 3

Compound **3-4** (80 mg, 172.43 µmol) was dissolved in dioxane (2 mL). Cyclopropylcarboxamide (44.02 mg, 517.29 µmol), cesium carbonate (168.54 mg, 517.29 µmol) and (2-dicyclohexylphosphino)-3,6-dimethoxy-2',4',6'-triisopropyl-1,1'-biphenyl)(2'-amino-1,1'-biphenyl-2-yl)palladium (II) mesylate (15.63 mg, 17.24 µmol) were added. The atmosphere was replaced with nitrogen three times, and then the mixture was heated to 110°C and stirred under nitrogen for 4 hours. The reaction solution was diluted with dichloromethane (50 mL), and extracted with water (5 mL×3). The organic phase was washed with saturated brine (5 mL×3), dried with anhydrous sodium sulfate, and filtered. The filtrate was concentrated under reduce pressure to give a crude product. The crude product was purified by thin layer chromatography (silica gel, ethyl acetate/ethanol = 10/1) to give compound **3.**

MS *m*/*z*: 513[M+H]⁺;

¹H NMR (400 MHz, DMSO-d₆) δ 10.77 (s, 1H), 10.66 (s, 1H), 8.66 - 8.56 (m, 2H), 8.52 (s, 1H), 8.14 (d, *J* = 1.1 Hz, 1H), 8.06 (s, 1H), 7.45 (br d, *J* = 7.5 Hz, 2H), 7.31 - 7.19 (m, 1H), 3.53 (s, 3H), 3.46 (s, 6H), 1.05 (t, *J* = 7.0 Hz, 1H), 0.77 (br d, *J* = 5.0 Hz, 4H).

### Example 4

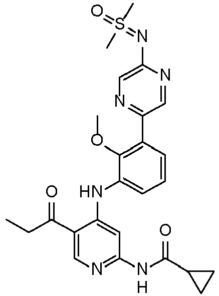

### Synthetic route:

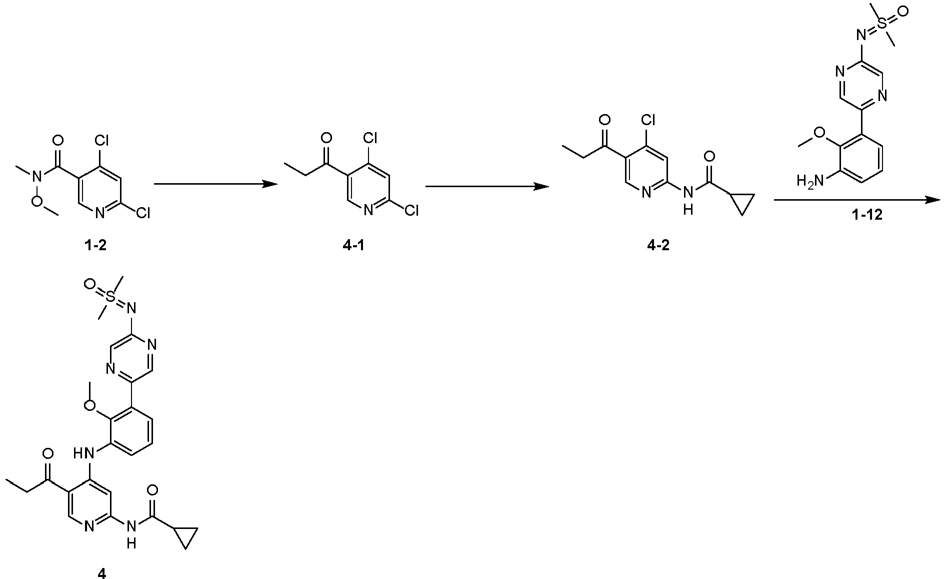

### Step 1: Synthesis of compound 4-1

Ethylmagnesium bromide (3M in diethyl ether, 4.25 mL) was added dropwise to a solution of compound **1-2** (1 g, 4.25 mmol) in tetrahydrofuran (20 mL) under nitrogen at 0°C, and the mixture was stirred at 20 °C for 3 hours. The reaction solution was quenched with saturated ammonium chloride (10 mL), and extracted with ethyl acetate (20 mL×2). The organic phases were combined, washed with brine (30 mL×1), dried over anhydrous sodium sulfate, and filtered. The filtrate was concentrated under reduced pressure to give a crude product, which was purified by column chromatography (silica gel, petroleum ether/ethyl acetate = 30/1 - 15/1) to give compound **4-1.**

MS *m*/*z*: 204[M+H]⁺

### Step 2: Synthesis of compound 4-2

A solution of compound **4-1** (390 mg, 1.74 mmol), cyclopropylcarboxamide (133.07 mg, 1.56 mmol), potassium carbonate (480.24 mg, 3.47 mmol), 4,5-bis(diphenylphosphino)-9,9-dimethylxanthene (201.05 mg, 347.47 µmol) and tris(dibenzylideneacetone)dipalladium chloroform complex (159.09 mg, 173.73 µmol) in dioxane (10 mL) was purged with nitrogen three times, and the mixture was stirred at 80°C for 2 hours. The reaction solution was concentrated to give a crude product, which was purified by column chromatography (silica gel, petroleum ether/ethyl acetate = 10/1 - 5/1) to give compound **4-2.**

MS *m*/*z*: 253[M+H]⁺.

### Step 3: Synthesis of compound 4

A solution of compound **4-2** (70 mg, 277.01 µmol), **1-12** (80.99 mg, 277.01 µmol), cesium carbonate (180.51 mg, 554.02 µmol), (±)-2,2-bis(diphenylphosphino)-1,1-binaphthyl (34.50 mg, 55.40 µmol) and tris(dibenzylideneacetone)dipalladium chloroform (25.37 mg, 27.70 µmol) in dioxane (5 mL) was purged with nitrogen three times. The mixture was stirred at 110 °C for 3 hours. The reaction solution was concentrated to give a crude product, which was purified by column chromatography (silica gel, petroleum ether/ethyl acetate = 2/1 ~ 1/3), and then separated by preparative high-performance liquid chromatography (column: Waters Xbridge 150*25mm* 5µm; mobile phase: [water (10mM ammonium bicarbonate)-acetonitrile]; acetonitrile%: 34%-64%,9min) to give compound **4**.

MS *m*/*z*: 509[M+H]⁺

¹HNMR (400 MHz, DMSO-d₆) δ 11.02 (s, 1H), 10.91 (s, 1H), 8.88 (s, 1H), 8.59 (d, *J* = 1.5 Hz, 1H), 8.14 (d, *J* = 1.3 Hz, 1H), 8.02 (s, 1H), 7.52 (dd, *J* = 1.5, 7.8 Hz, 1H), 7.47 (dd, *J* = 1.3, 7.9 Hz, 1H), 7.33 - 7.24 (m, 1H), 3.52 (s, 3H), 3.46 (s, 6H), 3.13 (d, *J* = 7.2 Hz, 2H), 2.01 (quin, *J* = 6.1 Hz, 1H), 1.12 (t, *J* = 7.2 Hz, 3H), 0.78 (d, *J* = 6.2 Hz, 4H).

### Example 5

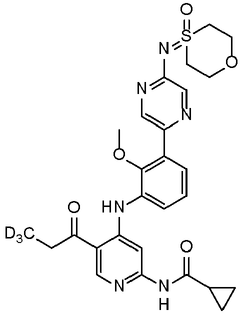

### Synthetic route:

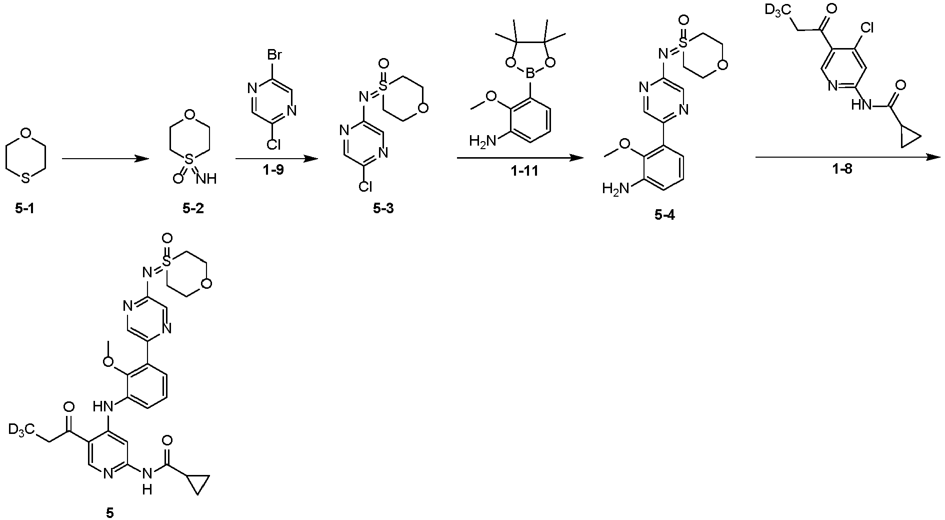

### Step 1: Synthesis of compound 5-2

To a solution of compound **5-1** (400 mg, 3.84 mmol) in methanol (20 mL) were added iodobenzene diacetate (3.09 g, 9.60 mmol) and ammonium carbamate (599.56 mg, 7.68 mmol), and the reaction solution was stirred at 25 °C for 2 hours. The reaction solution was concentrated, and the crude product was purified by column chromatography (silica gel, petroleum ether/ethyl acetate = 4/1 - 1/2) to give compound **5-2.**

MS *m*/*z*: 136[M+H]⁺.

### Step 2: Synthesis of compound 5-3

A solution of compound **5-2** (220.15 mg, 1.63 mmol), **1-9** (300 mg, 1.55 mmol), cesium carbonate (1.01 g, 3.1 mmol), 4,5-bis(diphenylphosphino)-9,9-dimethylxanthene (179.48 mg, 310.19 µmol) and tris(dibenzylideneacetone)dipalladium (142.02 mg, 155.10 µmol) in dioxane (10 mL) was purged with nitrogen three times. The mixture was stirred at 110 °C for 3 hours. The reaction solution was concentrated, and the crude product was purified by column chromatography (silica gel, petroleum ether/ethyl acetate = 10/1 - 4/1) to give compound **5-3**.

MS *m*/*z*: 248[M+H]⁺.

### Step 3: Synthesis of compound 5-4

A solution of compound **5-3** (240 mg, 968.91 µmol), **1-11** (253.44 mg, 1.02 mmol), 1,1-bis(diphenylphosphino)ferrocene palladium chloride (70.90 mg, 96.89 µmol) and potassium phosphate (411.34 mg, 1.94 mmol) in dioxane (8 mL) and water (2 mL) was purged with nitrogen three times. The mixture was stirred at 100 ° C for 2 hours. The reaction solution was concentrated under reduced pressure, and the crude product was purified by column chromatography (silica gel, petroleum ether/ethyl acetate = 2/1 - 1/3) to give compound **5-4**.

MS *m*/*z*: 335[M+H]⁺.

### Step 4: Synthesis of compound 5

A solution of compound **5-4** (104.61 mg, 312.85 µmol), **1-8** (80 mg, 312.85 µmol), cesium carbonate (203.86 mg, 625.70 µmol), 2,2-bis(diphenylphosphino)-1,1-binaphthyl (38.96 mg, 62.57 µmol) and tris(dibenzylideneacetone)dipalladium chloroform complex (28.65 mg, 31.28 µmol) in dioxane (5 mL) was purged with nitrogen three times. The mixture was stirred at 110 ° C for 3 hours. The reaction solution was concentrated to give a crude product, which was purified by column chromatography (silica gel, petroleum ether/ethyl acetate = 1/3 ~ 1/3), and then separated by preparative high-performance liquid chromatography (column: Waters Xbridge 150*25mm* 5µm; mobile phase: [water (10 mM ammonium bicarbonate)-acetonitrile]; acetonitrile%: 37%-67%,9min.) to give compound **5**.

MS *m*/*z*: 554[M+H]⁺;

¹H NMR (400 MHz, DMSO-d₆) δ 11.02 (s, 1H), 10.91 (s, 1H), 8.88 (s, 1H), 8.58 (d, *J* = 1.3 Hz, 1H), 8.24 (d, *J* = 1.3 Hz, 1H), 8.03 (s, 1H), 7.53 (dd, *J* = 1.3, 7.8 Hz, 1H), 7.48 (s, 1H), 7.32 - 7.25 (m, 1H), 4.12 (ddd, *J* = 3.0, 5.7, 12.6 Hz, 2H), 4.05 - 3.93 (m, 2H), 3.84 (td, *J* = 2.7, 14.4 Hz, 2H), 3.71 - 3.58 (m, 2H), 3.53 (s, 3H), 3.11 (s, 2H), 2.08 - 1.96 (m, 1H), 0.78 (d, *J* = 5.9 Hz, 4H).

### Example 6

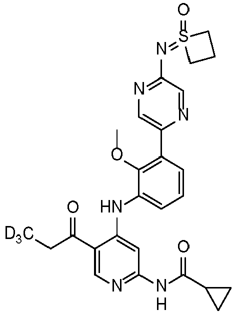

### Synthetic route:

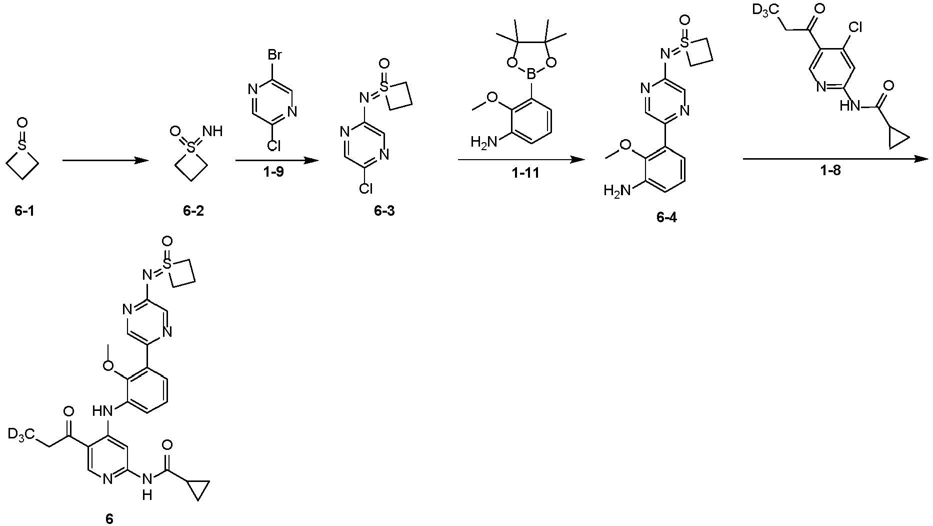

### Step 1: Synthesis of compound 6-2

Compound **6-1** (500 mg, 6.74 mmol) was dissolved in anhydrous methanol (10 mL), and then iodobenzene diacetate (5.43 g, 16.86 mmol) and ammonium carbamate (1.05 g, 13.49 mmol) were added. The mixture was stirred at 25 °C for three hours. The reaction solution was concentrated under reduced pressure, and the crude product was purified by column chromatography (silica gel, petroleum ether/ethyl acetate = 4/1 - 1/2) to give compound 6-2.

MS *m*/*z*: 106[M+H]⁺.

### Step 2: Synthesis of compound 6-3

Compounds **1-9** (600.00 mg, 3.10 mmol) and **6-2** (600 mg, 5.71 mmol) were dissolved in anhydrous dioxane (10 mL), and then cesium carbonate (2.02 g, 6.20 mmol), 4,5-bis(diphenylphosphino)-9,9-dimethylxanthene (358.97 mg, 620.38 µmol) and tris(dibenzylideneacetone)dipalladium (284.05 mg, 310.19 µmol) were added. The mixture was sstirred at 110 °C for four hours. The reaction mixture was concentrated under reduced pressure, and the crude product was purified by column chromatography (silica gel, petroleum ether/ethyl acetate = 10/1 - 4/1) to give compound **6-3.**

MS *m*/*z*: 218[M+H]⁺.

### Step 3: Synthesis of compound 6-4

Compounds **1-11** (50.35 mg, 202.14 µmol) and **6-3** (40 mg, 183.76 µmol) were dissolved in dioxane (2 mL), and a solution of potassium phosphate (78.01 mg, 367.52 µmol) in water (0.5 mL) was added. Then 1,1-bis(diphenylphosphino)ferrocene palladium chloride (13.45 mg, 18.38 µmol) was added. The system was replaced with nitrogen gas and stirred at 100 °C for four hours. The reaction solution was concentrated, and the crude product was purified by column chromatography (silica gel, petroleum ether/ethyl acetate = 1/3 - 1/3) to give compound **6-4.**

MS *m*/*z*: 305[M+H]⁺.

### Step 4: Synthesis of compound 6

Compound **6-4** (80 mg, 262.84 µmol) and compound **1-8** (67.21 mg, 262.84 µmol) were dissolved in anhydrous dioxane (2 mL), and cesium carbonate (171.28 mg, 525.68 µmol) was added. Then tris(dibenzylideneacetone)dipalladium (36.10 mg, 39.43 µmol) and (±)-2,2-bis(diphenylphosphino)-1,1-binaphthyl (32.73 mg, 52.57 µmol) were added, and the mixture was stirred at 90°C for 6 hours. The reaction solution was concentrated to give a crude product, which was purified by preparative thin-layer chromatography (petroleum ether/ethyl acetate = 1/1) to give compound **6.**

MS *m*/*z*: 524[M+H]⁺;

¹H NMR (400 MHz, CDCl₃) δ 11.11 (s, 1 H), 8.57 - 8.70 (m, 2 H), 8.23 (d, *J* = 1.38 Hz, 1H), 7.91 - 8.08 (m, 2 H), 7.49 (dd, *J* = 7.82, 1.56 Hz, 1 H), 7.46 (dd, *J* = 7.88, 1.63 Hz, 1 H), 7.21 (s, 1 H), 4.41 (br d, *J* = 6.63 Hz, 2 H), 4.20 - 4.33 (m, 2 H), 3.42 (s, 3 H), 2.95 (s, 2 H), 2.34 - 2.46 (m, 2 H), 1.01 (br dd, *J* = 4.25, 3.00 Hz, 1 H), 0.78 - 0.84 (m, 4 H).

### Example 7

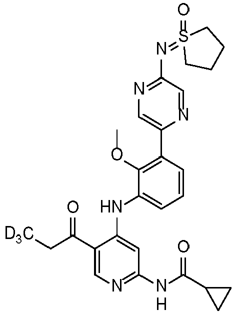

### Synthetic route:

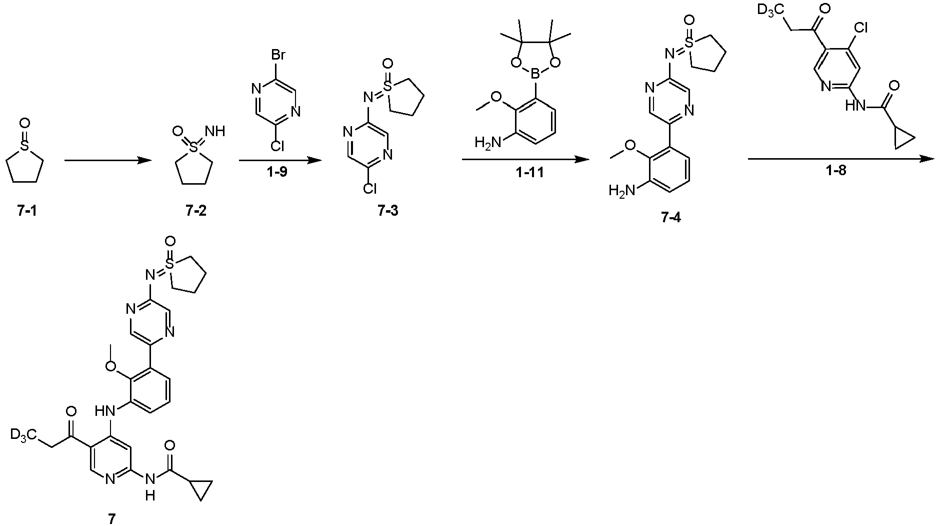

### Step 1: Synthesis of compound 7-2

Compound **7-1** (500 mg, 4.80 mmol) was dissolved in anhydrous methanol (10 mL), and iodobenzene diacetate (3.87 g, 12.00 mmol) and ammonium carbamate (749.45 mg, 9.60 mmol) were added. The mixture was stirred at 25°C for three hours. The reaction solution was concentrated under reduced pressure, and the crude product was purified by column chromatography (silica gel, petroleum ether/ethyl acetate = 4/1 - 1/2) to give compound **7-2.**

MS *m*/*z*: 120[M+H]⁺.

### Step 2: Synthesis of compound 7-3

Compounds **1-9** (973.76 mg, 5.03 mmol) and **7-2** (600 mg, 5.03 mmol) were dissolved in anhydrous dioxane (15 mL), and then cesium carbonate (3.28 g, 10.07 mmol), 4,5-bis(diphenylphosphino)-9,9-dimethylxanthene (582.58 mg, 1.01 mmol) and tris(dibenzylideneacetone)dipalladium (460.99 mg, 503.42 µmol) were added. The mixture was stirred at 110°C for four hours. The reaction mixture was concentrated under reduced pressure, and the crude product was purified by column chromatography (silica gel, petroleum ether/ethyl acetate = 10/1 - 4/1) to give compound **7-3.**

MS *m*/*z*: 232[M+H]⁺.

### Step 3: Synthesis of compound 7-4

Compounds **1-11** (236.53 mg, 949.50 µmol) and **7-3** (200 mg, 863.18 µmol) were dissolved in dioxane (3 mL), and a solution of potassium phosphate (366.45 mg, 1.73 mmol) in water (0.5 mL) was added to the system. Then 1,1-bis(diphenylphosphino)ferrocene palladium chloride (63.16 mg, 86.32 µmol) was added. The atmosphere was replaced with nitrogen, and the system was stirred at 100°C for 4 hours. The reaction system was concentrated, and the crude product was purified by column chromatography (silica gel, petroleum ether/ethyl acetate = 1/3 - 1/3) to give compound **7-4.**

MS *m*/*z*: 319[M+H]⁺.

### Step 4: Synthesis of compound 7

Compound **7-4** (100 mg, 314.08 µmol) and compound **1-8** (80.31 mg, 314.08 µmol) were dissolved in anhydrous dioxane (2 mL), and then cesium carbonate (204.66 mg, 628.15 µmol) was added. The atmosphere was replaced with nitrogen. Then tris(dibenzylideneacetone)dipalladium (43.14 mg, 47.11 µmol) and (±)-2,2-bis(diphenylphosphino)-1,1-binaphthyl (39.11 mg, 62.82 µmol) were added. The atmosphere was replaced with nitrogen again, and the system was stirred at 90°C for 6 hours. The reaction solution was concentrated, and the crude product was purified by preparative chromatography (column: Waters Xbridge 150*25mm* 5µm; mobile phase: [water (10mM ammonium bicarbonate)-acetonitrile]; acetonitrile %: 39%-69%,10min) to give compound 7.

MS *m*/*z*: 538[M+H]⁺;

¹H NMR (400 MHz, CDCl₃) δ 11.11 (s, 1 H), 8.63 - 8.69 (m, 2 H), 8.23 (d, *J* = 1.50 Hz, 2H), 8.01 (s, 1 H), 7.47 (ddd, *J* = 13.60, 7.91, 1.50 Hz, 2 H), 7.20 - 7.23 (m, 1 H), 3.61 (dt, *J* = 13.41, 6.86 Hz, 2 H), 3.52 (s, 3 H), 3.34 (dt, *J* = 13.45, 6.79 Hz, 2 H), 2.95 (s, 2 H), 2.27 - 2.38 (m, 2 H), 2.16 - 2.26 (m, 2 H), 1.42 - 1.51 (m, 1 H), 1.02 (br dd, *J* = 4.38, 3.00 Hz, 2 H), 0.81 (dd, *J* = 7.75, 3.13 Hz, 2 H).

### Example 8

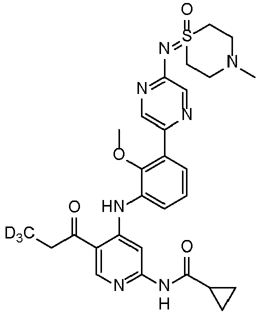

### Synthetic route:

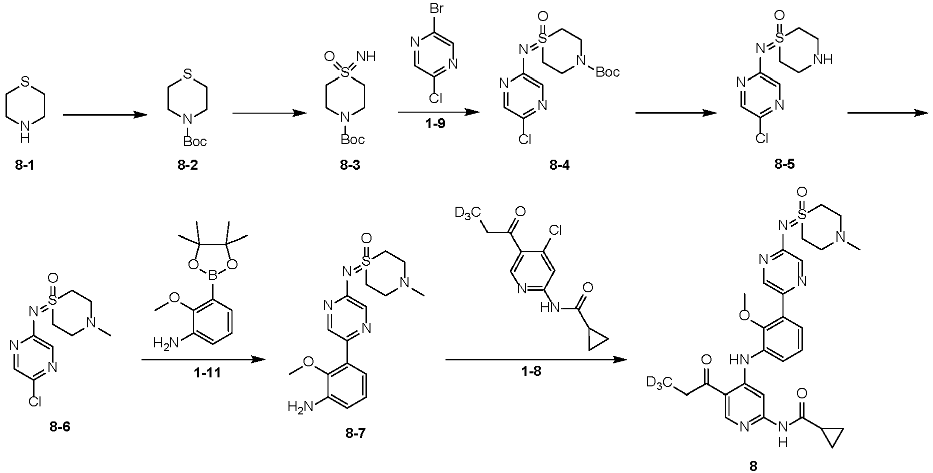

### Step 1: Synthesis of compound 8-2

To a solution of compound **8-1** (5 g, 48.46 mmol) in water (50 mL) and tetrahydrofuran (50 mL) were added sodium carbonate (10.27 g, 96.91 mmol) and di-tert-butyl dicarbonate (11.10 g, 50.88 mmol). The mixture was stirred at 25°C for 15 hours. The mixture was extracted with ethyl acetate (60 mL×3). The combined organic phase was washed with saturated brine (50 mL×1). The organic phase was dried over anhydrous sodium sulfate, and filtered. The filtrate was concentrated under reduced pressure to give compound **8-2.**

¹H NMR (400 MHz, CDCl₃) δ 3.66 - 3.57 (m, 4H), 2.50 (brs, 4H), 1.39 (s, 9H).

### Step 2: Synthesis of compound 8-3

To a solution of compound **8-2** (2 g, 9.84 mmol) in methanol (40 mL) were added iodobenzene diacetate (6.65 g, 20.66 mmol) and ammonium carbamate (1.23 g, 15.74 mmol). The mixture was stirred at 25°C for 2 hours. The reaction solution was concentrated to give a crude product, which was purified by column chromatography (silica gel, petroleum ether/ethyl acetate = 5/1~1/1, then ethyl acetate/methanol = 40/0-40/1) to give compound **8-3.**

MS *m*/*z*: 235[M+H]⁺;

¹H NMR (400 MHz, CDCl₃) δ 4.03 - 3.92 (m, 2H), 3.92 - 3.78 (m, 2H), 3.06 (br s, 4H), 1.49 (s, 9H).

### Step 3: Synthesis of compound 8-4

Compound **8-3** (1.62 g, 6.91 mmol) was dissolved in dioxane (22 mL), and **1-9** (1.41 g, 6.91 mmol), potassium carbonate (1.91 g, 13.83 mmol), tris(dibenzylideneacetone)dipalladium (316.55 mg, 345.69 µmol) and 4,5-bis(diphenylphosphino)-9,9-dimethylxanthene (400.04 mg, 691.38 µmol) were added. The atmosphere was replaced with nitrogen three times. The mixture was heated to 90°C and stirred for 4 hours. The reaction solution was directly filtered. The filtrate was concentratedd under reduced pressure to give a crude product. The crude product was purified by column chromatography (petroleum ether/ethyl acetate = 10/1 - 2/1) to give compound **8-4.**

MS *m*/*z*: 347[M+H]⁺.

### Step 4: Synthesis of compound 8-5

Compound **8-4** (500 mg, 1.44 mmol) was dissolved in methanol (2 mL), and hydrochloric acid in methanol (4 M, 4 mL) was added. The mixture was stirred at 25°C for 4 hours. The reaction solution was concentrated to give compound **8-5.**

MS *m*/*z*: 247[M+H]⁺

### Step 5: Synthesis of compound 8-6

To a solution of compound **8-5** (500 mg, 2.03 mmol) in dichloroethane (5 mL) were added 37% formaldehyde aqueous solution (608.51 mg, 7.50 mmol) and sodium triacetoxyborohydride (859.05m g, 4.05 mmol) under nitrogen at 0 °C. The mixture was stirred at 25°C for 2 hours. Thin layer chromatography showed that the raw material was completely consumed. The mixture was treated with saturated sodium bicarbonate (50 mL), and the mixture was stirred for 10 minutes, and then extracted with dichloromethane (50mL ×3). The combined organic phase was washed with brine (50 mL), dried over anhydrous sodium sulfate, and filtered. The filtrate was concentrated under reduced pressure, and the crude product was purified by column chromatography (silica gel, petroleum ether/ethyl acetate = 3/1 - 0/1) to give compound **8-6.**

MS *m*/*z*: 261[M+H]⁺.

### Step 6: Synthesis of compound 8-7

To a solution of compound **8-6** (290mg, 1.16mmol) in dioxane (8 mL) was added compound **1-11** (318.72mg, 1.22mmol) under nitrogen at 0°C. The mixture was mixed evenly, and then a solution of potassium phosphate (494.21 mg, 2.33 mmol) in water (2 mL) and 1,1'-bis(diphenylphosphino)ferrocene]palladium dichloride (85.18 mg, 116.41 µmol) were added. The mixture was stirred at 100 °C for 2 hours. The reaction solution was concentrated under reduced pressure to give a crude product, which was purified by column chromatography (silica gel, petroleum ether/ethyl acetate = 8/1 - 0/1) to give compound **8-7.**

MS *m*/*z*: 348[M+H]⁺.

### Step 7: Synthesis of compound 8

To a solution of compound **8-7** (100mg, 287.82 µmol) in dioxane (5 mL) were added successively compound **1-8** (82.24mg, 316.60µmol), cesium carbonate (187.55mg, 575.64 µmol), 2,2-bis(diphenylphosphino)-1,1-binaphthyl (35.84mg, 57.56 µmol) and tris(dibenzylideneacetone)dipalladium (26.36mg, 28.78 µmol) under nitrogen at 0 °C. The mixture was stirred at 110°C for 3 hours. The reaction solution was concentrated under reduced pressure to give a crude product, which was purified by column chromatography (silica gel, petroleum ether/ethyl acetate = 4/1 - 0/1), and then separated by preparative high-performance liquid chromatography (column: Waters Xbridge 150*25mm*5µm; mobile phase: [water (ammonia, 0.05% v/v)-acetonitrile]; acetonitrile%:28%-58%) to give compound **8.**

MS *m*/*z:* 567[M+H]⁺;

¹H NMR (400 MHz, CDCl₃) δ 11.27 (br s, 1H), 9.46 - 9.11 (m, 1H), 8.60 (d, *J* = 1.2 Hz, 2H), 8.22 (d, *J* = 1.5 Hz, 1H), 8.03 (s, 1H), 7.54 (br d, *J* = 7.7 Hz, 1H), 7.42 (d, *J* = 7.8 Hz, 1H), 7.26 - 7.21 (m, 1H), 3.73 - 3.67 (m, 2H), 3.49 - 3.42 (m, 2H), 3.10 - 2.71 (m, 8H), 2.24 - 2.01 (m, 3H), 1.58 (br s, 1H), 0.84 (br d, *J* = 4.4 Hz, 4H).

### Example 9

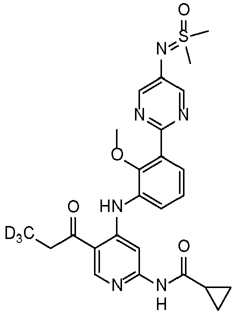

### Synthetic route:

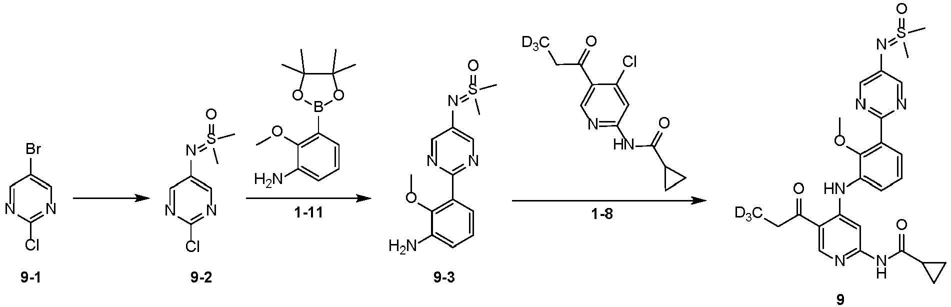

### Step 1: Synthesis of compound 9-2

A solution of compound **9-1** (500 mg, 2.58 mmol), dimethyl sulfoximine (264.86 mg, 14.85 mmol), cesium carbonate (1.68 g, 5.17 mmol), 4,5-bis(diphenylphosphino)-9,9-dimethylxanthene (299.14 mg, 516.99 µmol) and tris(dibenzylideneacetone)dipalladium chloroform complex (236.71 mg, 258.49 µmol) in dioxane (10 mL) was purged with nitrogen three times. The mixture was stirred at 110 °C for 4 hours. The reaction solution was concentrated under reduced pressure, and the crude product was purified by column chromatography (silica gel, petroleum ether/ethyl acetate = 5/1 - 1/2) to give compound **9-2.**

MS *m*/*z*: 206[M+H]⁺.

### Step 2: Synthesis of compound 9-3

A solution of compound **9-2** (100 mg, 496.23 µmol), **1-11** (133.24 mg, 534.85 µmol), 1,1-bis(diphenylphosphino)ferrocene palladium chloride (35.58 mg, 48.62 µmol) and potassium phosphate (206.42 mg, 972.46 µmol) in dioxane (4 mL) and water (1 mL) was purged with nitrogen three times. The mixture was stirred at 100 ° C for 2 hours. The reaction solution was concentrated under reduced pressure, and the crude product was purified by column chromatography (silica gel, petroleum ether/ethyl acetate = 3/1 - 1/4) to give compound **9-3.**

MS *m*/*z*: 293[M+H]⁺.

### Step 3: Synthesis of compound 9

A solution of compound **9-3** (95 mg, 324.95 µmol), **1-8** (91.40 mg, 357.44 µmol), cesium carbonate (211.75 mg, 649.89 µmol), 2,2-bis(diphenylphosphino)-1,1-binaphthyl (40.47 mg, 64.99 µmol) and tris(dibenzylideneacetone)dipalladium chloroform complex (29.76 mg, 32.49 µmol) in dioxane (5 mL) was purged with nitrogen three times. The mixture was stirred at 110 °C for 4 hours. The reaction solution was concentrated to give a crude product, which was purified by column chromatography (silica gel, petroleum ether/ethyl acetate = 1/3 ~ 1/3), and then separated by preparative high-performance liquid chromatography (column: Waters Xbridge 150*25mm* 5µm; mobile phase: [water (10mM ammonium bicarbonate)-acetonitrile]; acetonitrile%: 30%-60%,9min.) to give compound **9.**

MS *m*/*z*: 512[M+H]⁺;

¹H NMR (400 MHz, DMSO-d₆) δ 11.07 - 10.97 (m, 1H), 10.93 - 10.85 (m, 1H), 8.97 - 8.82 (m, 1H), 8.50 (s, 2H), 8.02 (s, 1H), 7.57 - 7.47 (m, 2H), 7.33 - 7.18 (m, 1H), 3.71 - 3.61 (m, 3H), 3.39 (s, 6H), 3.10 (s, 2H), 2.01 (quin, *J* = 6.1 Hz, 1H), 0.78 (d, *J* = 6.1 Hz, 4H).

### Example 10

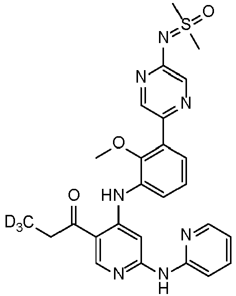

### Synthetic route:

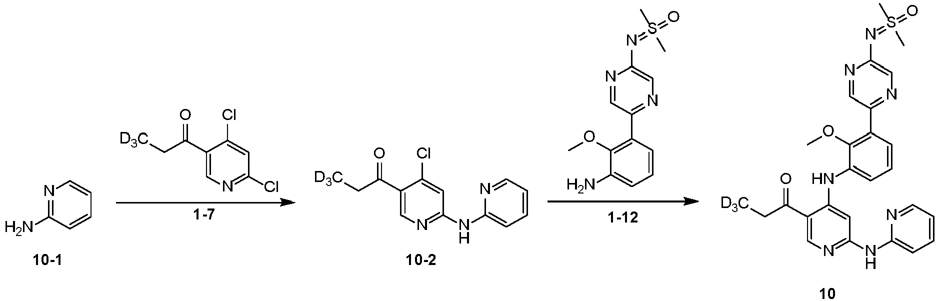

### Step 1: Synthesis of compound 10-2

A solution of compound **10-1** (0.2 g, 965.85 µmol), **1-7** (90.90 mg, 965.85 µmol), potassium carbonate (266.97 mg, 1.93 mmol), 4,5-bis(diphenylphosphino)-9,9-dimethylxanthene (111.77 mg, 193.17 µmol) and tris(dibenzylideneacetone)dipalladium (88.44 mg, 96.58 µmol) in dioxane (5 mL) was purged with nitrogen three times. The mixture was stirred at 110 °C for 16 hours. Water (20 mL) and ethyl acetate (20 mL) were added to the reaction solution. The layers were separated. The aqueous phase was extracted with ethyl acetate (10 mL×3). The organic phases were combined, washed with saturated brine (10 mL×1), dried over anhydrous sodium sulfate, and filtered. The filtrate was concentrated under reduced pressure to give a crude product. The crude product was purified by column chromatography (silica gel, petroleum ether/ethyl acetate = 20/1 - 10/1) to give compound **10-2.**

MS *m*/*z*: 265[M+H]⁺;

¹H NMR (400 MHz, CDCl₃) δ 8.53 (s, 1H), 8.27 (br d, *J* = 4.8 Hz, 1H), 8.00 - 7.92 (m, 1H), 7.90 (s, 1H), 7.67 - 7.52 (m, 1H), 7.27 (d, *J* = 8.3 Hz, 1H), 6.89 (dd, *J* = 5.4, 6.8 Hz, 1H), 2.91 (s, 2H).

### Step 2: Synthesis of compound 10

A solution of compound **10-2** (0.08 g, 302.20 µmol), **1-12** (88.35 mg, 302.20 µmol), cesium carbonate (196.93 mg, 604.40 µmol), (±)-2,2-bis(diphenylphosphino)-1,1-binaphthyl (37.63 mg, 60.44 µmol) and tris(dibenzylideneacetone)dipalladium chloroform complex (27.67 mg, 30.22 µmol) in dioxane (5 mL) was purged with nitrogen three times. The mixture was stirred at 110 °C for 6 hours. 20 mL of water and 20 mL of ethyl acetate were added to the reaction solution. The layers were separated. The aqueous phase was extracted with ethyl acetate (10 mL×3). The organic phases were combined, washed with saturated brine (5 mL×1), dried over anhydrous sodium sulfate, and filtered. The filtrate was concentrated under reduced pressure to give a crude product. The crude product was purified by column chromatography (silica gel, petroleum ether/ethyl acetate = 3/1 ~ 1/3), and then separated by preparative high-performance liquid chromatography (column: Waters Xbridge 150*25mm* 5µm; mobile phase: [water (10mM ammonium bicarbonate)-acetonitrile]; acetonitrile%: 38%-68%,8min.) to give compound **10.**

MS *m*/*z*: 521[M+H]⁺;

¹HNMR (400 MHz, CDCl₃) δ 11.00 (s, 1H), 8.75 - 8.64 (m, 2H), 8.20 (d, *J* = 1.4 Hz, 1H), 8.14 (dd, *J* = 1.2, 4.9 Hz, 1H), 7.59 (s, 1H), 7.55 - 7.48 (m, 3H), 7.45 (br s, 1H), 7.25 - 7.21 (m, 1H), 7.17 (s, 1H), 6.79 (ddd, *J* = 0.8, 5.6, 6.7 Hz, 1H), 3.55 (s, 3H), 3.36 (s, 6H), 2.94 (s, 2H).

### Example 11

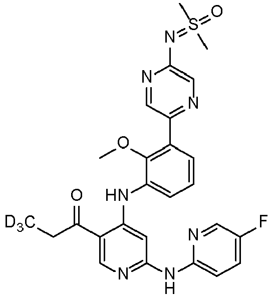

### Synthetic route:

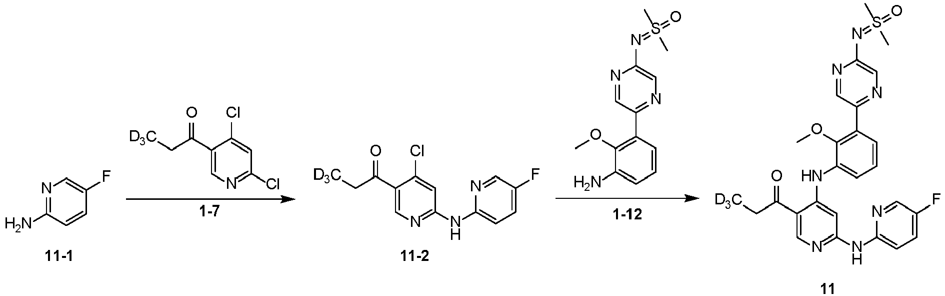

### Step 1: Synthesis of compound 11-2

A solution of compound **11-1** (0.2 g, 965.85 µmol), **1-7** (108.28 mg, 965.85 µmol), potassium carbonate (266.97 mg, 1.93 mmol), 4,5-bis(diphenylphosphino)-9,9-dimethylxanthene (111.77 mg, 193.17 µmol) and tris(dibenzylideneacetone)dipalladium (88.44 mg, 96.58 µmol) in dioxane (40 mL) was purged with nitrogen three times. The mixture was stirred at 110 °C for 16 hours. 20 mL of water and 20 mL of ethyl acetate were added to the reaction solution. The layers were separated. The aqueous phase was extracted with ethyl acetate (10 mL×3). The organic phases were combined, washed with saturated brine (5 mL×1), dried over anhydrous sodium sulfate, and filtered. The filtrate was concentrated under reduced pressure to give a crude product. The crude product was purified by column chromatography (silica gel, petroleum ether/ethyl acetate = 20/1 - 10/1) to give compound **11-2.**

MS *m*/*z*: 283[M+H]⁺

### Step 2: Synthesis of compound 11

A solution of compound **11-2** (0.14 g, 495.20 µmol), **1-12** (144.77 mg, 495.20 µmol), cesium carbonate (322.69 mg, 990.40 µmol), (±)-2,2-bis(diphenylphosphino)-1,1-binaphthyl (61.67 mg, 99.04 µmol) and tris(dibenzylideneacetone)dipalladium chloroform complex (45.35 mg, 49.52 µmol) in dioxane (5 mL) was purged with nitrogen three times. The mixture was stirred at 110 °C for 6 hours. 20 mL of water and 20 mL of ethyl acetate were added to the reaction solution. The layers were separated. The aqueous phase was extracted with ethyl acetate (10 mL×3). The organic phases were combined, washed with saturated brine (5 mL×1), dried over anhydrous sodium sulfate, and filtered. The filtrate was concentrated under reduced pressure to give a crude product. The crude product was purified by column chromatography (silica gel, petroleum ether/ethyl acetate = 1/1 ~ 1/3), and then separated by preparative high-performance liquid chromatography (column: Waters Xbridge 150*25mm* 5µm; mobile phase: [water (10mM ammonium bicarbonate)-acetonitrile]; acetonitrile%: 40%-70%,8min.) to give compound **11.**

MS *m*/*z*: 539[M+H]⁺;

¹H NMR (400 MHz, CDCl₃) δ 11.00 - 10.93 (m, 1H), 8.72 - 8.65 (m, 2H), 8.20 (d, *J* = 1.5 Hz, 1H), 7.99 (d, *J* = 2.9 Hz, 1H), 7.53 (dd, *J* = 1.6, 7.8 Hz, 1H), 7.40 - 7.35 (m, 1H), 7.34 - 7.32 (m, 1H), 7.29 - 7.25 (m, 1H), 7.19 (s, 3H), 3.55 (s, 3H), 3.36 (s, 6H), 2.94 (s, 2H).

### Example 12

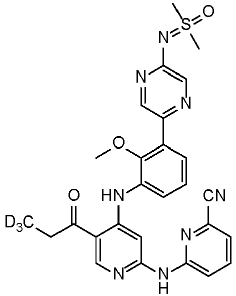

### Synthetic route:

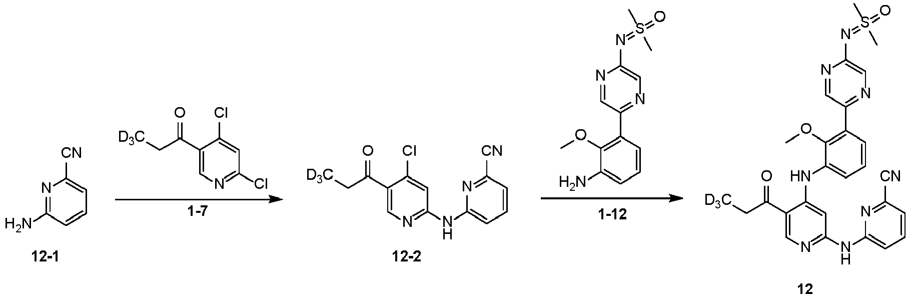

### Step 1: Synthesis of compound 12-2

A solution of compound **12-1** (0.25 g, 1.21 mmol), 1-7 (144.14 mg, 1.21 mmol), potassium carbonate (334.47 mg, 2.42 mmol), 4,5-bis(diphenylphosphino)-9,9-dimethylxanthene (140.03 mg, 242.00 µmol) and tris(dibenzylideneacetone)dipalladium (110.80 mg, 121.00 µmol) in dioxane (5 mL) was purged with nitrogen three times. The mixture was stirred at 110 °C for 16 hours. Water (10 mL) and ethyl acetate (10 mL) were added to the reaction solution. The layers were separated. The aqueous phase was extracted with ethyl acetate (5 mL×2). The organic phases were combined, washed with saturated brine (2 mL×1), dried over anhydrous sodium sulfate, and filtered. The filtrate was concentrated under reduced pressure to give a crude product. The crude product was purified by column chromatography (silica gel, petroleum ether/ethyl acetate = 15/1 - 8/1) to give compound **12-2.**

MS *m*/*z*: 290[M+H]⁺.

### Step 2: Synthesis of compound 12

A solution of compound **12-2** (0.03 g, 103.54 µmol), **1-12** (27.24 mg, 93.19 µmol), cesium carbonate (67.47 mg, 207.09 µmol), 2,2-bis(diphenylphosphino)-1,1-binaphthyl (12.89 mg, 20.71 µmol) and tris(dibenzylideneacetone)dipalladium chloroform complex (9.48 mg, 10.35 µmol) in dioxane (5 mL) was purged with nitrogen three times. The mixture was stirred at 110 °C for 6 hours. 5 mL of water and 5 mL of ethyl acetate were added to the reaction solution. The layers were separated. The aqueous phase was extracted with ethyl acetate (5 mL×3). The organic phases were combined, washed with saturated brine (2 mL×1), dried over anhydrous sodium sulfate, and filtered. The filtrate was concentrated under reduced pressure to give a crude product. The crude product was purified by preparative thin-layer chromatography (silica gel, petroleum ether/ethyl acetate = 2:1), and then separated by preparative high-performance liquid chromatography (column: Waters Xbridge BEH C18 150*25mm* 5µm; mobile phase: [water (10mM ammonium bicarbonate)-acetonitrile]; acetonitrile%: 38%-68%,8min.) to give compound **12.**

MS *m*/*z*: 546[M+H]⁺;

¹H NMR (400 MHz, DMSO-d₆) δ 11.26 (s, 1H), 10.42 (s, 1H), 8.89 (s, 1H), 8.61 (d, *J* = 1.4 Hz, 1H), 8.15 (d, *J* = 1.4 Hz, 1H), 7.95 (s, 1H), 7.92-7.85 (m, 1H), 7.83 - 7.77 (m, 1H), 7.70 (dd, *J* = 1.4, 7.9 Hz, 1H), 7.57 - 7.48 (m, 2H), 7.45 - 7.36 (m, 1H), 3.56 (s, 3H), 3.47 (s, 6H), 2.17 - 2.05 (m, 1H), 2.08 (s, 1H).

### Example 13

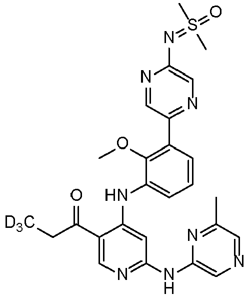

### Synthetic route:

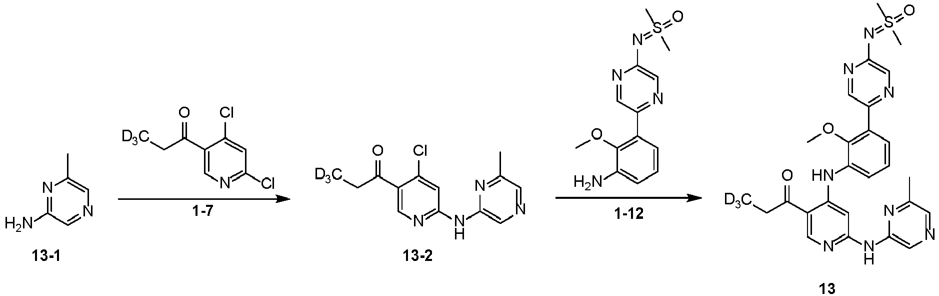

### Step 1: Synthesis of compound 13-2

To a solution of **1-7** (0.1 g, 482.92 µmol) in dioxane (2mL) were added **13-1** (47.43 mg, 434.63 µmol), potassium carbonate (133.49 mg, 965.85 µmol), 4,5-bisdiphenylphosphino-9,9-dimethylxanthene (55.89 mg, 96.59 µmol) and tris(dibenzylideneacetone)dipalladium (44.22 mg, 48.29 µmol). The atmosphere was replaced with nitrogen three times. The mixture was stirred at 110°C for 4 hours. The reaction solution was diluted with water (30 mL), and extracted with ethyl acetate (20 mL×3). The combined organic phase was washed with saturated brine (10 mL×3), dried over anhydrous sodium sulfate, and filtered. The filtrate was concentrated under reduced pressure, and the crude product was purified by column chromatography (silica gel, petroleum ether/ethyl acetate = 50/1 ~ 1/1), and then preparative thin layer chromatography (silica gel, petroleum ether/ethyl acetate = 2/1) to give compound **13-2.**

MS *m*/*z*: 280[M+H]⁺.

### Step 2: Synthesis of compound 13

To a solution of **13-2** (30 mg, 107.24 µmol) in dioxane (2mL) were added **1-12** (31.35 mg, 107.24 µmol), cesium carbonate (69.88 mg, 214.49 µmol), 2,2-bis(diphenylphosphino)-1,1-binaphthyl (13.36 mg, 21.45 µmol) and tris(dibenzylideneacetone)dipalladium (9.82 mg, 10.72 µmol). The atmosphere was replaced with nitrogen three times. The mixture was stirred at 110°C for 6 hours. The reaction solution was directly filtered. The filtrate was concentrated under reduced pressure, and the crude product was purified by preparative thin-layer chromatography (silica gel, petroleum ether/ethyl acetate = 0/1), and then separated by preparative high-performance liquid chromatography (column: Waters Xbridge 150*25mm* 5µm; mobile phase: [water (10mM ammonium bicarbonate)-acetonitrile]; acetonitrile%: 30%-60%,8min) to give compound **13.**

MS *m*/*z*: 536[M+H]⁺;

¹H NMR (400 MHz, DMSO-d₆) δ 11.11 (s, 1H), 10.24 (s, 1H), 8.87 (s, 1H), 8.63 - 8.54 (m, 2H), 8.14 (d, *J* = 1.5 Hz, 1H), 8.03 (s, 1H), 7.98 (s, 1H), 7.63 (dd, *J* = 1.4, 8.0 Hz, 1H), 7.54 (dd, *J* = 1.5, 7.8 Hz, 1H), 7.33 (t, *J* = 7.9 Hz, 1H), 3.55 (s, 3H), 3.46 (s, 6H), 3.08 (s, 3H), 2.30 (s, 2H).

### Example 14

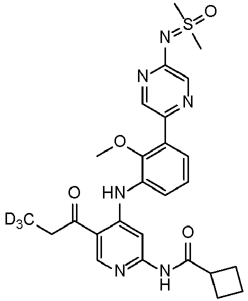

### Synthetic route:

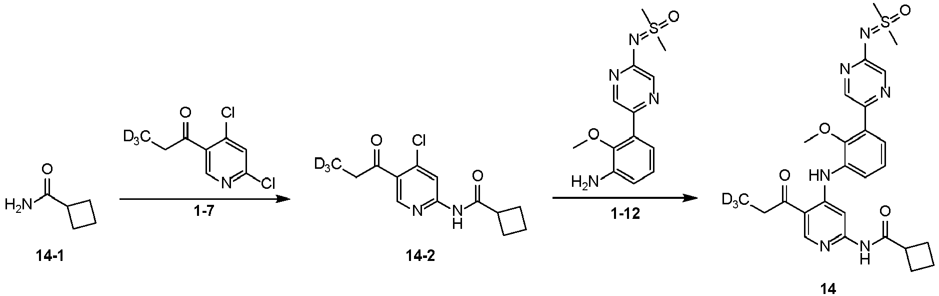

### Step 1: Synthesis of compound 14-2

Compound **14-1** (515.03 mg, 2.49 mmol) was dissolved in dioxane (10 mL), and **1-7** (246.56 mg, 2.49 mmol), potassium carbonate (687.49 mg, 4.97 mmol), tris(dibenzylideneacetone)dipalladium (227.76 mg, 248.72 µmol) and 4,5-bis(diphenylphosphino)-9,9-dimethylxanthene (287.83 mg, 497.44 µmol) were added. The atmosphere was replaced with nitrogen three times. The mixture was heated to 90°C and stirred for 16 hours. The reaction solution was directly filtered. The filtrate was concentratedd under reduced pressure to give a crude product. The crude product was purified by column chromatography (petroleum ether/ethyl acetate = 15/1 ~ 3/1) to give compound **14-2.**

MS *m*/*z*: 270[M+H]⁺.

### Step 2: Synthesis of compound 14

Compound **14-2** (100 mg, 370.73 µmol) was dissolved in dioxane (4 mL), and **11-4** (90.32 mg, 308.94 µmol), cesium carbonate (201.32 mg, 617.88 µmol), tris(dibenzylideneacetone)dipalladium (28.29 mg, 30.89 µmol) and (R)-(+)-2,2-bis(diphenylphosphino)-1,1-binaphthyl (38.47 mg, 61.79 µmol) were added. The atmosphere was replaced with nitrogen three times. The mixture was heated to 110°C and stirred for 16 hours. The crude product was separated by high performance liquid chromatography (column: Waters Xbridge 150*25mm* 5µm; mobile phase: [water (10mM ammonium bicarbonate)-acetonitrile]; acetonitrile%: 40%-70%,9min) to give compound **14.**

MS *m*/*z*: 526[M+H]⁺;

¹H NMR (400 MHz, CDCl₃) δ 11.60 (s, 1H), 8.64 (d, *J* = 1.4 Hz, 1H), 8.49 (s, 1H), 8.22 - 8.18 (m, 2H), 8.14 (s, 1H), 7.68 (dd, *J* = 1.6, 7.8 Hz, 1H), 7.42 - 7.37 (m, 1H), 7.33 - 7.28 (m, 1H), 3.51 (s, 3H), 2.91 (s, 2H), 2.34 - 2.18 (m, 6H), 2.02 - 1.76 (m, 7H).

### Example 15

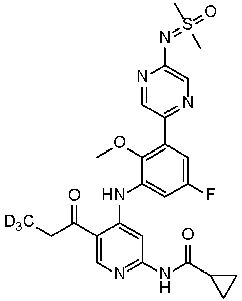

### Synthetic route:

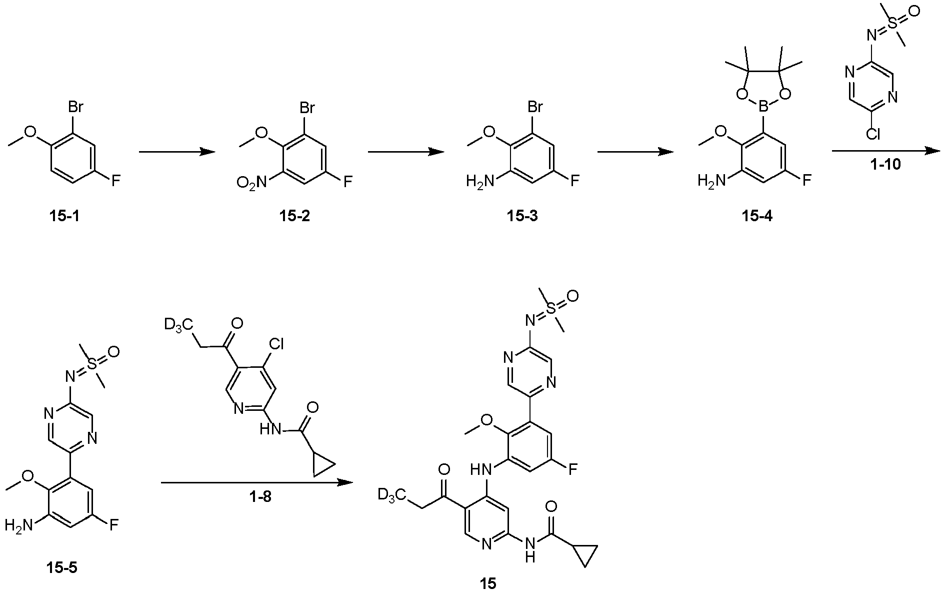

### Step 1: Synthesis of compound 15-2

Compound **15-1** (5 g, 24.39 mmol) was dissolved in concentrated sulfuric acid (20 mL). At -10 °C, concentrated nitric acid (2.21 g, 34.99 mmol, 1.58 mL) was added dropwise, and the mixture was stirred at -10 °C for 0.5 hours. Thin layer chromatography showed that the starting materials were consumed completely. The reaction solution was quenched by pouring into crushed ice, and extracted with ethyl acetate (50 mL×3). The organic phases were combined, washed with saturated brine (15 mL×2), dried over anhydrous sodium sulfate, and filtered. The filtrate was concentrated under reduced pressure to give a crude product. The crude product was purified by column chromatography (silica gel, petroleum ether/ethyl acetate = 1/0 ∼ 0/1) to give compound **15-2.**

¹H NMR (400 MHz, DMSO-d₆) δ 7.58 (dd, *J* = 3.1, 7.1 Hz, 1H), 7.54 (dd, *J* = 3.1, 7.3 Hz, 1H), 4.01 (s, 3H).

### Step 2: Synthesis of compound 15-3

Compound **15-2** (3 g, 12.00 mmol) was dissolved in water (30 mL) and ethanol (10 mL), and iron powder (3.35 g, 59.99 mmol) and ammonium chloride (6.42 g, 119.99 mmol) were added. The mixture was stirred at 100°C for 2 hours and TLC showed the starting materials were consumed completely. The reaction solution was filtered through diatomaceous earth to remove iron powder. The filtrate was diluted with ethyl acetate (150 mL), and washed with water (10 mL×3). The organic phases were combined, washed with saturated brine (5 mL×2), dried over anhydrous sodium sulfate, and filtered. The filtrate was concentrated under reduced pressure to give a crude product. The crude product was purified by column chromatography (silica gel, petroleum ether/ethyl acetate = 100/1 ~ 40/1) to give compound **15-3.**

¹H NMR (400 MHz, DMSO-d₆) δ 6.63 (dd, *J* = 2.9, 8.1 Hz, 1H), 6.41 (dd, *J* = 2.9, 9.8 Hz, 1H), 3.80 (s, 3H).

### Step 3: Synthesis of compound 15-4

Compound **15-3** (1 g, 4.54 mmol) was dissolved in dioxane (30 mL). Bis(pinacolato)diboron (1.73 g, 6.82 mmol), potassium acetate (1.34 g, 13.63 mmol) and [1,1-bis(diphenylphosphino)ferrocene]palladium dichloride dichloromethane (185.57 mg, 227.23 µmol) were added. The atmosphere was replaced with nitrogen three times, and the mixture was stirred at 100 °C for 3 hours. The reaction solution was diluted with water (10 mL), and extracted with ethyl acetate (20 mL×3). The organic phases were combined, washed with brine (5 mL×3), dried over anhydrous sodium sulfate, and filtered. The filtrate was concentrated under reduced pressure to give a crude product. The crude product was purified by column chromatography (silica gel, petroleum ether/ethyl acetate = 40/1 ~ 8/1) to give compound **15-4.**

MS *m*/*z:* 268[M+H]⁺.

### Step 4: Synthesis of compound 15-5

A solution of compound **15-4** (100.00 mg, 374.40 µmol), **1-10** (70 mg, 340.36 µmol), potassium phosphate (144.49 mg, 680.72 µmol) and 1,1-bis(diphenylphosphino)ferrocene palladium chloride (24.90 mg, 34.04 µmol) in dioxane (2 mL) and water (0.5 mL) was purged with nitrogen three times. The mixture was stirred at 100 °C for 2 hours. The reaction solution was diluted with ethyl acetate (60 mL), and extracted with water (5 mL×3). The organic phases were combined, washed with brine (5 mL×2), dried over anhydrous sodium sulfate, and filtered. The filtrate was concentrated under reduced pressure to give a crude product. The crude product was purified by column chromatography (silica gel, petroleum ether/ethyl acetate = 2/1 ~ 1/2,) to give compound **15-5.**

MS *m*/*z:* 311[M+H]⁺.

### Step 5: Synthesis of compound 15

Compound **15-5** (90 mg, 290.00 µmol) was dissolved in dioxane (4 mL), and compound **1-8** (83 mg, 324.58 µmol, cesium carbonate (211.51 mg, 649.16 µmol, tris(dibenzylideneacetone)dipalladium (29.72 mg, 32.46 µmol) and (R)-(+)-2,2-bis(diphenylphosphino)-1,1-binaphthyl (40.42 mg, 64.92 µmol) were added. The atmosphere was replaced with nitrogen three times. The mixture was stirred at 110 °C for 3 hours. The reaction solution was filtered. The filtrate was concentratedd under reduced pressure to give a crude product. The crude product was separated by high performance liquid chromatography (column: Waters Xbridge 150*25mm*5µm; mobile phase: [water (10mM ammonium bicarbonate)-acetonitrile]; acetonitrile%: 37%-67%,9min) to give compound 15.

MS *m*/*z:* 530[M+H]⁺;

¹H NMR (400 MHz, DMSO-d6) δ 11.15 (s, 1H), 10.98 (s, 1H), 8.91 (s, 1H), 8.67 (d, *J* = 1.0 Hz, 1H), 8.15 (d, *J* = 1.1 Hz, 1H), 8.11 (s, 1H), 7.34 (ddd, *J* = 3.0, 9.5, 18.1 Hz, 2H), 3.53 (s, 3H), 3.47 (s, 6H), 3.11 (s, 2H), 2.07 - 1.98 (m, 1H), 0.87 - 0.75 (m, 4H).

### Example 16

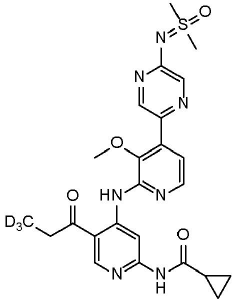

### Synthetic route:

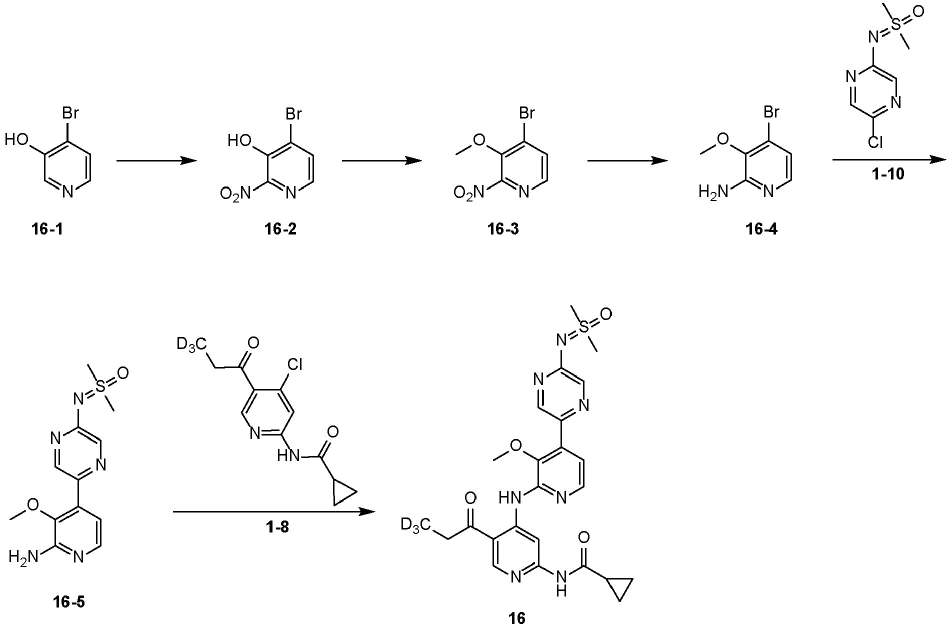

### Step 1: Synthesis of compound 16-2

Compound **16-1** (5g, 28.74 mmol) was dissolved in concentrated sulfuric acid (15 mL), and the solution was cooled to 0 °C and stirred for 10 minutes. Then nitric acid (4.2 g, 66.65 mmol) was added dropwise to the reaction solution. The atmosphere was replaced with nitrogen three times. The mixture was stirred at 25 °C for 16 hours. The reaction solution was poured into ice water (200 mL), and extracted with ethyl acetate (100 mL×3). The organic phases were combined, washed with saturated brine (100 mL×1), dried over anhydrous sodium sulfate, and filtered. The filtrate was concentrated under reduced pressure to give crude compound **16-2,** which was directly used in the next reaction.

MS *m*/*z:* 219[M+H]⁺, 221[M+2+H]⁺.

### Step 2: Synthesis of compound 16-3

Compound **16-2** (4.6 g, 16.80 mmol, 80% purity) was dissolved in N,N-dimethylformamide (50 mL). Potassium carbonate (4.64 g, 33.61 mmol) was added. The mixture was stirred at 25 °C for 10 minutes. Methyl iodide (4.77 g, 33.61 mmol) was added dropwise into the above solution. The atmosphere was replaced with nitrogen three times. The mixture was stirred at 25 °C for 16 hours. The reaction solution was diluted with water (100 mL), and extracted with ethyl acetate (100 mL×2). The organic phases were combined, washed with saturated brine (50 mL×1), dried over anhydrous sodium sulfate, and filtered. The filtrate was concentrated under reduced pressure to a crude product. The crude product was purified by column chromatography (silica gel, petroleum ether/ethyl acetate = 1/0 ~ 20/1) to give compound **16-3.**

MS *m*/*z:* 233[M+H]⁺, 235[M+2+H]⁺;

¹H NMR (400 MHz, DMSO-d₆) δ 8.25 - 8.23 (m, 1H), 8.21 - 8.18 (m, 1H), 3.97 (s, 3H).

### Step 3: Synthesis of compound 16-4

Compound **16-3** (1.48 g, 6.35 mmol) was dissolved in ethanol (4 mL) and water (2 mL). Acetic acid (4 mL) and iron powder (1.77 g, 31.76 mmol) were added. The mixture was stirred at 25 °C for 1.5 hours. The reaction solution was filtered. The filtrate was washed successively with saturated sodium bicarbonate solution (20 mL) and saturated brine (20 mL), dried over anhydrous sodium sulfate, and filtered. The filtrate was concentrated under reduced pressure to give a crude product. The crude product was stirred with petroleum ether and ethyl acetate (petroleum ether/ethyl acetate = 5/1), and filtered. The filter cake was collected to give compound **16-4.**

MS *m*/*z:* 203[M+H]⁺, 205[M+2+H]⁺;

¹H NMR (400 MHz, DMSO-d₆) δ 7.54 (d, *J* = 5.4 Hz, 1H), 6.72 (d, *J* = 5.4 Hz, 1H), 6.22 (s, 2H), 3.69 (s, 3H).

### Step 4: Synthesis of compound 16-5

A solution of compound **16-4** (0.5 g, 2.46 mmol), bis(pinacolato)diboron (1.25 g, 4.93 mmol), 1,1-bis(diphenylphosphino)ferrocene palladium chloride and tris(dibenzylideneacetone)dipalladium (180.19 mg, 246.26 µmol) and potassium acetate in dioxane (25 mL) was purged with nitrogen three times. The mixture was stirred at 80°C for 16 hours. To the reaction solution were added compound **1-10** (1.52 g, 7.39 mmol), potassium phosphate (1.05 g, 4.93 mmol) and water (6 mL). The atmosphere was replaced with nitrogen three times, and the mixture was stirred at 100 °C for 16 hours. The reaction solution was concentrated to give a crude product, which was purified by column chromatography (silica gel, petroleum ether/ethyl acetate = 2/1 ~ 0/1, then ethyl acetate/ethanol = 10/1), and then purified by preparative thin layer chromatography (silica gel, ethyl acetate/ethanol = 10/1) to give compound **16-5.**

MS *m*/*z:* 294[M+H]⁺;

¹H NMR (400 MHz, DMSO-d₆) δ 8.69 (d, *J* = 1.4 Hz, 1H), 8.14 (d, *J* = 1.5 Hz, 1H), 7.75 (d, *J* = 5.3 Hz, 1H), 6.90 (d, *J* = 5.3 Hz, 1H), 5.97 (s, 2H), 3.55 (s, 3H), 3.47 (s, 6H).

### Step 5: Synthesis of compound 16

A solution of compound **16-5** (25.70 mg, 87.60 µmol, compound **1-8** (0.028 g, 109.50 µmol, cesium carbonate (71.35 mg, 218.99 µmol, 2,2-bis(diphenylphosphino)-1,1-binaphthyl (12.89 mg, 20.71 µmol) and tris(dibenzylideneacetone)dipalladium chloroform complex (10.03 mg, 10.95 µmol) in dioxane (2 mL) was purged with nitrogen three times. The mixture was stirred at 110°C for 6 hours. The reaction solution was concentrated to give a crude product, which was purified by column chromatography (silica gel, petroleum ether/ethyl acetate = 2/1 ~0/1), and then separated by high-performance liquid chromatography (column: Waters Xbridge 150*25mm*5µm; mobile phase: [water (10mM ammonium bicarbonate)-acetonitrile]; acetonitrile%: 31%-61%,8min) to give compound **16.**

MS *m*/*z:* 513[M+H]⁺;

¹H NMR (400 MHz, DMSO-d₆) δ 12.33 (s, 1H), 10.93 (s, 1H), 9.69 (s, 1H), 8.97 (s, 1H), 8.78 (d, *J* = 1.5 Hz, 1H), 8.28 - 8.11 (m, 2H), 7.41 (d, *J* = 5.3 Hz, 1H), 3.71 (s, 3H), 3.50 (s, 6H), 3.15 (s, 2H), 2.08 (s, 1H), 0.92 - 0.80 (m, 4H).

### Example 17

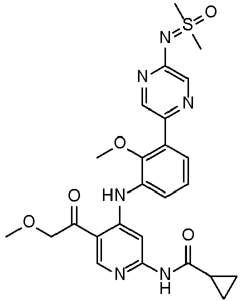

### Synthetic route:

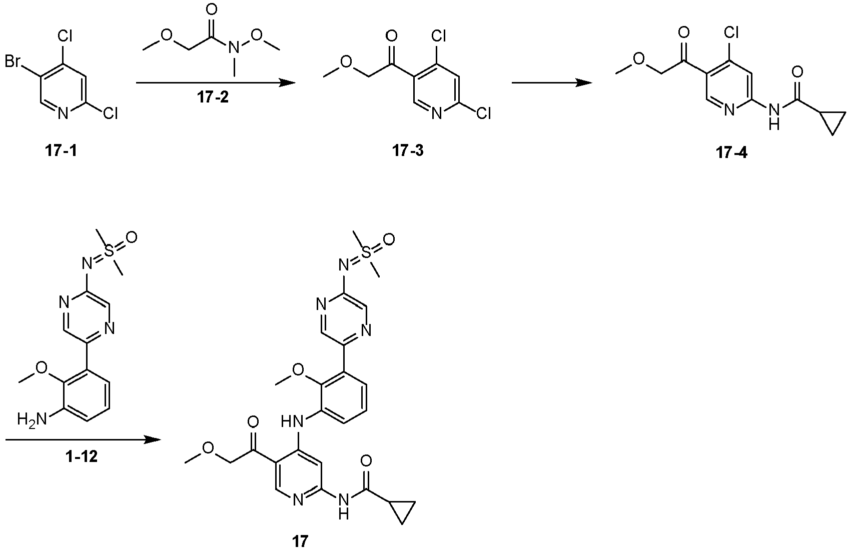

### Step 1: Synthesis of compound 17-3

At -65°C, isopropylmagnesium chloride (2 M, 8.81 mL) was added dropwise to a solution of compound **17-1** (2 g, 8.81 mmol) in tetrahydrofuran (10 mL), and the mixture was stirred at -65°C for 1 hour. A solution of compound **17-2** (1.6 g, 12.02 mmol) in tetrahydrofuran (5 mL) was added dropwise, and the mixture was stirred at 20 °C for 2 hours. Thin layer chromatography showed that the starting materials were consumed completely. The reaction solution was quenched with saturated ammonium chloride solution (10 mL), diluted with water (10 mL), and extracted with ethyl acetate (20 mL×3). The organic phases were combined, washed with saturated brine (5 mL×3), dried over anhydrous sodium sulfate, and filtered. The filtrate was concentrated under reduced pressure to give a crude product. The crude product was purified by column chromatography (silica gel, petroleum ether/ethyl acetate = 50/1 ~ 30/1) to give compound **17-3.**

¹H NMR (400 MHz, CDCl₃) δ 8.57 (s, 1H), 7.46 (s, 1H), 4.53 (s, 2H), 3.46 (s, 3H).

### Step 2: Synthesis of compound 17-4

Compound **17-3** (200 mg, 908.87 µmol) was dissolved in dioxane (5 mL), and compound cyclopropylcarboxamide (81.22 mg, 954.32 µmol, potassium carbonate (376.85 mg, 2.73 mmol), tris(dibenzylideneacetone)dipalladium (83.23 mg, 90.89 µmol) and 4,5-bis(diphenylphosphino)-9,9-dimethylxanthene (105.18 mg, 181.77 µmol) were added. The atmosphere was replaced with nitrogen three times, and the mixture was stirred at 80°C for 6 hours. The reaction solution was diluted with water (10 mL), and extracted with ethyl acetate (20 mL×3). The organic phases were combined, washed with saturated brine (5 mL×3), dried over anhydrous sodium sulfate, and filtered. The filtrate was concentrated under reduced pressure to give a crude product. The crude product was purified by column chromatography (silica gel, petroleum ether/ethyl acetate = 5/1 ~ 3/1) to give compound **17-4.**

MS *m*/*z:* 269[M+H]⁺;

¹H NMR (400 MHz, CDCl₃) δ 8.57 (s, 1H), 8.44 (br s, 1H), 8.37 (s, 1H), 4.59 (s, 2H), 3.48 (s, 3H), 1.59 (dt, *J* = 3.9, 8.1 Hz, 1H), 1.18 - 1.13 (m, 2H), 1.00 - 0.95 (m, 2H).

### Step 3: Synthesis of compound 17

Compound **17-4** (90 mg, 334.95 µmol) was dissolved in dioxane (4 mL), and compound **1-12** (88.13 mg, 301.46 µmol,), potassium carbonate (92.58 mg, 669.90 µmol, palladium acetate (7.52 mg, 33.50 µmol) and 4,5-bis(diphenylphosphino)-9,9-dimethylxanthene (38.76 mg, 66.99 µmol) were added. The atmosphere was replaced with nitrogen three times, and the mixture was stirred at 80°C for 4 hours. The reaction solution was diluted with water (10 mL), and extracted with ethyl acetate (20 mL×3). The organic phases were combined, washed with saturated brine (5 mL×3), dried over anhydrous sodium sulfate, and filtered. The filtrate was concentrated under reduced pressure to give a crude product. The crude product was purified by high performance liquid chromatography (column: Waters Xbridge 150*25mm* 5µm; mobile phase: [water (10mM ammonium bicarbonate)-acetonitrile]; acetonitrile%: 29%-59%, 10min) to give compound **17.**

MS *m*/*z:* 525[M+H]⁺;

¹H NMR (400 MHz, DMSO-d₆) δ 10.94 (s, 1H), 10.84 (s, 1H), 8.76 (s, 1H), 8.59 (d, *J* = 1.3 Hz, 1H), 8.14 (d, *J* = 1.3 Hz, 1H), 8.04 (s, 1H), 7.54 (dd, *J* = 1.4, 7.8 Hz, 1H), 7.49 (dd, *J* = 1.3, 7.9 Hz, 1H), 7.33 - 7.26 (m, 1H), 4.80 (s, 2H), 3.53 (s, 3H), 3.47 (s, 6H), 3.39 (s, 3H), 2.02 (quin, *J* = 6.1 Hz, 1H), 0.79 (d, *J* = 6.1 Hz, 4H).

### Example 18

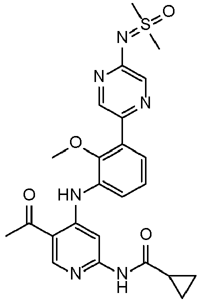

### Synthetic route:

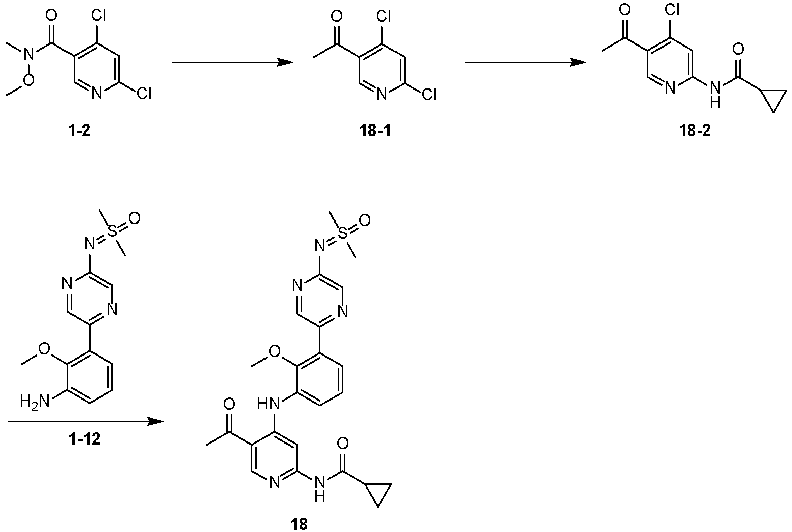

### Step 1: Synthesis of compound 18-1

To a solution of compound **1-2** (1 g, 4.25 mmol) in THF (10 mL) was added methyl magnesium bromide (3M in diethyl ether, 2.13 mL), and the mixture was stirred at 0 °C for 1 hour. The reaction solution was concentrated, and the crude product was purified by column chromatography (silica gel, petroleum ether/ethyl acetate = 50/1 ~ 3/1) to give compound **18-1.**

MS *m*/*z:* 190[M+H]⁺.

### Step 2: Synthesis of compound 18-2

To a solution of compound **18-1** (200 mg, 1.05 mmol) in dioxane (3 mL) were added cyclopropylcarboxamide (98.53 mg, 1.16 mmol), potassium carbonate (290.92 mg, 2.10 mmol), tris(dibenzylideneacetone)dipalladium (48.19 mg, 52.62 µmol) and 4,5-bisdiphenylphosphino-9,9-dimethylxanthene (60.90 mg, 105.25 µmol) under nitrogen. The mixture was stirred at 90 °C for 3 hours. The reaction solution was concentrated under reduced pressure to give a crude product, which was purified by column chromatography (silica gel, petroleum ether/ethyl acetate = 20/1 ~ 3/1) to give compound **18-2.**

¹H NMR (400 MHz, DMSO-d₆) δ 11.36 (br s, 1H), 8.79 (s, 1H), 8.24 (s, 1H), 2.61 (s, 3H), 2.09 - 1.99 (m, 1H), 0.89 - 0.84 (m, 4H).

### Step 3: Synthesis of compound 18

To a solution of compound **18-2** (117.56 mg, 492.55 µmol) in dioxane (2 mL) were slowly added in batches compound **1-12** (120 mg, 410.46 µmol, cesium carbonate (267.47 mg, 820.922 µmmol), tris(dibenzylideneacetone)dipalladium (48.19 mg, 52.62 µmol) and (R)-(+)-2,2-bis(diphenylphosphino)-1,1-binaphthyl (25.56 mg, 41.05 µmol) under nitrogen. The mixture was stirred at 110 °C for 4 hours. The reaction solution was concentrated under reduced pressure to give a crude product, which was purified by column chromatography (silica gel, petroleum ether/ethyl acetate = 20/1 ~ 3/1), and then separated by preparative high-performance liquid chromatography (column: Waters Xbridge 150*25mm*5µm; mobile phase: [water (ammonia, 0.05%v/v)-acetonitrile]; acetonitrile%: 22%-52%,9min) to give compound **18.**

MS *m*/*z:* 495[M+H]⁺;

¹H NMR (400 MHz, DMSO-d6) δ 11.52 (s, 1H), 11.44 (s, 1H), 9.34 (s, 1H), 9.09 (s, 1H), 8.64 (s, 1H), 8.53 (s, 1H), 8.01 (br dd, *J* = 7.6, 19.6 Hz, 2H), 7.85 - 7.72 (m, 1H), 4.01 (s, 3H), 3.97 (s, 6H), 3.15 (s, 3H), 2.57 - 2.48 (m, 1H), 1.29 (br d, *J* = 5.9 Hz, 4H).

### Example 19

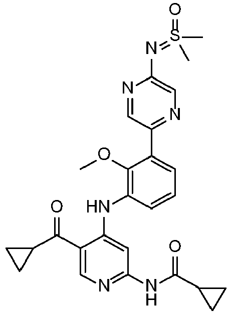

### Synthetic route:

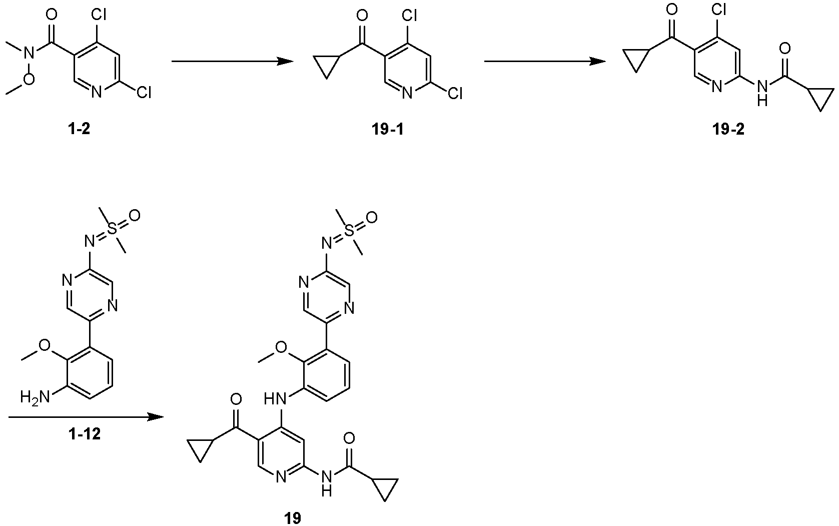

### Step 1: Synthesis of compound 19-1

To a solution of compound **1-2** (1 g, 4.25 mmol) in THF (10 mL) was added cyclopropyl magnesium bromide (0.5 M in tetrahydrofuran, 25.52 mL), and the mixture was stirred at 0 °C for 1 hour. The reaction solution was concentrated, and the crude product was purified by column chromatography (silica gel, petroleum ether/ethyl acetate = 50/1 ~ 1/1) to give compound **19-1.**

MS *m*/*z:* 216[M+H]⁺.

### Step 2: Synthesis of compound 19-2

To a solution of compound **19-1** (200 mg, 925.65 µmol) in dioxane (3 mL) were added cyclopropylcarboxamide (86.65 mg, 1.02 mmol), potassium carbonate (255.86 mg, 1.85 mmol), tris(dibenzylideneacetone)dipalladium (42.38 mg, 46.28 µmol) and 4,5-bisdiphenylphosphino-9,9-dimethylxanthene (53.56 mg, 92.57 µmol) under nitrogen. The mixture was stirred at 90 °C for 3 hours. The reaction solution was concentrated under reduced pressure to give a crude product, which was purified by column chromatography (silica gel, petroleum ether/ethyl acetate = 10/1 ~ 1/1) to give compound **19-2.**

¹H NMR (400 MHz, DMSO-d₆) δ 11.36 (s, 1H), 8.69 (s, 1H), 8.26 (s, 1H), 2.66 - 2.61 (m, 1H), 2.09 - 1.99 (m, 1H), 1.16 - 1.11 (m, 2H), 1.11 - 1.07 (m, 2H), 0.88 (d, *J* = 2.3 Hz, 2H), 0.86 (s, 2H).

### Step 3: Synthesis of compound 19

To a solution of compound **19-2** (95.07 mg, 359.15 µmol) in dioxane (2 mL) were slowly added in batches compound **1-12** (100 mg, 342.05 µmol, cesium carbonate (222.89 mg, 684.10 µmmol), tris(dibenzylideneacetone)dipalladium (15.66 mg, 17.10 µmol) and (R)-(+)-2,2-bis(diphenylphosphino)-1,1-binaphthyl (21.30 mg, 34.20 µmol) under nitrogen. The mixture was stirred at 110 °C for 4 hours. The reaction solution was concentrated under reduced pressure to give a crude product, which was purified by column chromatography (silica gel, petroleum ether/ethyl acetate = 20/1 ~ 3/1), and then separated by preparative high-performance liquid chromatography (column: Waters Xbridge 150*25mm*5µm; mobile phase: [water (ammonia, 0.05% v/v)-acetonitrile]; acetonitrile%: 27%-57%,9min) to give compound **19.**

¹H NMR (400 MHz, DMSO-d6) δ 11.00 (s, 1H), 10.93 (s, 1H), 9.13 (s, 1H), 8.58 (d, *J* = 1.3 Hz, 1H), 8.14 (d, *J* = 1.3 Hz, 1H), 8.03 (s, 1H), 7.50 (ddd, *J* = 1.3, 7.8, 18.3 Hz, 2H), 7.31 - 7.25 (m, 1H), 3.50 (s, 3H), 3.46 (s, 6H), 3.07 - 2.94 (m, 1H), 2.09 - 1.96 (m, 1H), 1.13 - 1.01 (m, 4H), 0.79 (d, *J* = 6.1 Hz, 4H).

### Example 20

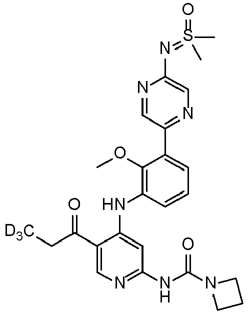

### Synthetic route:

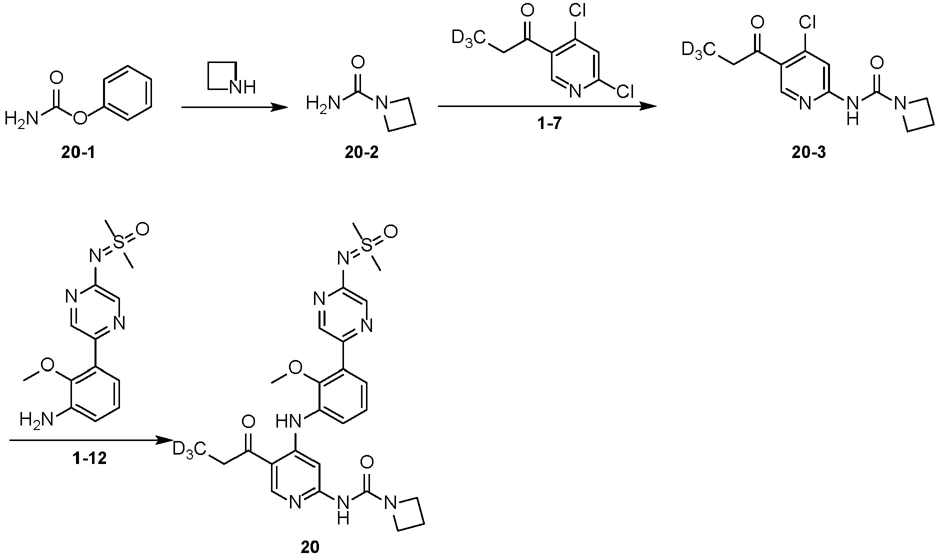

### Step 1: Synthesis of compound 20-2

To a solution of compound **20-1** (0.8 g, 5.83 mmol) in dioxane (12mL) was added cyclobutylamine (999.20 mg, 17.50 mmol), and the mixture was stirred at 25 °C for 3 hours. The reaction solution was concentrated under reduced pressure, and the crude product was purified by slurrying with dichloromethane to give compound **20-2.**

¹H NMR (400 MHz, DMSO-d₆) δ 5.73 (br s, 2H), 3.75 (t, *J* = 7.5 Hz, 4H), 2.08 (t, *J* = 7.5 Hz, 2H).

### Step 2: Synthesis of compound 20-3

A solution of compound **20-2** (100 mg, 482.92 µmol, compound **1-7** (53.18 mg, 531.22 µmol, potassium carbonate (200.24 mg, 1.45 mmol), tris(dibenzylideneacetone)dipalladium (22.11 mg, 24.15 µmol) and 4,5-bisdiphenylphosphino-9,9-dimethylxanthene (27.94 mg, 48.29 µmol) in dioxane (2 mL) was purged with nitrogen three times, and the mixture was stirred at 90 °C for 3 hours. The reaction solution was concentrated under reduced pressure, and the crude product was purified by column chromatography (silica gel, petroleum ether/ethyl acetate = 30/1 ~ 1/1) to give compound **20-3.**

MS m/z: 271[M+H]⁺.

### Step 3: Synthesis of compound 20

A solution of compound **1-12** (100 mg, 342.05 µmol, **20-3** (111.12 mg, 410.46 µmol, cesium carbonate (222.89 mg, 684.10 µmol, 2,2-bis(diphenylphosphino)-1,1-binaphthyl (21.30 mg, 34.20 µmol) and tris(dibenzylideneacetone)dipalladium chloroform complex (15.66 mg, 17.10 µmol) in dioxane (2 mL) was purged with nitrogen three times. The mixture was stirred at 110 °C for 3 hours. The reaction solution was concentrated to give a crude product, which was purified by column chromatography (silica gel, petroleum ether/ethyl acetate = 10/1 ~ 0/1 and dichloromethane/methanol =5/1), and then purified by preparative high-performance liquid chromatography (column: Waters Xbridge 150*25mm*5µm; mobile phase: [water (ammonia, 0.05% v/v)-acetonitrile]; acetonitrile%: 23%-53%,9min) to give compound 20.

MS m/z: 527[M+H]⁺;

¹H NMR (400 MHz, DMSO-d₆) δ 11.01 (br s, 1H), 9.17 (br s, 1H), 8.80 (br s, 1H), 8.60 (br s, 1H), 8.14 (br s, 1H), 7.89 (br s, 1H), 7.49 (br t, *J* = 8.0 Hz, 2H), 7.28 (br t, *J* = 7.4 Hz, 1H), 3.96 (br s, 4H), 3.53 (br s, 3H), 3.46 (br s, 6H), 3.07 (br s, 2H), 2.18 - 2.08 (m, 2H).

### Example 21

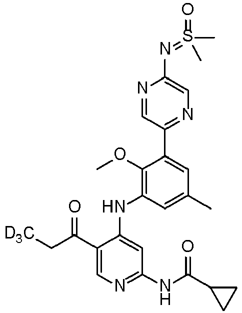

### Synthetic route:

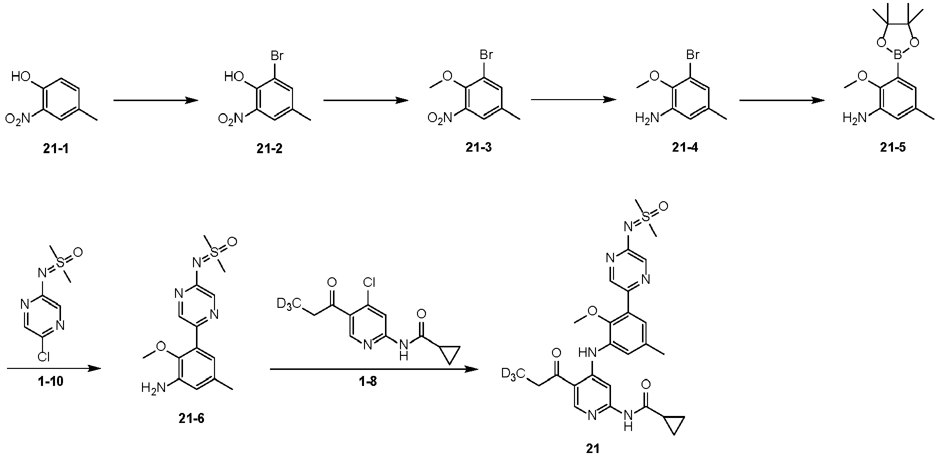

### Step 1: Synthesis of compound 21-2

Compound **21-1** (10 g, 65.30 mmol) was dissolved in N,N-dimethylformamide (100 mL). N-bromosuccinimide (13.95 g, 78.36 mmol) was added. The atmosphere was replaced with nitrogen three times. The mixture was stirred at 25 °C for 20 hours. The reaction solution was diluted with water (300 mL), and extracted with ethyl acetate (300 mL×3). The combined organic phase was washed with saturated brine (100 mL×3), dried over anhydrous sodium sulfate, and filtered. The filtrate was concentrated under reduced pressure, and purified by column chromatography (silica gel, petroleum ether/ethyl acetate = 1/0) to give compound **21-2.**

¹H NMR (400 MHz,DMSO-d₆) δ 10.77 (br s, 1H), 7.94 - 7.63 (m, 2H), 2.29 - 2.25 (m, 3H).

### Step 2: Synthesis of compound 21-3

To a solition of compound **21-2** (1 g, 4.31 mmol) and potassium carbonate (1.19 g, 8.62 mmol) in N,N-dimethylformamide (3.5 mL) was added methyl iodide (734.06 mg, 5.17 mmol, 321.96 µL) at 25°C. The mixture was stirred at 60 °C for 4 hours. The reaction solution was diluted with water (10 mL), and extracted with ethyl acetate (30 mL×3). The combined organic phase was washed with saturated brine (30 mL×3), dried over anhydrous sodium sulfate, and filtered. The filtrate was concentrated under reduced pressure to give compound **21-3.**

¹H NMR (400 MHz, DMSO-d₆) δ 7.83 (br d, *J* = 2.1 Hz, 1H), 7.76 (br s, 1H), 3.87 (s, 3H), 2.34 (s, 3H).

### Step 3: Synthesis of compound 21-4

To a solution of compound **21-3** (2 g, 8.13 mmol, 1 eq) in ethanol (32 mL) and water (8 mL) were added iron (2.27 g, 40.64 mmol) and ammonium chloride (4.35 g, 81.28 mmol). The mixture was stirred at 80 °C for 16 hours. The reaction solution was filtered, and the filtrate was extracted with ethyl acetate (5 mL×3). The combined organic phase was washed with saturated brine (50 mL×3), dried over anhydrous sodium sulfate, and filtered. The filtrate was concentrated under reduced pressure to give compound **21-4.**

¹H NMR (400 MHz, DMSO-d₆) δ 6.52 (d,*J* = 1.5 Hz, 1H), 6.49 (s, 1H), 5.10 (br s, 2H), 3.65 (s, 3H), 2.11 (s, 3H).

### Step 4: Synthesis of compound 21-5

To a solution of compound **21-4** (1.7g, 7.87 mmol) in dioxane (25 mL) were added bis(pinacolato)diboron (3.00 g, 11.80 mmol), potassium acetate (2.32 g, 23.60 mmol), and 1,1'-bis(diphenylphosphino)ferrocene]palladium dichloride (287.84 mg, 393.38 µmol). The atmosphere was replaced with nitrogen three times. The mixture was stirred at 100 °C for 15 hours. The reaction solution was concentrated under reduced pressure, and the crude product was purified by column chromatography (silica gel, petroleum ether/ethyl acetate = 1/0 ~ 6/1) to give compound **21-5.**

¹H NMR (400 MHz, DMSO-d₆) δ 6.60 (s, 1H), 6.56 (s, 1H), 4.70 (br s, 2H), 3.60 (s, 3H), 2.11 (s, 3H), 1.27 (s, 12H).

### Step 5: Synthesis of compound 21-6

To a solution of compounds **21-5** (1.34 g, 5.11 mmol) and **1-10** (1 g, 4.86 mmol) in dioxane (16 mL) and water (4 mL) were added potassium phosphate (2.06 g, 9.72 mmol) and 1,1'-bis(diphenylphosphino)ferrocene]palladium dichloride (71.16 mg, 97.25 µmol). The mixture was stirred at 100°C for 3 hours. The reaction solution was concentrated under reduced pressure, and the crude product was purified by column chromatography (silica gel, petroleum ether/ethyl acetate = 1/0- 0/1) to give compound **21-6.**

MS m/z: 307[M+H]⁺.

### Step 6: Synthesis of compound 21

A solution of compounds **21-6** (200 mg, 652.78 µmol, **1-8** (200.31 mg, 783.33 µmol, cesium carbonate (425.38 mg, 1.31 mmol), 2,2-bis(diphenylphosphino)-1,1-binaphthyl (40.65 mg, 65.28 µmol) and tris(dibenzylideneacetone)dipalladium chloroform complex (28.89 mg, 32.64 µmol) in dioxane (10 mL) was pueged with nitrogen three times. The mixture was stirred at 100°C for 3 hours. The reaction solution was diluted with water (20 mL), and ethyl acetate (20 mL×3) was added. The resulting organic phase was washed with brine (10 mL×2) and dried over anhydrous sodium sulfate. The dried organic phase was concentrated under reduced pressure to give a crude product, which was purified by column chromatography (silica gel, petroleum ether/ethyl acetate = 1/1 ~ 0/1), and then separated by preparative high performance liquid chromatography (column: 3_Phenomenex Luna C18 75*30mm*3µm; mobile phase: [water (ammonia, 0.05%,v/v)-acetonitrile]; acetonitrile%: 34%-64%,8min) to give compound **21.**

¹H NMR (400 MHz, DMSO-d₆) δ 10.97 (s, 1H), 10.88 (s, 1H), 8.86 (s, 1H), 8.59 (s, 1H), 8.13 (s, 1H), 8.01 (s, 1H), 7.36 (s, 1H), 7.29 (s, 1H), 3.49 (s, 3H), 3.47 (s, 6H), 3.09 (s, 2H), 2.31 (s, 3H), 2.02 (quin,*J*= 5.9 Hz, 1H), 0.79 (br d, *J* = 5.5 Hz, 4H).

### Example 22

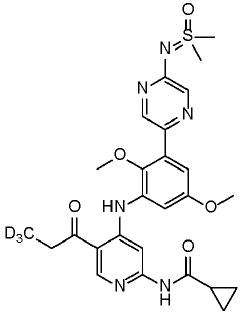

### Synthetic route:

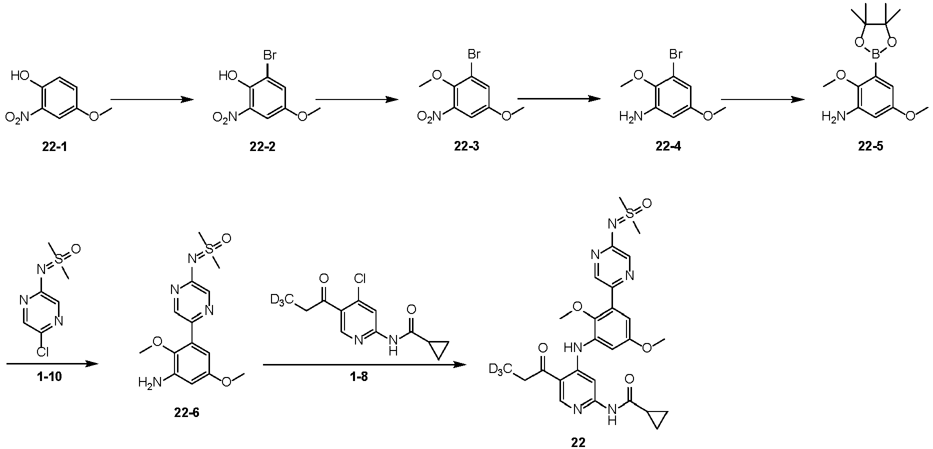

### Step 1: Synthesis of compound 22-2

Compound **22-1** (1 g, 5.91 mmol, 806.45 µL) was dissolved in water (5 mL) and glacial acetic acid (15 mL). Potassium bromide (703.59 mg, 5.91 mmol, 255.85 µL) and bromine (944.86 mg, 5.91 mmol, 304.79 µL) were added. The atmosphere was replaced with nitrogen three times. The mixture was stirred at 25 °C for 1 hour. The reaction solution was diluted with water (30 mL), and extracted with ethyl acetate (30 mL×3). The organic phase was dried over anhydrous sodium sulfate, and filtered. The filtrate was concentrated under reduced pressure, and then purified by column chromatography (silica gel, petroleum ether/ethyl acetate = 1/0 ~ 15/1) to give compound **22-2.**

¹H NMR (400 MHz, DMSO-d₆) δ 10.52 (br s, 1H), 7.63 (t, *J* = 2.6 Hz, 1H), 7.48 (dd, *J* = 1.4, 3.0 Hz, 1H), 3.78 (s, 3H).

### Step 2: Synthesis of compound 22-3

To a solition of compound **22-2** (1.2 g, 4.84 mmol) in N,N-dimethylformamide (5 mL) were added potassium carbonate (1.34 g, 9.68 mmol) and potassium iodide (824.06 mg, 5.81 mmol, 361.43 µL) at 20°C. The mixture was stirred at 60 °C for 4 hours. The reaction solution was quenched with saturated ammonium chloride solution (20 mL), diluted with water (10 mL), and extracted with ethyl acetate (30 mL×3). The combined organic phase was washed with saturated brine (30 mL×3), dried over anhydrous sodium sulfate, and filtered. The filtrate was concentrated under reduced pressure to give compound **22-3.**

¹H NMR (400 MHz,CDCl₃) δ 7.35 (d, *J* = 3.1 Hz, 1H), 7.29 (d, *J* = 3.1 Hz, 1H), 3.97 (s, 3H), 3.84 (s, 3H).

### Step 3: Synthesis of compound 22-4

To a solution of compound **22-3** (0.75 g, 2.86 mmol) in ethanol (5 mL) and water (5 mL) were added iron powders (799.13 mg, 14.31 mmol) and ammonium chloride (1.53 g, 28.62 mmol). The mixture was stirred at 80 °C for 6 hours. The reaction solution was diluted with water (40 mL), and extracted with ethyl acetate (50 mL×3). The combined organic phase was washed with saturated brine (50 mL×3), dried over anhydrous sodium sulfate, and filtered. The filtrate was concentrated under reduced pressure to give compound **22-4.**

¹H NMR (400 MHz, CDCl₃) δ 6.47 (d, *J* = 2.8 Hz, 1H), 6.25 (d, *J* = 2.9 Hz, 1H), 3.93 (br s, 2H), 3.79 (s, 3H), 3.72 (s, 3H).

### Step 4: Synthesis of compound 22-5

To a solution of compound **22-4** (700mg, 3.02 mmol) in dioxane (7 mL) were added bis(pinacolato)diboron (1.15 g, 4.52 mmol), potassium acetate (592.05 mg, 6.03 mmol), and 1,1'-bis(diphenylphosphino)ferrocene]palladium dichloride (44.14 mg, 60.33 µmol). The atmosphere was replaced with nitrogen three times. The reaction solution was stirred at 90 °C for 20 hours. Bis(pinacolato)diboron (382.97 mg, 1.51 mmol), potassium acetate (296.02 mg, 3.02 mmol) and 1,1'-bis(diphenylphosphino)ferrocene]palladium dichloride (44.14 mg, 60.33 µmol) were added to the reaction solution. The atmosphere was replaced with nitrogen three times. The mixture was stirred at 90 °C for 24 hours. The reaction solution was diluted with water (40 mL), and extracted with ethyl acetate (50 mL×3). The combined organic phase was washed with saturated brine (50 mL×3), dried over anhydrous sodium sulfate, and filtered. The filtrate was concentrated under reduced pressure, and purified by column chromatography (silica gel, petroleum ether/ethyl acetate = 100/1 ~ 10/1) to give compound **22-5.**

MS m/z: 280[M+H]⁺.

### Step 5: Synthesis of compound 22-6

To a solution of compounds **22-5** (354mg, 1.27 mmol) and **1-10** (230mg, 1.12 mmol) in dioxane (4 mL) and water (1 mL) were added potassium phosphate (474.76 mg, 2.24 mmol) and 1,1'-bis(diphenylphosphino)ferrocene]palladium dichloride (16.37 mg, 22.37 µmol). The mixture was stirred at 100 °C for 4 hours. The reaction solution was diluted with water (10 mL), and extracted with ethyl acetate (10 mL×3). The combined organic phase was washed with saturated brine (10 mL×3), dried over anhydrous sodium sulfate, and filtered. The filtrate was concentrated under reduced pressure, and the crude product was purified by column chromatography (silica gel, petroleum ether/ethyl acetate = 2/1 ~ 0/1) to give compound **22-6.**

MS m/z: 323[M+H]⁺.

### Step 6: Synthesis of compound 22

To a solution of compound **22-6** (126 mg, 390.84 µmol) in isopropanol (2 mL) was added concentrated hydrochloric acid (97.71 µmol, 9.44 µL), and the mixture was stirred at 80 °C for 16 hours. The reaction solution was concentrated under reduced pressure to remove isopropanol and concentrated hydrochloric acid to give a crude product. The crude product was separated by preparative high performance liquid chromatography (column: 3_Phenomenex Luna C18 75*30mm*3µm; mobile phase: [water (HCl, 0.05%v/v)-acetonitrile]; acetonitrile%: 19%-39%,8min) to give compound **22** hydrochloride.

MS m/z: 542[M+H]⁺;

¹H NMR (400 MHz, CDCl₃) δ 12.20 (br s, 1H), 11.61 (s, 1H), 9.03 (br s, 1H), 9.00 (br s, 1H), 8.42 (br s, 1H), 7.85 (s, 1H), 7.38 (br s, 1H), 7.03 (s, 1H), 3.93 (s, 3H), 3.57 (br s, 6H), 3.51 (br s, 3H), 3.09 (br s, 2H), 2.04 - 1.93 (m, 1H), 1.11 (br d, *J* = 3.5 Hz, 2H), 1.05 - 0.96 (m, 2H).

### Example 23

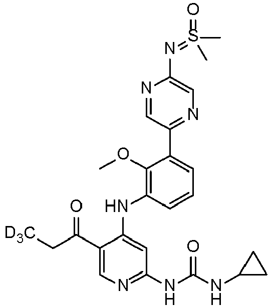

### Synthetic route:

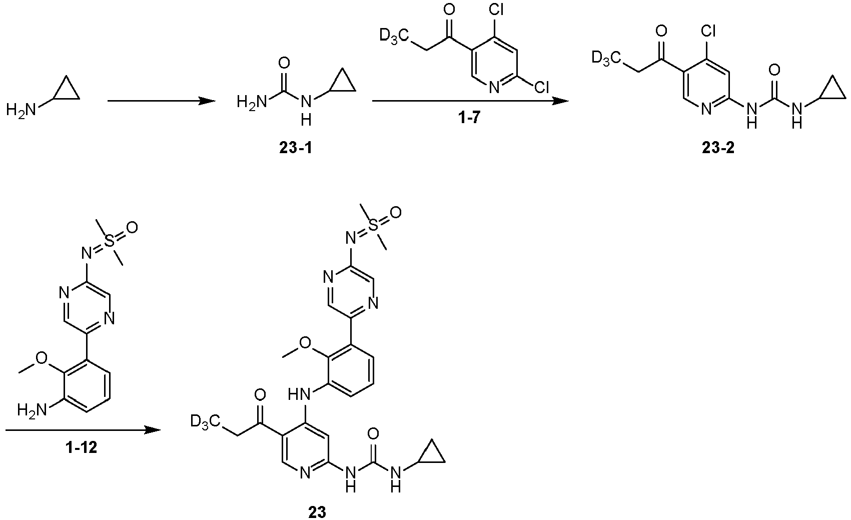

### Step 1: Synthesis of compound 23-1

Cyclopropylamine (1 g, 17.51 mmol, 1.21 mL) and potassium cyanate (1.70 g, 21.02 mmol, 827.61 µL) were dissolved in water (6 mL), and the mixture was refluxed at 100 °C for half an hour. The reaction mixture was concentrated under reduced pressure to remove the solvent. The residue was dissolved in isopropanol (10 mL). The solution was heated to 85°C and stirred for 30 minutes, and filtered. The filter cake was washed with isopropanol (10 mL), and the filtrate was concentrated under reduced pressure to give compound **23-1.**

¹H NMR (400 MHz, DMSO-d₆) δ 6.22 (br s, 1H), 5.51 (br s, 2H), 2.40 - 2.32 (m, 1H), 0.58 - 0.49 (m, 2H), 0.34 - 0.27 (m, 2H).

### Step 2: Synthesis of compound 23-2

Compound **1-7** (200 mg, 965.85 µmol) was dissolved in dioxane (4 mL). **23-1** (125.71 mg, 1.26 mmol), cesium carbonate (629.38 mg, 1.93 mmol), palladium acetate (21.68 mg, 96.58 µmol) and 4,5-bis(diphenylphosphino)-9,9-dimethylxanthene (111.77 mg, 193.17 µmol) were added. The atmosphere was replaced with nitrogen three times, and the mixture was stirred at 80°C for 3 hours. The reaction solution was directly concentrated under reduced pressure to give a crude product. The crude product was purified by column chromatography (silica gel, petroleum ether/ethyl acetate = 5/1 ~ 1/2) to give compound **23-2.**

MS m/z: 271[M+H]⁺;

¹H NMR (400 MHz, DMSO-d₆) δ 9.50 (br s, 1H), 8.65 (s, 1H), 7.77 (s, 1H), 7.55 (br s, 1H), 2.96 (s, 2H), 2.59 (dt, *J* = 3.4, 6.8 Hz, 1H), 0.71 - 0.63 (m, 2H), 0.48 - 0.41 (m, 2H).

### Step 3: Synthesis of compound 23

Compounds **23-2** (60 mg, 221.62 µmol) and **1-12** (61.55 mg, 210.54 µmol) were dissolved in isopropanol (2 mL). Hydrochloric acid (55.41 µmol, 5.35 µL, 37% purity) was added, and the mixture was stirred at 80 °C for 16 hours. The mixture was diluted with 30 mL of a mixed solution of ethyl acetate and ethanol (ethyl acetate/ethanol = 4/1), and washed with saturated sodium bicarbonate (10 mL × 3). The combined organic layer was washed with saturated brine (5 mL × 2), dried over anhydrous sodium sulfate, and filtered. The filtrate was concentrated under reduced pressure to give a crude product. The crude product was separated by preparative high performance liquid chromatography (column: Waters Xbridge 150*25mm*5µm; mobile phase: [water(10mM ammonium bicarbonate)-acetonitrile]; acetonitrile%: 34%-64%,8 min) to give compound **23.**

MS m/z: 527[M+H]⁺;

¹H NMR (400 MHz, DMSO-d₆) δ 10.98 (s, 1H), 9.15 (br s, 1H), 8.80 (s, 1H), 8.61 (d, *J* = 1.6 Hz, 1H), 8.14 (d, *J* = 1.5 Hz, 1H), 7.94 (br s, 1H), 7.53 (dd, *J* = 1.5, 7.8 Hz, 1H), 7.48 (dd, *J* = 1.5, 7.9 Hz, 1H), 7.36 (s, 1H), 7.33 - 7.26 (m, 1H), 3.52 (s, 3H), 3.47 (s, 6H), 3.06 (s, 2H), 2.60 - 2.54 (m, 1H), 0.68 - 0.61 (m, 2H), 0.44 - 0.37 (m, 2H).

### Example 24

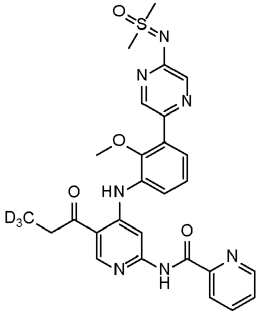

### Synthetic route:

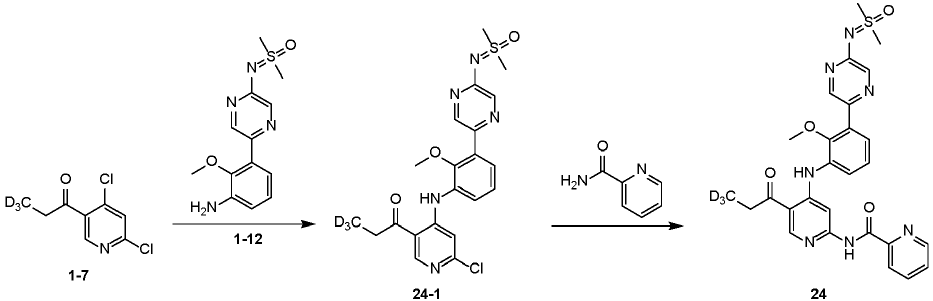

### Step 1: Synthesis of compound 24-1

**1-7** (1 g, 4.83 mmol) and **1-12** (1.27 g, 4.35 mmol) were dissolved in isopropanol (15 mL). 0.3 mL of concentrated hydrochloric acid was added, and the mixture was stirred at 70 °C for 16 hours. The reaction solution was filtered, and the filter cake was collected and washed with isopropyl alcohol to give a crude product. The crude product was stirred with a mixture of ethyl acetate and ethanol (ethyl acetate/ethanol = 8 mL/2 mL), and filtered. The filter cake was collected to give compound **24-1.**

MS m/z: 463[M+H]⁺;

¹H NMR (400 MHz, DMSO-d₆) δ 10.98 (s, 1H), 8.98 (s, 1H), 8.65 (s, 1H), 8.21 (s, 1H), 7.69 (d, *J* = 7.5 Hz, 1H), 7.58 (d, *J* = 7.6 Hz, 1H), 7.45 - 7.34 (m, 1H), 6.97 (s, 1H), 3.60 (s, 3H), 3.53 (s, 6H), 3.22 (s, 2H).

### Step 2: Synthesis of compound 24

A solution of **24-1** (50 mg, 108.00 µmol, 2-picolinamide (26.38 mg, 216.00 µmol, cesium carbonate (70.38 mg, 216.00 µmol, 2,2-bis(diphenylphosphino)-1,1-binaphthyl (12.50 mg, 21.60 µmol) and tris(dibenzylideneacetone)dipalladium chloroform complex (9.89 mg, 10.80 µmol) in dioxane (1.5 mL) was purged with nitrogen three times. The mixture was stirred at 110 °C for 16 hours. The reaction solution was concentrated under reduced pressure to give a crude product. The crude product was purified by column chromatography (silica gel, petroleum ether/ethyl acetate = 1/0 ~ 0/1), and then separated by preparative high-performance liquid chromatography (column: Waters Xbridge 75*30mm* 3µm; mobile phase: [water (HCl, 0.05%v/v)-acetonitrile]; acetonitrile%: 12%-42%,8min.) to give compound **24** hydrochloride.

MS m/z: 549[M+H]⁺;

¹H NMR (400 MHz, CDCl₃) δ 11.00 (s, 1H), 8.75 - 8.64 (m, 2H), 8.20 (d, *J* = 1.4 Hz, 1H), 8.14 (dd, *J* = 1.2, 4.9 Hz, 1H), 7.59 (s, 1H), 7.55 - 7.48 (m, 3H), 7.45 (br s, 1H), 7.25 - 7.21 (m, 1H), 7.17 (s, 1H), 6.79 (ddd, *J* = 0.8, 5.6, 6.7 Hz, 1H), 3.55 (s, 3H), 3.36 (s, 6H), 2.94 (s, 2H).

### Example 25

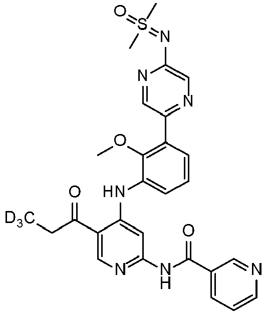

### Synthetic route:

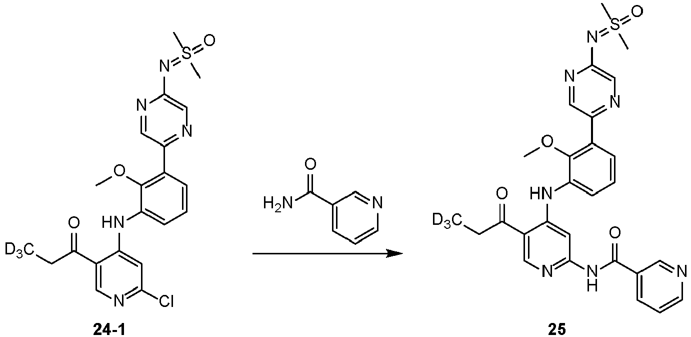

### Step 1: Synthesis of compound 25

A solution of **24-1** (100 mg, 216.00 µmol, nicotinamide (52.76 mg, 432.00 µmol, cesium carbonate (140.75 mg, 432.00 µmol, 2,2-bis(diphenylphosphino)-1,1-binaphthyl (25.00 mg, 43.20 µmol) and tris(dibenzylideneacetone)dipalladium chloroform complex (19.78 mg, 21.60 µmol) in dioxane (1.5 mL) was purged with nitrogen three times. The mixture was stirred at 110 °C for 16 hours. The reaction solution was concentrated under reduced pressure to give a crude product. The crude product was purified by column chromatography (silica gel, petroleum ether/ethyl acetate = 1/0 ~ 0/1), and then separated by preparative high-performance liquid chromatography (column: Waters Xbridge 75*30mm*3µm; mobile phase: [water (HCl, 0.05% v/v)-acetonitrile]; acetonitrile%: 4%-34%, 8min.) to give compound **25** hydrochloride.

MS m/z: 549[M+H]⁺;

¹H NMR (400 MHz, DMSO-d6) δ 12.10 - 11.77 (m, 1H), 11.30 (br d, *J* = 3.9 Hz, 1H), 9.29 - 9.17 (m, 1H), 9.04 (d, *J* = 2.9 Hz, 1H), 8.87 (br d, *J* = 3.3 Hz, 1H), 8.63 (d, *J* = 1.3 Hz, 1H), 8.56 - 8.41 (m, 1H), 8.17 (d, *J* = 1.3 Hz, 1H), 7.87 - 7.63 (m, 3H), 7.55 (br d, *J* = 7.8 Hz, 1H), 7.39 (t, *J* = 7.9 Hz, 1H), 3.57 (s, 3H), 3.47 (s, 6H), 3.17 (s, 2H).

### Example 26

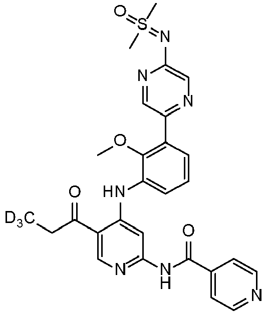

### Synthetic route:

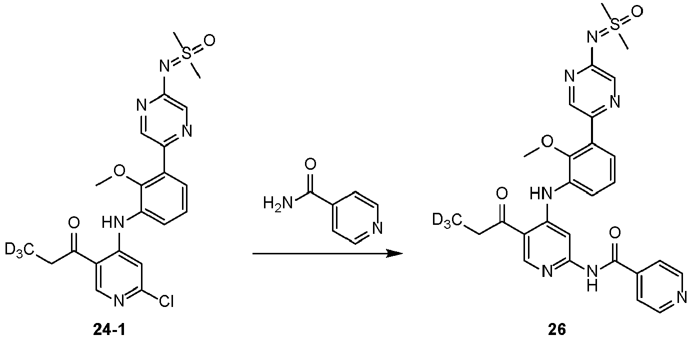

### Step 1: Synthesis of compound 26

A solution of **24-1** (100 mg, 216.00 µmol, 4-pyridinecarboxamide (52.76 mg, 432.00 µmol, cesium carbonate (140.75 mg, 432.00 µmol, 2,2-bis(diphenylphosphino)-1,1-binaphthyl (25.00 mg, 43.20 µmol) and tris(dibenzylideneacetone)dipalladium chloroform complex (19.78 mg, 21.60 µmol) in dioxane (1.5 mL) was purged with nitrogen three times. The mixture was stirred at 110 °C for 16 hours. The reaction solution was concentrated under reduced pressure to give a crude product. The crude product was purified by column chromatography (silica gel, petroleum ether/ethyl acetate = 1/0 ~ 0/1), and then separated by preparative high-performance liquid chromatography (column: Waters Xbridge 150*25mm*5µm; mobile phase: [water (HCl,0.05% V/V)-acetonitrile]; B%: 4%-34%,8min.) to give compound **26** hydrochloride.

MS m/z: 549[M+H]⁺;

¹H NMR (400 MHz, DMSO-d₆) δ 11.22 (br s, 1H), 11.07 (s, 1H), 8.97 (s, 1H), 8.81 - 8.70 (m, 2H), 8.63 (d, *J* = 1.5 Hz, 1H), 8.22 - 8.11 (m, 2H), 7.91 - 7.80 (m, 2H), 7.64 - 7.49 (m, 2H), 7.43 - 7.28 (m, 1H), 3.57 (s, 3H), 3.47 (s, 6H), 3.16 (s, 2H).

### Example 27

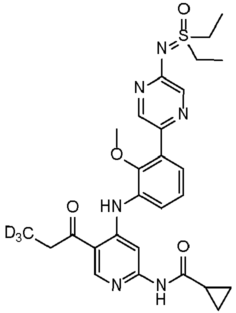

### Synthetic route:

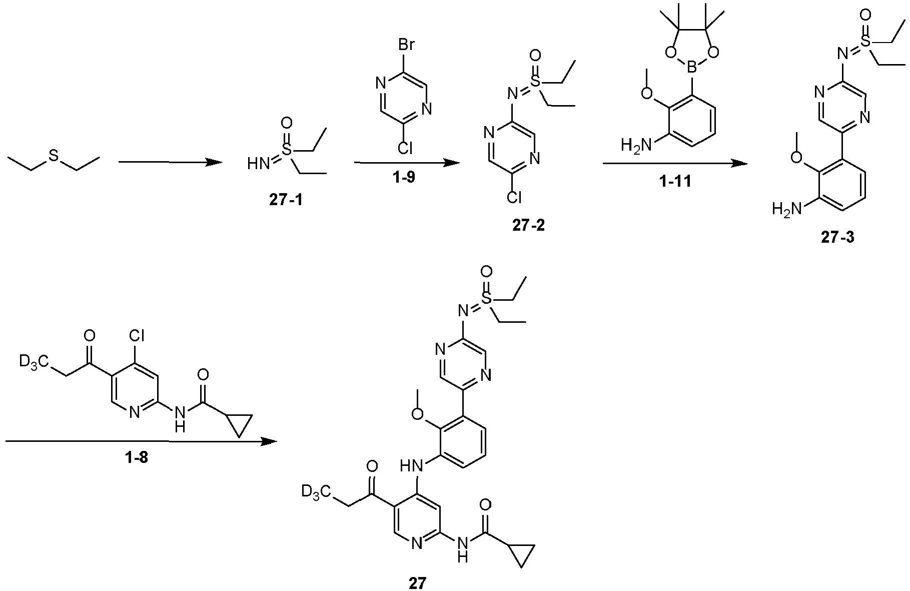

### Step 1: Synthesis of compound 27-1

To a solution of diethyl sulfide (2.32 g, 25.72 mmol, 2.77 mL) in anhydrous methanol (4 mL) were added iodobenzene diacetate (20.71 g, 64.31 mmol) and ammonium carbamate (4.02 g, 51.45 mmol), and the mixture was stirred at 20 °C for 16 hours. The reaction solution was concentrated under reduced pressure to give compound **27-1.**

¹H NMR (400 MHz, CDCl₃) δ 3.06 (q, *J* = 7.4 Hz, 4H), 1.46 - 1.37 (m, 6H).

### Step 2: Synthesis of compound 27-2

To a solution of compounds **27-1** (626.59 mg, 5.17 mmol) and **1-9** (1 g, 5.17 mmol) in dioxane (20 mL) were added cesium carbonate (3.37 g, 10.34 mmol), tris(dibenzylideneacetone)dipalladium (0) (236.71 mg, 258.49 µmol) and 4,5-bisdiphenylphosphino-9,9-dimethylxanthene (299.14 mg, 516.99 µmol). The atmosphere was replaced with nitrogen three times. The mixture was stirred at 110 °C for 2 hours. The reaction solution was diluted with water (30 mL), and extracted with ethyl acetate (50 mL×3). The organic phase was washed with saturated brine (30 mL×3), dried over anhydrous sodium sulfate, and filtered. The filtrate was concentrated under reduced pressure, and purified by column chromatography (silica gel, petroleum ether/ethyl acetate = 20/1 ~ 4/1) to give crude compound **27-2.**

MS m/z: 234[M+H]⁺.

### Step 3: Synthesis of compound 27-3

To a solution of compounds **27-2** (389 mg) and **1-11** (348.52 mg, 1.40 mmol) in dioxane (5 mL) and water (1 mL) were added potassium phosphate (539.94 mg, 2.54 mmol) and 1,1'-bis(diphenylphosphino)ferrocene]palladium dichloride (18.61 mg, 25.44 µmol). The atmosphere was replaced with nitrogen three times. The mixture was stirred at 100 °C for 18 hours. The reaction solution was diluted with water (15 mL), and extracted with ethyl acetate (30 mL×3). The combined organic phase was washed with saturated brine (30 mL×3), dried over anhydrous sodium sulfate, and filtered. The filtrate was concentrated under reduced pressure, and purified by column chromatography (silica gel, petroleum ether/ethyl acetate = 10/1 ~ 1/2) to give crude compound **27-3.**

MS m/z: 321[M+H]⁺;

¹H NMR (400 MHz, CDCl₃) δ 8.63 (s, 1H), 8.27 (s, 1H), 7.12 - 7.05 (m, 1H), 7.01 (dt, *J* = 2.3, 7.7 Hz, 1H), 6.79 (br d, *J* = 7.6 Hz, 1H), 4.13 (dq, *J* = 2.1, 7.1 Hz, 2H), 3.61 - 3.47 (m, 7H), 1.54 - 1.41 (m, 6H).

### Step 4: Synthesis of compound 27

To a solution of compound **27-3** (50 mg) in isopropanol (2 mL) were added **1-8** (40 mg, 156.42 µmol) and concentrated hydrochloric acid (3.34 mg, 33.91 µmol, 3.28 µL, 37% purity), and the mixture was stirred at 80 °C for 16 hours. Ammonia was added dropwise to the reaction solution to adjust the pH to 8~9. The reaction solution was concentrated under reduced pressure to remove isopropanol and concentrated hydrochloric acid to give a crude product. The crude product was separated by preparative high performance liquid chromatography (column: Waters Xbridge 150*25mm* 5µm; mobile phase: [water (10mM ammonium bicarbonate)-acetonitrile]; acetonitrile%: 41%-71%,8min) to give compound **27.**

MS m/z: 540[M+H]⁺;

¹H NMR (400 MHz, DMSO-d6) δ 11.02 (s, 1H), 10.90 (s, 1H), 8.87 (s, 1H), 8.56 (d, *J* = 1.5 Hz, 1H), 8.16 (d, *J* = 1.5 Hz, 1H), 8.02 (s, 1H), 7.52 (dd, *J* = 1.4, 7.8 Hz, 1H), 7.47 (dd, *J* = 1.3, 7.9 Hz, 1H), 7.32 - 7.24 (m, 1H), 3.59 (q, *J* = 7.4 Hz, 4H), 3.51 (s, 3H), 3.10 (s, 2H), 2.05 - 1.97 (m, 1H), 1.30 (t, *J* = 7.3 Hz, 6H), 0.78 (d, *J* = 6.0 Hz, 4H).

### Example 28

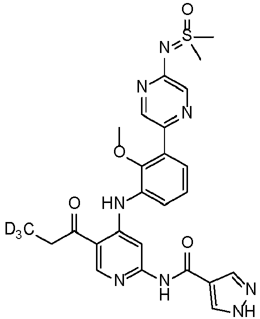

### Synthetic route:

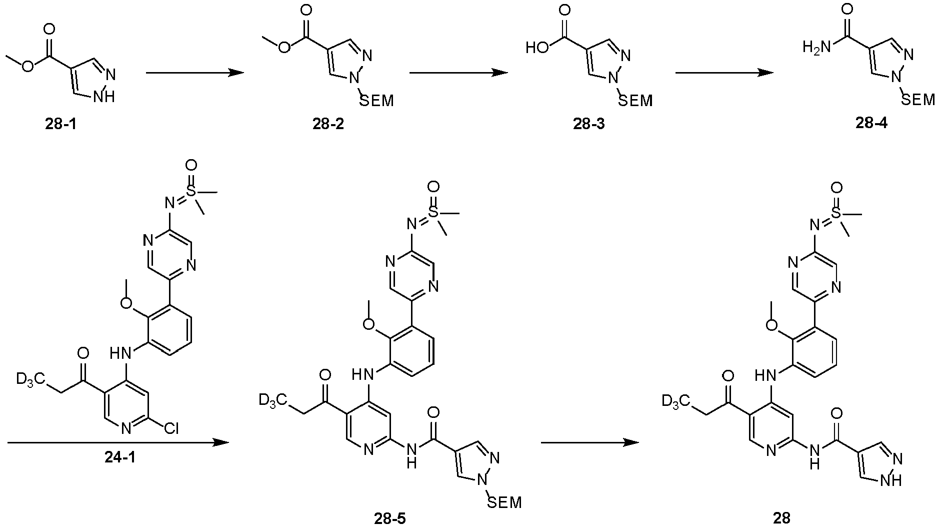

### Step 1: Synthesis of compound 28-2

Compound **28-1** (0.5 g, 3.96 mmol) was dissolved in N,N-dimethylformamide (5 mL), and the solution was cooled to 0 °C and stirred for 10 minutes. Then sodium hydride (190.29 mg, 4.76 mmol, 60% purity) was added to the reaction solution, and the mixture was stirred at 0 °C for 0.5 hours. 2-(Trimethylsilyl)ethoxymethyl chloride (727.10 mg, 4.36 mmol) was added to the reaction solution, and the mixture was stirred at 0 °C for 2 hours. Saturated aqueous ammonium chloride solution (5 mL) was added to the reaction solution. The mixture was diluted with water (10 mL), and extracted with ethyl acetate (10 mL×3). The organic phases were combined, washed with saturated brine (10 mL×1), dried over anhydrous sodium sulfate, and filtered. The filtrate was concentrated under reduced pressure to give a crude product. The crude product was purified by column chromatography (silica gel, petroleum ether/ethyl acetate = 1/0 ∼ 10/1) to give compound **28-2.**

MS m/z: 257[M+H]⁺;

¹H NMR (400 MHz, CDCl₃) δ 8.09 (s, 1H), 8.01 - 7.89 (m, 1H), 5.46 (s, 2H), 3.86 (s, 3H), 3.69 - 3.53 (m, 2H), 0.97 - 0.91 (m, 2H), 0.00 (s, 9H).

### Step 2: Synthesis of compound 28-3

Compound **28-2** (0.7 g, 2.73 mmol) was dissolved in tetrahydrofuran (3 mL) and methanol (3 mL). A solution of sodium hydroxide (0.25 g, 6.25 mmol) in water (3 mL) was added, and the mixture was stirred at 25 °C for 2 hours. The reaction solution was adjusted to pH of 3 with 2M aqueous hydrochloric acid solution, and filtered. The filter cake was washed with water (15 mL) to give compound **28-3.**

MS m/z: 243[M+H]⁺;

¹H NMR (400 MHz, DMSO-d₆) δ 12.88 - 11.99 (m, 1H), 8.46 (s, 1H), 7.92 (s, 1H), 5.48 (s, 2H), 3.68 - 3.56 (m, 2H), 0.88 (t, *J* = 8.0 Hz, 2H), 0.00 (s, 9H).

### Step 3: Synthesis of compound 28-4

Compound **28-3** (0.25 g, 1.03 mmol) was dissolved in N,N-dimethylformamide (2.5 mL). N,N-diisopropylethylamine (399.97 mg, 3.09 mmol), O-(7-azabenzotriazole-1-yl)-N,N,N,N-tetramethyluronium hexafluorophosphate (431.46 mg, 1.13 mmol), and 1-hydroxybenzotriazole (139.39 mg, 1.03 mmol) were added, and the mixture was stirred at 25 °C for 1 hour. Then ammonium chloride (275.90 mg, 5.16 mmol) was added and the mixture was stirred at 25 °C for 16 hours. Saturated brine (10 mL) was added to the reaction solution, and the mixture was extracted with ethyl acetate (10 mL×3). The organic phases were combined, washed with saturated brine (10 mL×1), dried over anhydrous sodium sulfate, and filtered. The filtrate was concentrated under reduced pressure to give a crude product. The crude product was purified by column chromatography (silica gel, petroleum ether/ethyl acetate = 3/1 ~ 0/1, dichloromethane/methanol = 10/1) to give compound **28-4.**

MS m/z: 242[M+H]⁺;

¹H NMR (400 MHz, DMSO-d₆) δ 8.28 (s, 1H), 7.89 (s, 1H), 7.61 (br s, 1H), 7.07 (br s, 1H), 5.41 (s, 2H), 3.57 - 3.49 (m, 2H), 0.94 - 0.74 (m, 2H), -0.05 (s, 9H).

### Step 4: Synthesis of compound 28-5

A solution of compound **28-4** (100 mg, 216.00 µmol, **24-1** (62.56 mg, 259.20 µmol, cesium carbonate (140.75 mg, 432.00 µmol, 2,2-bis(diphenylphosphino)-1,1-binaphthyl (25.00 mg, 43.20 µmol) and tris(dibenzylideneacetone)dipalladium chloroform complex (19.78 mg, 21.60 µmol) in dioxane (1.5 mL) was purged with nitrogen three times. The mixture was stirred at 110 °C for 16 hours. The reaction solution was concentrated under reduced pressure to give a crude product. The crude product was purified by thin-layer chromatography (silica gel, ethyl acetate) to give compound **28-5.**

MS m/z: 668[M+H]⁺.

### Step 5: Synthesis of compound 28

Compound **28-5** (0.04 g, 59.89 µmol) was dissolved in dichloromethane (2 mL). Trifluoroacetic acid (2 mL) was added, and the mixture was stirred at 25 °C for 1 hour. The reaction solution was concentrated under reduced pressure. Dichloromethane (5 mL) and saturated aqueous sodium bicarbonate solution (2 mL) were added. The layers were separated. The organic phase was washed respectively with saturated aqueous sodium bicarbonate solution (2 mL) and saturated brine (2 mL), dried over anhydrous sodium sulfate, and filtered. The filtrate was concentrated under reduced pressure to give a crude product. The crude product was separated by preparative high performance liquid chromatography (column: Waters Xbridge 150*25mm*5µm; mobile phase: [water (10mM ammonium bicarbonate)-acetonitrile]; acetonitrile%: 26%-56%,8min.) to give compound **28.**

MS m/z: 538[M+H]⁺;

¹H NMR (400 MHz, DMSO-d₆) δ 13.42 - 13.13 (m, 1H), 11.05 (s, 1H), 10.59 (s, 1H), 8.91 (s, 1H), 8.69 - 8.47 (m, 2H), 8.28 - 8.07 (m, 3H), 7.56 (d, *J* = 7.9 Hz, 2H), 7.45 - 7.26 (m, 1H), 3.55 (s, 3H), 3.47 (s, 6H), 3.13 (s, 2H).

### Example 29

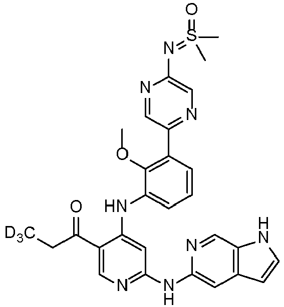

### Synthetic route:

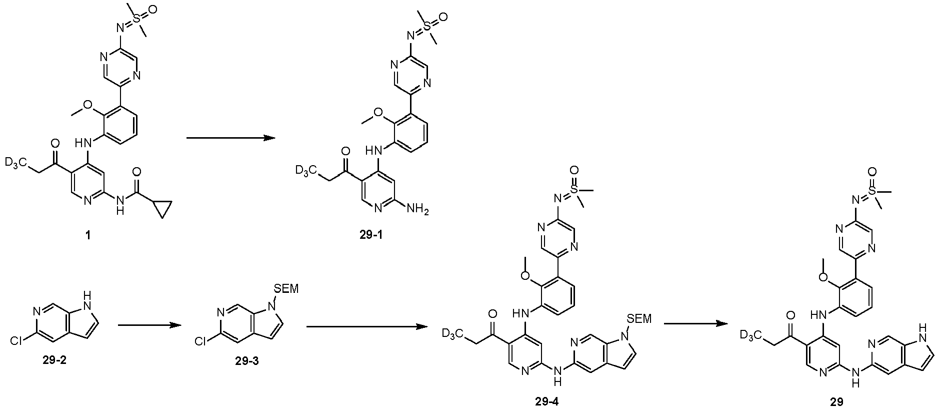

### Step 1: Synthesis of compound 29-1

Compound **1** (5 g, 9.77 mmol) was dissolved in water (25 mL) and ethanol (25 mL). Sodium hydroxide (781.79 mg, 19.55 mmol) was added. The mixture was stirred at 100 °C for 3 hours. The reaction solution was diluted with water (20 mL), and extracted with ethyl acetate (30 mL×3). The organic phases were combined, washed with brine (15 mL×2), dried over anhydrous sodium sulfate, and filtered. The filtrate was concentrated under reduced pressure to give a crude product. The crude product was purified by column chromatography (silica gel, petroleum ether/ethyl acetate = 1/1 ~ 0/1, ethyl acetate/ethanol = 10/1 -4/1) to give compound **29-2.**

MS m/z: 444[M+H]⁺;

¹H NMR (400 MHz, DMSO-d₆) δ 10.88 (s, 1H), 8.66 - 8.56 (m, 2H), 8.14 (d, *J* = 1.5 Hz, 1H), 7.47 (ddd, *J* = 1.4, 7.9, 11.1 Hz, 2H), 7.31 - 7.22 (m, 1H), 6.55 (br s, 2H), 6.12 (s, 1H), 3.54 (s, 3H), 3.47 (s, 6H), 2.94 (s, 2H).

### Step 2: Synthesis of compound 29-3

To a solution of compound **29-2** (500 mg, 3.28 mmol) in tetrahydrofuran (5mL) was added sodium hydride (157.28 mg, 3.93 mmol) under nitrogen at 0 °C, and the mixture was stirred at 0 °C for 0.5 hours. 2-(Trimethylsilyl)ethoxymethyl chloride (600.97 mg, 3.60 mmol) was added, and the mixture was stirred at 0°C for 2 hours. The mixture was extracted with ethyl acetate (10 mL×3). The combined organic phase was washed with saturated brine (20 mL×1), dried over anhydrous sodium sulfate, and filtered. The filtrate was concentrated under reduced pressure to give a crude product. The crude product was purified by column chromatography (silica gel, petroleum ether/ethyl acetate = 1/0- 8/1) to give compound **29-3.**

¹H NMR (400 MHz, CDCl₃) δ 8.70 (s, 1H), 7.57 (s, 1H), 7.48 - 7.36 (m, 1H), 6.53 (d, *J* = 3.1 Hz, 1H), 5.58 (s, 2H), 3.55 - 3.50 (m, 2H), 0.98 - 0.93 (m, 2H), 0.00 (s, 9H).

### Step 3: Synthesis of compound 29-4

Compound **29-3** (191.31 mg, 676.38 µmol) was dissolved in toluene (5 mL). **29-1** (200 mg, 450.92 µmol, cesium carbonate (293.84 mg, 901.84 µmol, tris(dibenzylideneacetone)dipalladium (41.29 mg, 45.09 µmol) and 2-dicyclohexylphosphino-2,4,6-triisopropylbiphenyl (42.99 mg, 90.18 µmol) were added. The atmosphere was replaced with nitrogen three times. The mixture was heated to 110 °C and stirred for 5 hours. The reaction solution was directly filtered, and the filtrate was concentrated under reduced presure to give a crude product. The crude product was purified by column chromatography (silica gel, petroleum ether/ethyl acetate = 10/1 ~ 1/5), and then separated by preparative high-performance liquid chromatography (column: Waters Xbridge C18 150*50mm* 10µm; mobile phase: [water (10mM ammonium bicarbonate)-acetonitrile]; acetonitrile%: 47%-77%,10min) to give compound **29-4.**

MS m/z: 690[M+H]⁺.

### Step 4: Synthesis of compound 29

Compound **29-4** (250 mg, 362.36 µmol) was dissolved in dichloromethane (10 mL). Trifluoroacetic acid (15.40 g, 135.06 mmol) was added, and the mixture was stirred at 25 °C for 3 hours. The reaction solution was concentrated. Ethanol (20 mL) and potassium carbonate (500.00 mg, 3.62 mmol) were added, and the mixture was stirred at 60 °C for 3 hours. The mixture was extracted with ethyl acetate (20 mL×3). The combined organic phase was washed with saturated brine (20 mL×1), dried over anhydrous sodium sulfate, and filtered. The filtrate was concentrated under reduced pressure to give a crude product. The crude product was purified by column chromatography (silica gel, petroleum ether/ethyl acetate = 1/0- 8/1, ethyl acetate/methanol = 100/1-30/1), and then separated by preparative high-performance liquid chromatography (column: Waters Xbridge 150*25mm* 5µm; mobile phase: [water (10mM ammonium bicarbonate)-acetonitrile]; acetonitrile%: 32%-62%,10min) to give compound **29.**

MS m/z: 560[M+H]⁺;

¹H NMR (400 MHz, DMSO-d₆) δ 11.34 (br s, 1H), 11.05 (s, 1H), 9.62 (s, 1H), 8.85 (s, 1H), 8.62 (s, 1H), 8.44 (s, 1H), 8.16 (s, 1H), 7.85 (br s, 1H), 7.65 (br d, *J* = 7.9 Hz, 1H), 7.59 - 7.54 (m, 2H), 7.49 (br d, *J* = 7.8 Hz, 1H), 7.39 - 7.26 (m, 1H), 6.42 (br s, 1H), 3.57 (s, 3H), 3.51 - 3.45 (m, 6H), 3.05 (s, 2H).

### Example 30

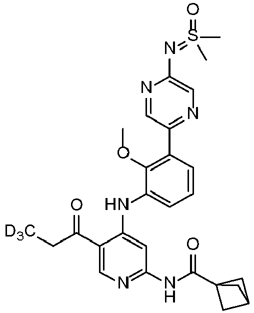

### Synthetic route:

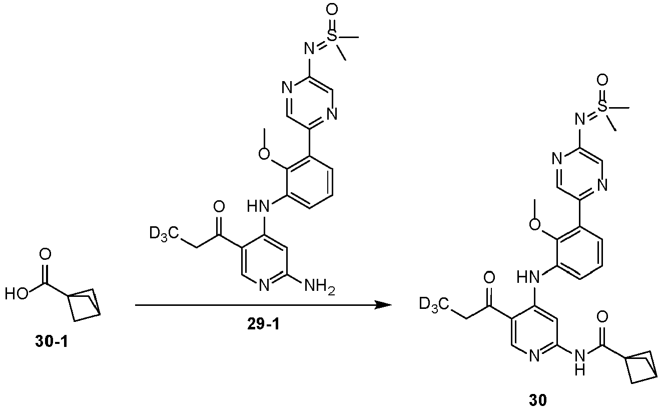

### Step 1: Synthesis of compound 30

Compound **30-1** (40.45 mg, 360.74 µmol) was dissolved in N,N-dimethylformamide (2 mL). N,N-diisopropylethylamine (153.85 mg, 1.19 mmol) and O-(7-azabenzotriazole-1-yl)-N,N,N,N-tetramethyluronium hexafluorophosphate (150.88 mg, 396.81 µmol) were added, and the mixture was stirred at 25 °C for 1 hour. Then compound **29-1** (0.08 g, 180.37 µmol) was added to the reaction solution and the mixture was stirred at 25 °C for 16 hours. Water (10 mL) and ethyl acetate (10 mL) were added to the reaction solution. The layers were separated. The aqueous phase was extracted with ethyl acetate (10 mLx3). The organic phases were combined, washed with saturated brine (10 mLx2), dried over anhydrous sodium sulfate, and filtered. The filtrate was concentrated under reduced pressure to give a crude product. The crude product was separated by preparative high performance liquid chromatography (column: Waters Xbridge 150*50mm*10µm; mobile phase: [water (10mM ammonium bicarbonate)-acetonitrile]; acetonitrile%: 33%-63%,10min.) to give compound **30.**

MS m/z: 538[M+H]⁺;

¹H NMR (400 MHz, DMSO-d₆) δ 11.02 (s, 1H), 10.35 (s, 1H), 8.89 (s, 1H), 8.61 (d, *J* = 1.5 Hz, 1H), 8.15 (d, *J* = 1.5 Hz, 1H), 8.04 (s, 1H), 7.61 - 7.48 (m, 2H), 7.41 - 7.22 (m, 1H), 3.54 (s, 3H), 3.48 (s, 6H), 3.32 - 3.30 (m, 3H), 2.09 (s, 6H).

### Example 31

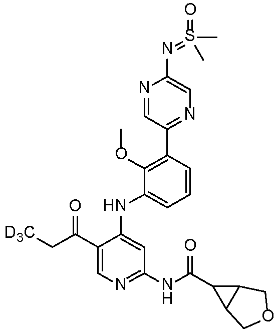

### Synthetic route:

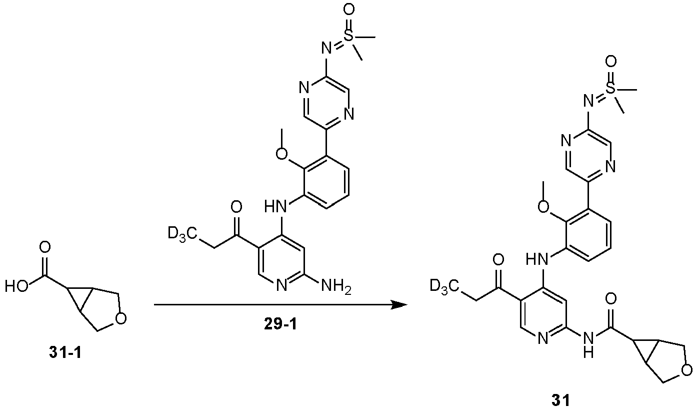

### Step 1: Synthesis of compound 31

Compound **31-1** (50 mg, 390.24 µmol) was dissolved in N,N-dimethylformamide (2 mL). N,N-diisopropylethylamine (151.30 mg, 1.17 mmol, 203.91 µL) and O-(7-azabenzotriazole-1-yl)-N,N,N,N-tetramethyluronium hexafluorophosphate (163.22 mg, 429.27 µmol) were added, and the mixture was stirred at 15 °C for 20 minutes. Compound **29-1** (86.54 mg, 195.12 µmol) was added to the mixture, and the mixture was stirred at 60 °C for 16 hours.The reaction solution was diluted with water (10 mL), and extracted with ethyl acetate (20 mL×3). The organic phases were combined, washed with brine (5 mL×3), dried over anhydrous sodium sulfate, and filtered. The filtrate was concentrated under reduced pressure to give a crude product. The crude product was purified by column chromatography (silica gel, petroleum ether/ethyl acetate = 1/1 ~ 0/1), and then separated by preparative high performance liquid chromatography (column: Waters Xbridge 150*25mm* 5µm; mobile phase: [water (10mM ammonium bicarbonate)-acetonitrile]; acetonitrile %: 31%-61%,8min) to give compound **31.**

MS m/z: 554[M+H]⁺;

¹H NMR (400 MHz, DMSO-d₆) δ 11.02 (s, 1H), 10.82 (s, 1H), 8.88 (s, 1H), 8.59 (d, *J* = 1.3 Hz, 1H), 8.14 (d, *J* = 1.5 Hz, 1H), 8.00 (s, 1H), 7.53 (dd, *J* = 1.5, 7.8 Hz, 1H), 7.47 (dd, *J* = 1.5, 7.9 Hz, 1H), 7.33 - 7.26 (m, 1H), 3.80 (d, *J* = 8.7 Hz, 2H), 3.63 (d, *J* = 8.4 Hz, 2H), 3.52 (s, 3H), 3.47 (s, 6H), 3.10 (s, 2H),2.07 (d, *J* = 1.8 Hz, 2H), 1.94 (br d, *J* = 2.1 Hz, 1H).

### Example 32

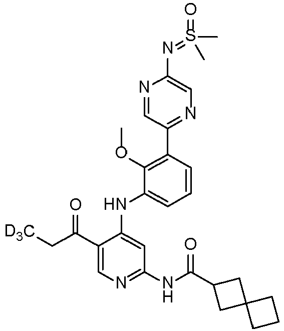

### Synthetic route:

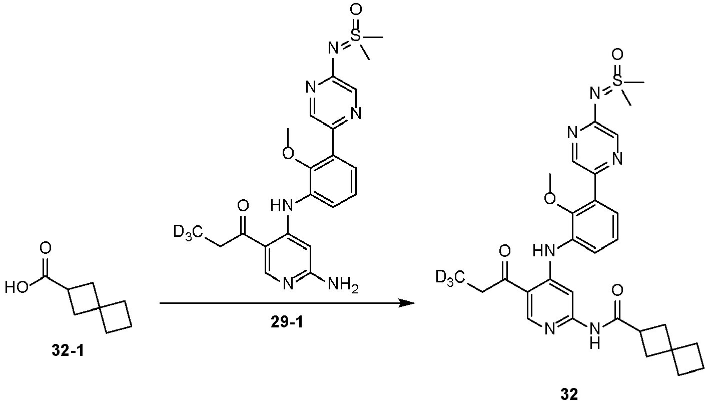

### Step 1: Synthesis of compound 32

To a solution of compound **32-1** (94.81 mg, 676.38 µmol) in N,N-dimethylformamide (4 mL) were added N,N-diisopropylethylamine (262.25 mg, 2.03 mmol, 353.44 µL) and 2-(7-azabenzotriazole)-N,N,N',N'-tetramethyluronium hexafluorophosphate (282.90 mg, 744.02 µmol), and the mixture was stirred at 20 °C for 1 hour. Then **29-1** (100 mg, 225.46 µmol) was added. The mixture was stirred at 60 °C under nitrogen for 3 hours. The reaction solution was diluted with water (15 mL), and extracted with ethyl acetate (20 mL×3). The organic phase was washed successively with water (20 mL×3) and saturated brine (20 mL×3), dried over anhydrous sodium sulfate, and filtered. The filtrate was concentrated under reduced pressure to give a crude product. The crude product was separated by preparative high performance liquid chromatography (column: Waters Xbridge C18 150*50mm* 10µm; mobile phase: [water (10mM ammonium bicarbonate)-acetonitrile]; acetonitrile%: 40%-70%,10 min) to give compound **32.**

MS m/z: 566[M+H]⁺;

¹H NMR (400 MHz, DMSO-d₆) δ 11.01 (s, 1H), 10.41 (s, 1H), 8.84 (s, 1H), 8.59 (d, *J* = 1.5 Hz, 1H), 8.13 (d, *J* = 1.3 Hz, 1H), 8.04 (s, 1H), 7.53 (dd, *J* = 1.4, 7.8 Hz, 1H), 7.49 (dd, *J* = 1.4, 7.9 Hz, 1H), 7.34 - 7.26 (m, 1H), 3.51 (s, 3H), 3.45 (s, 6H), 3.18 (t, *J* = 8.3 Hz, 1H), 3.08 (s, 2H), 2.11 - 2.05 (m, 4H), 1.98 (br t, *J* = 6.8 Hz, 2H), 1.85 - 1.78 (m, 2H), 1.77 - 1.69 (m, 2H).

### Example 33

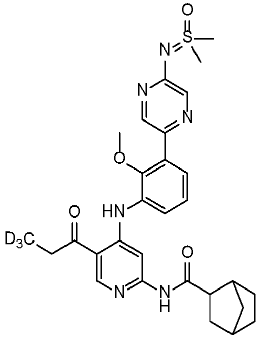

### Synthetic route:

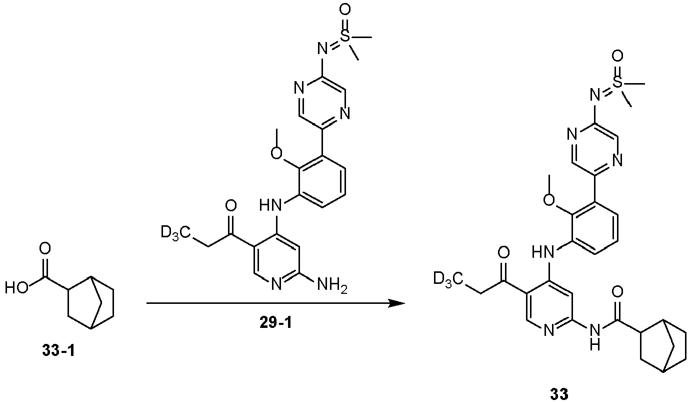

### Step 1: Synthesis of compound 33

Compound **33-1** (50.00 mg, 356.69 µmol) was dissolved in acetonitrile (1.5 mL). Methylimidazole (102.50 mg, 1.25 mmol, 99.51 µL) and N,N,N,N-tetramethylchloroformamidinium hexafluorophosphate (120.09 mg, 428.02 µmol) were added, and the mixture was stirred at 15 °C for 20 minutes. Compound **29-1** (79.10 mg, 178.34 µmol) was added to the mixture, and the mixture wsa stirred at 15 °C for 16 hours. The reaction solution was diluted with water (10 mL), and extracted with ethyl acetate (20 mL×3). The organic phases were combined, washed with brine (5 mL×2), dried over anhydrous sodium sulfate, and filtered. The filtrate was concentrated under reduced pressure to give a crude product. The crude product was separated by preparative high performance liquid chromatography (column: Waters Xbridge 150*25mm* 5µm; mobile phase: [water (10mM ammonium bicarbonate)-acetonitrile]; acetonitrile%: 45%-75%,8min) to give compound **33.**

MS m/z: 566[M+H]⁺;

¹H NMR (400 MHz, DMSO-d₆) δ 11.04 (s, 1H), 10.46 (s, 1H), 8.86 (s, 1H), 8.61 (d, *J* = 1.5 Hz, 1H), 8.15 (d, *J* = 1.5 Hz, 1H), 8.06 (s, 1H), 7.54 (dd, *J* = 1.5, 7.8 Hz, 1H), 7.51 (dd, *J* = 1.4, 7.9 Hz, 1H), 7.37 - 7.27 (m, 1H), 3.53 (s, 3H), 3.47 (s, 6H), 3.10 (s, 2H), 2.97 - 2.89 (m, 1H), 1.62 - 1.54 (m, 1H), 1.53 - 1.34 (m, 3H), 1.33 - 1.11 (m, 6H).

### Example 34

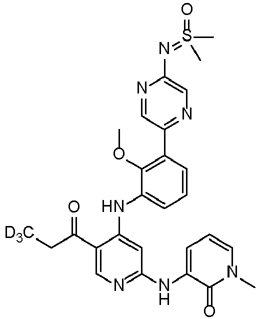

### Synthetic route:

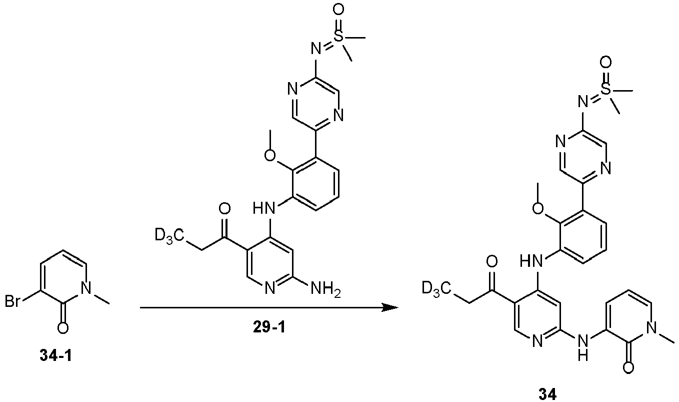

### Step 1: Synthesis of compound 34

Compound **29-1** (50 mg, 112.73 µmol) was dissolved in dioxane (2 mL). **34-1** (21.20 mg, 112.73 µmol), cesium carbonate (110.19 mg, 338.19 µmol), tris(dibenzylideneacetone)dipalladium (10.32 mg, 11.27 µmol) and 2-dicyclohexylphosphino-2,4,6-triisopropylbiphenyl (10.75 mg, 22.55 µmol) were added. The atmosphere was replaced with nitrogen three times. The mixture was stirred at 80 °C for 16 hours. The reaction solution was diluted with water (10 mL), and extracted with ethyl acetate (20 mL×3). The organic phases were combined, washed with brine (5 mL×2), dried over anhydrous sodium sulfate, and filtered. The filtrate was concentrated under reduced pressure to give a crude product. The crude product was separated by column chromatography (silica gel, petroleum ether/ethyl acetate = 1/1 ~ 0/1), and then separated by preparative high performance liquid chromatography (column: Waters Xbridge 150*25mm* 5µm; mobile phase: [water (10mM ammonium bicarbonate)-acetonitrile]; acetonitrile%: 36%-66%,8min) to give compound **34.**

MS m/z: 551[M+H]⁺;

¹H NMR (400 MHz, DMSO-d₆) δ 11.00 (s, 1H), 8.95 (s, 1H), 8.86 (s, 1H), 8.61 (d, *J* = 1.3 Hz, 1H), 8.48 (d, *J* = 7.5 Hz, 1H), 8.15 (d, *J* = 1.3 Hz, 1H), 7.61 (dd, *J* = 1.1, 7.9 Hz, 1H), 7.48 (dd, *J* = 1.3, 7.8 Hz, 1H), 7.34 - 7.24 (m, 2H), 7.06 (s, 1H), 6.24 (t, *J* = 7.2 Hz, 1H), 3.55 (s, 3H), 3.52 (s, 3H), 3.47 (s, 6H), 3.05 (s, 2H).

### Example 35

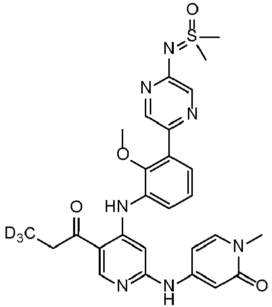

### Synthetic route:

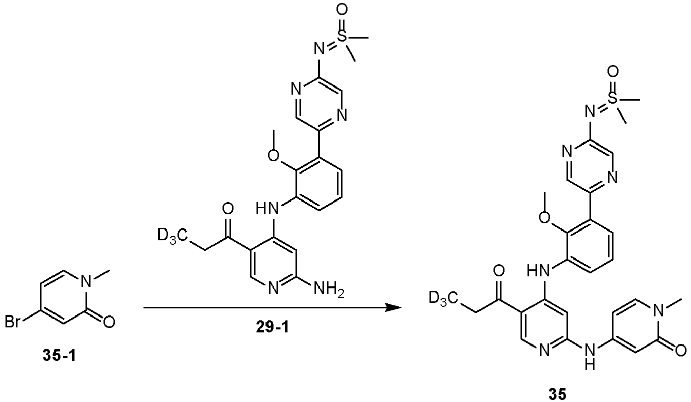

### Step 1: Synthesis of compound 35

Compound **29-1** (50 mg, 112.73 µmol) was dissolved in dioxane (2 mL). **35-1** (25.43 mg, 135.28 µmol), cesium carbonate (110.19 mg, 338.19 µmol), tris(dibenzylideneacetone)dipalladium (10.32 mg, 11.27 µmol) and 2-dicyclohexylphosphino-2,4,6-triisopropylbiphenyl (10.75 mg, 22.55 µmol) were added. The atmosphere was replaced with nitrogen three times. The mixture was stirred at 100 °C for 16 hours. The reaction solution was diluted with water (10 mL), and extracted with ethyl acetate (20 mL×3). The organic phases were combined, washed with brine (5 mL×2), dried over anhydrous sodium sulfate, and filtered. The filtrate was concentrated under reduced pressure to give a crude product. The crude product was separated by column chromatography (silica gel, petroleum ether/ethyl acetate = 1/1 ~ 0/1), and then separated by preparative high performance liquid chromatography (column: Waters Xbridge 150*25mm* 5µµm; mobile phase: [water (10mM ammonium bicarbonate)-acetonitrile]; acetonitrile%: 27%-57%, 8min) to give compound **35.**

MS m/z: 551[M+H]⁺;

¹H NMR (400 MHz, DMSO-d₆) δ 10.81 (s, 1H), 9.44 (s, 1H), 8.90 (s, 1H), 8.61 (d, *J* = 1.3 Hz, 1H), 8.15 (d, *J* = 1.3 Hz, 1H), 7.57 (dd, *J* = 1.2, 7.7 Hz, 1H), 7.53 - 7.45 (m, 2H), 7.36 - 7.28 (m, 1H), 7.01 (d, *J* = 2.3 Hz, 1H), 6.53 (s, 1H), 6.34 (dd, *J* = 2.3, 7.5 Hz, 1H), 3.54 (s, 3H), 3.47 (s, 6H), 3.31 (br s, 3H), 3.08 (s, 2H).

### Biological evaluation

### Assay example 1: Evaluation of enzymatic activity in vitro

### Assay process for Tyk2 JH2 enzymatic activity assay

0.5 nM TYK2 protein (His-TVMV-TYK2 JH2 (575 - 869)), 0.2 nM terbium-labeled His antibody, fluorescein-labeled kinase tracer at the relevant K_{d} value, and the assay compound were added to a buffer solution containing 20 mM Hepes pH 7.5, 10 mM MgCl₂, 0.015% Brij-35, 2 mM DTT, and 50 µg/mL BSA. The assay system was incubated at room temperature for 90 minutes. The resulting HTRF (homogeneous time-resolved fluorescence) signal, i.e., the ratio of the fluorescence intensity of the fluorescein acceptor (520 nm) and the terbium donor (495 nm) at the emission wavelength, was then measured on an Envision plate reader, and based on this, the IC50 value was calculated. The results of the in vitro enzymatic activity assay of the compounds of the present discloure are shown in Table 1:

**Table 1: Assay results of the compounds of the present discloure on enzymatic activity in vitro (IC₅₀)**

| No. | Tyk2 JH2 (IC₅₀ nM) |
|---|---|
| Compound 1 | 0.04 |
| Compound 2 | 0.06 |
| Compound 3 | 0.03 |
| Compound 4 | 0.07 |
| Compound 5 | 0.07 |
| Compound 6 | 0.18 |
| Compound 7 | 0.09 |
| Compound 8 | 0.15 |
| Compound 9 | 0.07 |
| Compound 10 | 0.06 |
| Compound 11 | 0.11 |
| Compound 12 | 0.05 |
| Compound 13 | 0.12 |
| Compound 14 | 0.25 |
| Compound 15 | 0.08 |
| Compound 16 | 0.08 |
| Compound 17 | 0.29 |
| Compound 18 | 0.09 |
| Compound 19 | 0.12 |
| Compound 20 | 0.19 |
| Compound 21 | 0.13 |
| Compound 22 hydrochloride | 0.14 |
| Compound 23 | 0.13 |
| Compound 27 | 0.09 |
| Compound 28 | 0.17 |
| Compound 29 | 0.11 |
| Compound 30 | 0.56 |
| Compound 31 | 0.34 |
| Compound 32 | 0.31 |
| Compound 33 | 0.30 |
| Compound 35 | 0.76 |

Assay conclusions: The compounds of the present disclosure have strong inhibitory activity against Tyk2 JH2.

### Assay example 2: Evaluation of in vitro cell activity

### Assay of IFNα-stimulated phosphorylated STAT1

Human peripheral blood mononuclear cells (hPBMC) were plated at a cell density of 1×10⁵ cells/well and placed in a 37°C incubator for 90 minutes. Different concentrations of compounds were then added to the cells, wherein the concentration of each compound started from 2 µM and was serially diluted 5-fold to a total of 8 concentrations. Compounds and cells were incubated at 37 °C for 30 minutes. IFN-a (1000 U/ml) was used to stimulate hPBMC, and then flow cytometry was used to detect the level of phosphorylated STAT1 in CD4+ T cells to evaluate the inhibitory activity of compounds on the IFN-α pathway. The assay results of the compounds of the present disclosure on cell activity *in vitro* are shown in Table 2:

**Table 2: Assay results of the compounds of the present disclosure on cell activity in vitro (IC₅₀)**

| No. | IFNα-stimulated phosphorylated STAT1 (IC₅₀ nM) |
|---|---|
| Compound 1 | 1.1 |
| Compound 2 | 0.2 |
| Compound 3 | 2.1 |
| Compound 4 | 0.6 |
| Compound 8 | 0.4 |
| Compound 9 | 0.5 |

Assay conclusions: The compounds of the present disclosure have strong cell activity against Tyk2-related IFNα-stimulated phosphorylated STAT1.

### Assay example 3: Evaluation of the pharmacokinetics of the compound in mice

Purpose of assay: To assay the pharmacokinetics of the compound in Balb/c mice
Assay materials: Male Balb/c mice, fasted

### Assay procedure:

The pharmacokinetic profiles of compound 1 in rodents after intravenous injection and oral administration were assayed using standard protocols.

Acclimatization/quarantine of Balb/c mice was performed for at least 3 days after arrival at the facility. Upon completion of the acclimatization/quarantine, a veterinarian or designee examined the health status of the Balb/c mice to evaluate whether the animals were suitable for the assay study. All Balb/c mice were fasted overnight before administration and resumed feeding 4 hours after administration. In the assay, the candidate compound was formulated into a homogeneous solution and given to Balb/c mice via a single intravenous injection and oral administration. The intravenous vehicle was a clear solution of 80% polyethylene glycol 400/20% water and the oral vehicle was a homogeneous suspension of ethanol/vitamin E polyethylene glycol succinate/polyethylene glycol 300 (5/5/90). The animals were weighed before administration and the administered volume was calculated based on body weight. Whole blood samples were collected by means of jugular vein puncture within 24 hours. All blood samples were immediately transferred to labeled commercial centrifuge tubes containing K2-EDTA. After blood samples were collected, the blood samples were centrifuged at 3200 g for 10 minutes at 4°C. The supernatant plasma was aspirated, quickly placed on dry ice, and then stored at -60°C or lower for LC-MS/MS analysis. The data of blood drug concentration-time were analyzed using the WinNonlin software package (Version 6.3 and above) using a non-compartmental model. Pharmacokinetic parameters were calculated, including (if data permitted), but not limited to, peak concentration (Cmax), time to peak (Tmax), elimination half-life (T1/2), area under plasma concentration-time curve (AUC), mean retention time (MRT), bioavailability, etc. The assay results of compound 1 of the present disclosure are shown in Table 3:

**Table 3 Results of the pharmacokinetic assay**

| IV (1mg/kg) | | | | PO (10mg/kg) | | | |
|---|---|---|---|---|---|---|---|
| Vd (L/kg) | Cl (mL/Kg/min) | T_{1/2} (h) | AUC (nM.h) | Cmax (nM) | Tmax (h) | AUC (nM.h) | F (%) |
| 0.45 | 3.39 | 1.61 | 9381 | 13400 | 0.75 | 47250 | 51.4 |

| | | | | | | | |
|---|---|---|---|---|---|---|---|
| Note: Vd: volume of distribution; Cl: clearance rate; T_{1/2}: half-life; AUC: exposure (area under the curve); Cₘₐₓ: maximum concentration; Tₘₐₓ: time to peak concentration; F%: bioavailability; IV: intravenous injection; PO: oral administration | | | | | | | |

Assay conclusions: The compound of the present disclosure shows excellent pharmacokinetic properties, low clearance rate, and high oral bioavailability.

## Claims

1. A compound of formula (II) or a pharmaceutically acceptable salt thereof, 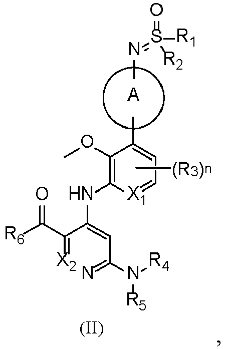 wherein
ring A is 6-membered heteroaryl;
X₁ and X₂ are each independently selected from N and CH;
R₁ and R₂ are independently selected from C₁₋₃ alkyl, wherein the C₁₋₃ alkyl is optionally substituted with 1, 2, 3 or 4 Rₐ;
alternatively, R₁ and R₂ are taken together with the S atom to which they are attached to form 4- to 6-membered heterocycloalkyl, wherein the 4- to 6-membered heterocycloalkyl is optionally substituted with 1, 2, 3 or 4 Rₐ;
each R₃ is independently selected from hydrogen, deuterium, fluorine, chlorine, bromine, C₁₋₃ alkyl and C₁₋₃ alkoxy;
R₄ is selected from hydrogen, -C(=O)R₄₁, -C(=O)NR₄₂R₄₃, 5- to 10-membered heteroaryl and phenyl, wherein the 5- to 10-membered heteroaryl and phenyl are optionally substituted with 1, 2 or 3 R_{b};
R₄₁ is selected from C₁₋₃ alkyl, C₃₋₈ cycloalkyl, 5- to 6-membered heteroaryl, phenyl and 4- to 6-membered heterocycloalkyl, wherein the C₁₋₃ alkyl, C₃₋₈ cycloalkyl, 5- to 6-membered heteroaryl, phenyl and 4- to 6-membered heterocycloalkyl are optionally substituted with 1, 2, 3 or 4 R_{c};
R₄₂ is selected from hydrogen and C₁₋₃ alkyl;
R₄₃ is selected from C₁₋₃ alkyl, C₃₋₈ cycloalkyl, 5- to 6-membered heteroaryl and 4- to 6-membered heterocycloalkyl, wherein the C₁₋₃ alkyl, C₃₋₈ cycloalkyl, 5- to 6-membered heteroaryl and 4- to 6-membered heterocycloalkyl are optionally substituted with 1, 2, 3 or 4 R_{c};
R₅ is selected from hydrogen and C₁₋₃ alkyl;
R₆ is selected from C₁₋₃ alkyl, C₁₋₃ alkoxy, C₃₋₆ cycloalkyl, -NH-C₁₋₃ alkyl and -NH-C₃₋₆ cycloalkyl, wherein the C₁₋₃ alkyl, C₁₋₃ alkoxy, C₃₋₆ cycloalkyl, -NH-C₁₋₃ alkyl and -NH-C₃₋₆ cycloalkyl are optionally substituted with 1, 2, 3 or 4 R_{d};
Rₐ, R_{b}, R_{c} and R_{d} are independently selected from H, deuterium, fluorine, chlorine, bromine, iodine, CN, NH₂, C₁₋₃ alkyl and C₁₋₃ alkoxy;
n is selected from 0, 1, 2 and 3.

2. The compound according to claim 1 or a pharmaceutically acceptable salt thereof, ring A is selected from

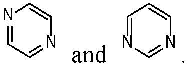

3. The compound according to claim 2 or a pharmaceutically acceptable salt thereof, ring A is selected from

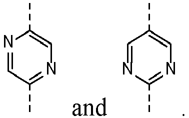

4. The compound according to claim 1 or a pharmaceutically acceptable salt thereof, R₁ and R₂ are independently selected from methyl, ethyl and propyl, wherein the methyl, ethyl and propyl are optionally substituted with 1, 2, 3 or 4 Rₐ.

5. The compound according to claim 4 or a pharmaceutically acceptable salt thereof, R₁ and R₂ are independently selected from methyl.

6. The compound according to claim 1 or a pharmaceutically acceptable salt thereof, R₁ and R₂ are taken together with the S atom to which they are attached to form 4- to 6-membered heterocycloalkyl, wherein the 4- to 6-membered heterocycloalkyl is optionally substituted with 1, 2, 3 or 4 Rₐ.

7. The compound according to claim 6 or a pharmaceutically acceptable salt thereof, the 4- to 6-membered heterocycloalkyl is selected from 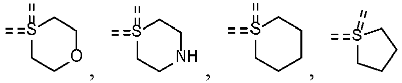 and 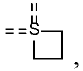 wherein the 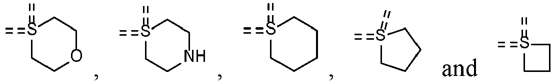 are optionally substituted with 1, 2, 3 or 4 Rₐ.

8. The compound according to claim 7 or a pharmaceutically acceptable salt thereof, the 4- to 6-membered heterocycloalkyl is selected from 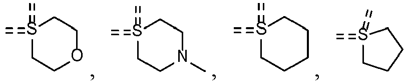 and

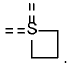

9. The compound according to any one of claims 1-7 or a pharmaceutically acceptable salt thereof, Rₐ is selected from hydrogen.

10. The compound according to claim 1 or a pharmaceutically acceptable salt thereof, structural moiety 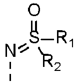 is selected from

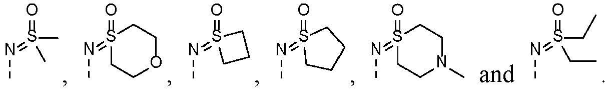

11. The compound according to claim 1 or a pharmaceutically acceptable salt thereof, X₁ is selected from N and CH.

12. The compound according to claim 1 or a pharmaceutically acceptable salt thereof, each R₃ is independently selected from hydrogen and fluorine.

13. The compound according to claim 1 or a pharmaceutically acceptable salt thereof, structural moiety 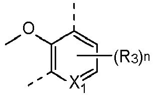 is selected from

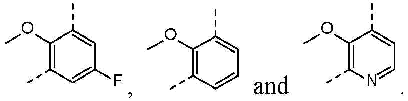

14. The compound according to claim 1 or a pharmaceutically acceptable salt thereof, R₄ is selected from hydrogen, -C(O)R₄₁, -C(O)NR₄₂R₄₃, 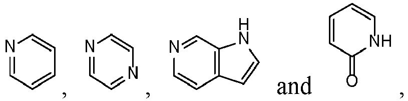 wherein the 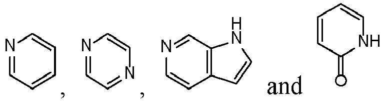 are optionally substituted with 1, 2 or 3 R_{b}.

15. The compound according to claim 14 or a pharmaceutically acceptable salt thereof, the R₄₁ is selected from methyl, ethyl, propyl, cyclopropyl, cyclobutyl, 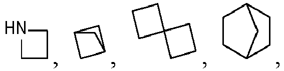 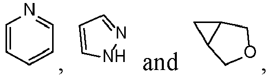 wherein the methyl, ethyl, propyl, cyclopropyl, cyclobutyl, 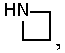 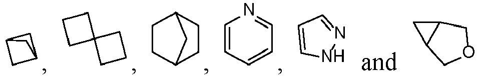 are optionally substituted with 1, 2, 3 or 4 R_{c}.

16. The compound according to claim 14 or a pharmaceutically acceptable salt thereof, the R₄₂ is selected from hydrogen.

17. The compound according to claim 14 or a pharmaceutically acceptable salt thereof, the R₄₃ is selected from methyl, ethyl, propyl, cyclopropyl, and cyclobutyl, wherein the methyl, ethyl, propyl, cyclopropyl and cyclobutyl are optionally substituted with 1, 2, 3 or 4 R_{c}.

18. The compound according to any one of claims 14-17 or a pharmaceutically acceptable salt thereof, wherein R_{c} is selected from H.

19. The compound according to claim 1 or a pharmaceutically acceptable salt thereof, wherein R_{b} is selected from hydrogen, fluorine, CN and methyl.

20. The compound according to claim 1 or a pharmaceutically acceptable salt thereof, wherein R₄ is selected from

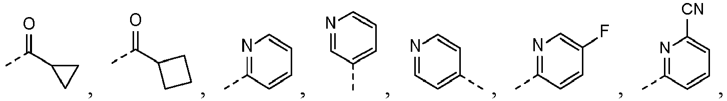

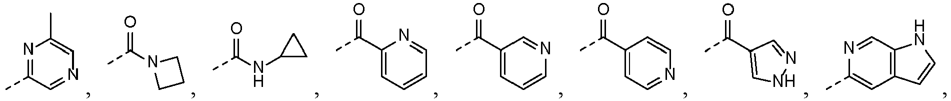

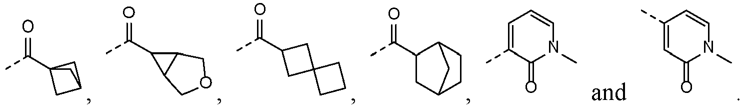

21. The compound according to claim 1 or a pharmaceutically acceptable salt thereof, R₅ is selected from hydrogen, methyl and ethyl.

22. The compound according to claim 1 or a pharmaceutically acceptable salt thereof, R₆ is selected from methyl, ethyl, propyl, methoxy, ethoxy, cyclopropyl, cyclobutyl, -NHCH₃, - NHCH₂CH₃, -NH-cyclopropyl and -NH-cyclobutyl, wherein the methyl, ethyl, propyl, methoxy, ethoxy, cyclopropyl, cyclobutyl, -NHCH₃, -NHCH₂CH₃, -NH-cyclopropyl and -NH-cyclobutyl are optionally substituted with 1, 2, 3 or 4 R_{d}.

23. The compound according to claim 22 or a pharmaceutically acceptable salt thereof, R₆ is selected from methyl, ethyl, -NHCH₃ and cyclopropyl, wherein the methyl, ethyl, -NHCH₃ and cyclopropyl are optionally substituted with 1, 2, 3 or 4 R_{d}.

24. The compound according to any one of claims 22-23 or a pharmaceutically acceptable salt thereof, R_{d} is selected from hydrogen, deuterium and methoxy.

25. The compound according to claim 1 or a pharmaceutically acceptable salt thereof, R₆ is selected from -CH₂CD₃, -CH₂CH₃, -NHCD₃, -CH₂OCH₃, -CH₃ and cyclopropyl.

26. The compound according to claim 1 or a pharmaceutically acceptable salt thereof, wherein the compound is represented by formula (II-1) or (II-2): 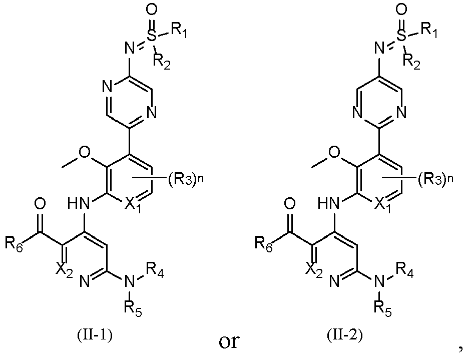 wherein R₁, R₂, R₃, R₄, R₅, R₆, X₁, X₂ and n are as defined in claim 1.

27. The compound according to claim 26 or a pharmaceutically acceptable salt thereof, the compound is represented by formula (II-1-1) or (II-1-2): 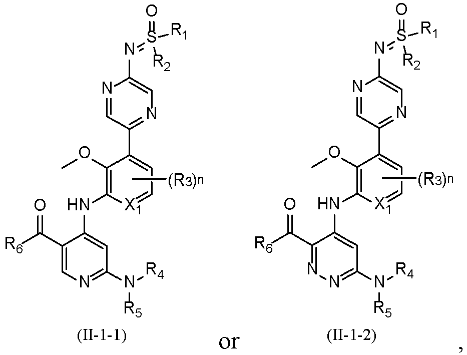 wherein R₁, R₂, R₃, R₄, R₅, R₆, X₁ and n are as defined in claim 24.

28. The compound according to claim 27 or a pharmaceutically acceptable salt thereof, the compound is represented by formula (II-1-1-1) or (II-1-1-2): 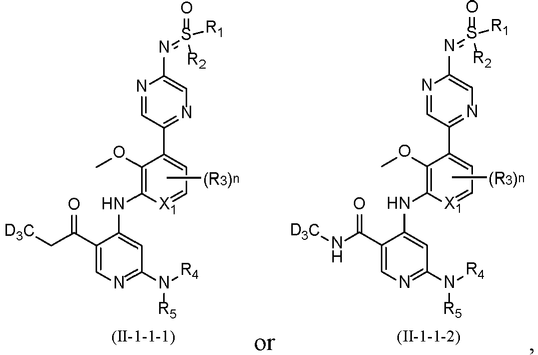 wherein R₁, R₂, R₃, R₄, R₅, Xi and n are as defined in claim 1.

29. A compound, which is one of the following compounds, or a pharmaceutically acceptable salt thereof,
